# EUROPEAN PATENT SPECIFICATION

(11) **EP 1 882 737 B1**
(45) Date of publication and mention of the grant of the patent: **14.09.2011**
(21) Application number: 06756354.4
(22) Date of filing: 18.05.2006
(51) Int. Cl.: C12N 15/09, C12N 1/15, C12N 1/19, C12N 1/21, C12N 5/10, C12N 9/10, C12P 13/04

(54) **PROCESS FOR PRODUCTION OF L-SERINE DERIVATIVE AND ENZYME USED IN THE PROCESS**
VERFAHREN ZUR HERSTELLUNG EINES L-SERIN-DERIVATS UND BEI DEM VERFAHREN VERWENDETES ENZYM
PROCÉDÉ DE FABRICATION D'UN DÉRIVÉ DE L-SÉRINE ET ENZYME UTILISÉE DANS LEDIT PROCÉDÉ

(30) Priority: 20.05.2005 JP 2005148660
(43) Date of publication of application: 30.01.2008
(73) Proprietor: Ajinomoto Co., Inc., Tokyo 104-8315 (JP)
(72) Inventor: KURODA, Shinji, c/o Ajinomoto Co., Inc., Kawasaki-shi, Kanagawa 2108681 (JP); NOZAKI, Hiroyuki, c/o Ajinomoto Co., Inc., Kawasaki-shi, Kanagawa 2108681 (JP); WATANABE, Kunihiko, c/o Ajinomoto Co., Inc., Kawasaki-shi, Kanagawa 2108681 (JP); YOKOZEKI, Kenzo, c/o Ajinomoto Co., Inc., Kawasaki-shi, Kanagawa 2108681 (JP); IMABAYASHI, Yuki, c/o Ajinomoto Co., Inc., Kawasaki-shi, Kanagawa 2108681 (JP)
(74) Representative: HOFFMANN EITLE
(86) International application number: PCT/JP2006/309949
(87) International publication number: WO 2006/123745

(56) References cited:
- JP-A- 06 181 788
- JP-A- 11 266 881
- US-A- 3 755 081
- US-A- 4 782 021
- US-A- 5 346 828
- HIROYUKI NOZAKI ET AL: "Purification and Gene Cloning of alpha-Methylserine Aldolase from Ralstonia sp. Strain AJ110405 and Application of the Enzyme in the Synthesis of alpha-Methyl-L-Serine" APPLIED AND ENVIRONMENTAL MICROBIOLOGY, AMERICAN SOCIETY FOR MICROBIOLOGY, US, vol. 74, no. 24, 1 December 2008 (2008-12-01), pages 7596-7599, XP007910881 ISSN: 0099-2240
- HIROYUKI NOZAKI ET AL: "Gene Cloning of á-Methylserine Aldolase from Variovorax paradoxus and Purification and Characterization of the Recombinant Enzyme" BIOSCIENCE BIOTECHNOLOGY BIOCHEMISTRY, JAPAN SOCIETY FOR BIOSCIENCE, BIOTECHNOLOGY, AND AGROCHEMISTRY, TOKYO, JAPAN, vol. 72, no. 10, 1 January 2008 (2008-01-01), pages 2580-2588, XP007910894 ISSN: 0916-8451
- TANAKA Y. ET AL.: 'Cultural Conditions for Microbial Conversion of Glycine into L-Serine in the presence of Tribasic Magnesium Phosphate' J. FERMENT. TECHNOL. vol. 58, no. 5, 1980, pages 417 - 422, XP003003137

### SEQUENCE LISTING

<110> AJINOMOTO CO., INC.
<120> Method for producing L-serine Derivative and Enzyme used Therefore
<130> PAMA-18262
<150> JP2005-148660
<151> 2005-05-20
<160> 34
<170> PatentIn version 3.1
<210> 1
<211> 30
<212> PRT
<213> Ralstonia sp.
<400> 1
   Met Leu Asn Ala Arg Pro Trp Val Pro Glu Gly Pro Glu Glu Tyr Met
   1 5 10 15
   Gln Ala Leu Ala Lys Arg Phe Ala Gly Gln Thr Pro Asp Gln
   20 25 30
<210> 2
<211> 23
<212> DNA
<213> Artificial
<220>
<223> primer
<220>
<221> misc_feature
<222> (18)..(18)
<223> a, c, g or t
<220>
<221> misc_feature
<222> (21)..(21)
<223> a, c, g or t
<400> 2
   gcytgcatrt aytcytcngg ncc 23
<210> 3
<211> 23
<212> DNA
<213> Artificial
<220>
<223> primer
<220>
<221> misc_feature
<222> (3)..(3)
<223> a, c, g or t
<220>
<221> misc_feature
<222> (6)..(6)
<223> a, c, g or t
<220>
<221> misc_feature
<222> (12)..(12)
<223> a, c, g or t
<220>
<221> misc_feature
<222> (18)..(18)
<223> a, c, g or t
<400> 3
   tcnggnaccc anggscgngc rtt 23
<210> 4
<211> 1317
<212> DNA
<213> Ralstonia sp.
<220>
<221> CDS
<222> (1)..(1317)
<400> 4
   atg ttg aac gca cgc ccc tgg gtg ccg gaa ggt ccc gaa gaa tat atg      48
   Met Leu Asn Ala Arg Pro Trp Val Pro Glu Gly Pro Glu Glu Tyr Met
   1               5                   10                  15
   caa gct ctg gcc aag cgt ttt gct ggc cag acc cct gac cag aac gaa      96
   Gln Ala Leu Ala Lys Arg Phe Ala Gly Gln Thr Pro Asp Gln Asn Glu
               20                  25                  30
   cgc gac ctg ctc gcc ttc gtt gaa gag aat cgc gtc att cac gaa cgt      144
   Arg Asp Leu Leu Ala Phe Val Glu Glu Asn Arg Val Ile His Glu Arg
           35                  40                  45
   gat tgc ttc aat ctg aac ccg gcc acc aat gcc atc aat ccg aag gcc      192
   Asp Cys Phe Asn Leu Asn Pro Ala Thr Asn Ala Ile Asn Pro Lys Ala
       50                  55                  60
   gag gcg atg ctg gcc tcc ggt gtc ggt tcg cgg cca tcg ctc ggc tac      240
   Glu Ala Met Leu Ala Ser Gly Val Gly Ser Arg Pro Ser Leu Gly Tyr
   65                  70                  75                  80
   ccg ggc gac aag tac gag atg ggc ctg gag ggc gtg gag aag att gag      288
   Pro Gly Asp Lys Tyr Glu Met Gly Leu Glu Gly Val Glu Lys Ile Glu
                   85                  90                  95
   gtg ttg gcg gcg gaa ctc gta gcc gag gtg ttc ggt gcc aag tac gcc      336
   Val Leu Ala Ala Glu Leu Val Ala Glu Val Phe Gly Ala Lys Tyr Ala
               100                 105                 110
   gag ttg cgt gtt gcc tcg ggc gcc ctc gcc aat ctc tac gcc tac atg      384
   Glu Leu Arg Val Ala Ser Gly Ala Leu Ala Asn Leu Tyr Ala Tyr Met
           115                 120                 125
   att gcc gcc aag ccg ggc gat acg gtg ttc gtc ccc agc gct acc atc      432
   Ile Ala Ala Lys Pro Gly Asp Thr Val Phe Val Pro Ser Ala Thr Ile
       130                 135                 140
   ggc ggc cac ttc agc cac cat gcg aat ggg gcg gcc ggc atg tac gga      480
   Gly Gly His Phe Ser His His Ala Asn Gly Ala Ala Gly Met Tyr Gly
   145                 150                 155                 160
   gtc aat tcg tac ctg atg ccg ttc gat gcc gac aag tac acc gtc gac      528
   Val Asn Ser Tyr Leu Met Pro Phe Asp Ala Asp Lys Tyr Thr Val Asp
                   165                 170                 175
   gtc gac cgt ctg cgc gag gat gca cgc agg ctc aag ccc aag atg att      576
   Val Asp Arg Leu Arg Glu Asp Ala Arg Arg Leu Lys Pro Lys Met Ile
               180                 185                 190
   acc ctg ggc aac agc ctc aac ctc ttt ccc cat ccc atc aag gag gtg      624
   Thr Leu Gly Asn Ser Leu Asn Leu Phe Pro His Pro Ile Lys Glu Val
           195                 200                 205
   agg gag att gcc gac gaa atc ggc gcg ctg gtc ctc ttt gac gcg gcg      672
   Arg Glu Ile Ala Asp Glu Ile Gly Ala Leu Val Leu Phe Asp Ala Ala
       210                 215                 220
   cat ctt tgc gga ctg att gcc ggc cac tcc tgg cag cag ccg ctg gaa      720
   His Leu Cys Gly Leu Ile Ala Gly His Ser Trp Gln Gln Pro Leu Glu
   225                 230                 235                 240
   gaa ggt gct cat ctc atg acc ttg agc acg tat aaa agc ctt gcc ggc      768
   Glu Gly Ala His Leu Met Thr Leu Ser Thr Tyr Lys Ser Leu Ala Gly
                   245                 250                 255
   ccc gcc ggc ggc ctt atc gtg acc aac gac gct gag gtt gcc aag cgc      816
   Pro Ala Gly Gly Leu Ile Val Thr Asn Asp Ala Glu Val Ala Lys Arg
               260                 265                 270
   ctg gac aca gtt gcc tat ccg ggc atg acc gcg aac ttc gat tcg gcc      864
   Leu Asp Thr Val Ala Tyr Pro Gly Met Thr Ala Asn Phe Asp Ser Ala
           275                 280                 285
   cgc tcg gca tcc att gcg atg acg atg ctg gac tgg caa gtc tat ggc      912
   Arg Ser Ala Ser Ile Ala Met Thr Met Leu Asp Trp Gln Val Tyr Gly
       290                 295                 300
   cgt gag tac gct gcc gag atg gtg cgt acc agc aag gcc ttc gcc gaa      960
   Arg Glu Tyr Ala Ala Glu Met Val Arg Thr Ser Lys Ala Phe Ala Glu
   305                 310                 315                 320
   gcg ctg gtc aag gag ggg ctc ccg gtg ttt gcg cgc gac cgc ggt atc     1008
   Ala Leu Val Lys Glu Gly Leu Pro Val Phe Ala Arg Asp Arg Gly Ile
                   325                 330                 335
   acg aca tct cat cag ttc gcg att gaa gcg cat gac ttc ggg ggg ggg     1056
   Thr Thr Ser His Gln Phe Ala Ile Glu Ala His Asp Phe Gly Gly Gly
               340                 345                 350
   cag gcg atg gca aag ctt ctg cgc cgc gcg aac att ctc gcg tgc gga     1104
   Gln Ala Met Ala Lys Leu Leu Arg Arg Ala Asn Ile Leu Ala Cys Gly
           355                 360                 365
   atc ggc tta cca ctt ccg gaa atc gcg ggg gac gtg aac ggt ctg cgt     1152
   Ile Gly Leu Pro Leu Pro Glu Ile Ala Gly Asp Val Asn Gly Leu Arg
       370                 375                 380
   atg gga acg ccc gag ctc gta cgt tgg gga atg cgt agc gag cac atg     1200
   Met Gly Thr Pro Glu Leu Val Arg Trp Gly Met Arg Ser Glu His Met
   385 390 395 400
   cca caa ctc gct aag ttc atc gca gac gtg ctg ctg ggt cgc cag gtg 1248
   Pro Gln Leu Ala Lys Phe Ile Ala Asp Val Leu Leu Gly Arg Gln Val
   405 410 415
   cca gaa gaa gtc gcg cca gcc gtc acc gac tac cgt cga cag ttc aac 1296
   Pro Glu Glu Val Ala Pro Ala Val Thr Asp Tyr Arg Arg Gln Phe Asn
   420 425 430
   aag ctg cat ttc ctg cgc taa 1317
   Lys Leu His Phe Leu Arg
   435
<210> 6
<211> 33
<212> DNA
<213> Artificial
<220>
<223> primer
<400> 6
   cggaattcga gaggaactga gcatgttgaa cgc 33
<210> 7 33
<211> 33
<212> DNA
<213> Artificial
<220>
<223> primer
<400> 7
   aactgcagtt agcgcaggaa atgcagcttg ttg 33
<210> 8
<211> 1326
<212> DNA
<213> Variovorax Paradoxus
<220>
<221> CDS
<222> (1)..(1326)
<400> 8
   atg cct gcc gcc gcc ctg caa cgc cgc ccc tgg gtt ccc gcc gcc agc      48
   Met Pro Ala Ala Ala Leu Gln Arg Arg Pro Trp Val Pro Ala Ala Ser
   1               5                   10                  15
   gaa gac cat gtg ctt tcc atc gcc gcc gat gcc gct gcg cgc gat gcg      96
   Glu Asp His Val Leu Ser Ile Ala Ala Asp Ala Ala Ala Arg Asp Ala
               20                  25                  30
   gcg agc gtc gcc gtc gag atc gaa cgg ctg gtg gcc gag aac cac cgc     144
   Ala Ser Val Ala Val Glu Ile Glu Arg Leu Val Ala Glu Asn His Arg
           35                  40                  45
   atc cac gac gtc gac ggc ctg aac ctc aac ccc gcc acc aac gtg atg     192
   Ile His Asp Val Asp Gly Leu Asn Leu Asn Pro Ala Thr Asn Val Met
       50                  55                  60
   aac ccg gcc gcc gaa gct ttg ctg tcg cgc ggg ctc ggc tcg cgc ccg     240
   Asn Pro Ala Ala Glu Ala Leu Leu Ser Arg Gly Leu Gly Ser Arg Pro
   65                  70                  75                  80
   tcg ctg ggc tac ccg ggc gac aag tac gag atg ggg ctc gag gcc atc     288
   Ser Leu Gly Tyr Pro Gly Asp Lys Tyr Glu Met Gly Leu Glu Ala Ile
                   85                  90                  95
   gag cgc atc gag gtc gtc gcg gcc gaa ctc gca gcc gaa gtg ttc ggc     336
   Glu Arg Ile Glu Val Val Ala Ala Glu Leu Ala Ala Glu Val Phe Gly
               100                 105                 110
   gcg aag ttc gcc gag gtg cgc gtg agc tcc ggc gcg ctg tcg aat ctc     384
   Ala Lys Phe Ala Glu Val Arg Val Ser Ser Gly Ala Leu Ser Asn Leu
           115                 120                 125
   tac gtg ttc atg gcg acc tgc cgg ccc ggc gac acg atc atc gtg ccg     432
   Tyr Val Phe Met Ala Thr Cys Arg Pro Gly Asp Thr Ile Ile Val Pro
       130                 135                 140
   ccg ccc agc atc ggt ggc cat gtc acg cac cat gcg gcg ggc gcg gcg     480
   Pro Pro Ser Ile Gly Gly His Val Thr His His Ala Ala Gly Ala Ala
   145                 150                 155                 160
   ggg ctc tat ggc ctg aag ccg gtt tcc gcg ccg gtc gat gcc gac ggc     528
   Gly Leu Tyr Gly Leu Lys Pro Val Ser Ala Pro Val Asp Ala Asp Gly
                   165                 170                 175
   tac acg gtc gac gtg gct gcg ctg gcg aag ttg gcg ggc gaa gtg aag     576
   Tyr Thr Val Asp Val Ala Ala Leu Ala Lys Leu Ala Gly Glu Val Lys
               180                 185                 190
   ccg aag ctc atc acc atc ggc ggc agc ctg aac ctg ttc ccg cat ccg     624
   Pro Lys Leu Ile Thr Ile Gly Gly Ser Leu Asn Leu Phe Pro His Pro
           195                 200                 205
   gtg ccc gcg atc cgc gag atc gcc gac ggc gtg ggc gcg aag ctg ctg     672
   Val Pro Ala Ile Arg Glu Ile Ala Asp Gly Val Gly Ala Lys Leu Leu
       210                 215                 220
   ttc gac gcc gcg cac ctc tcg ggc atg gtg gcc ggc aag gcg tgg ccg 720
   Phe Asp Ala Ala His Leu Ser Gly Met Val Ala Gly Lys Ala Trp Pro
   225 230 235 240
   cag ccg ctg gag cag ggc gcg cat gcg atc acc atg agc acc tac aag 768
   Gln Pro Leu Glu Gln Gly Ala His Ala Ile Thr Met Ser Thr Tyr Lys
   245 250 255
   agc ctg ggc ggc ccg gcg ggc ggg ctg atc gtg tcg aac gac gcg gcg 816
   Ser Leu Gly Gly Pro Ala Gly Gly Leu Ile Val Ser Asn Asp Ala Ala
   260 265 270
   ctg atg gag cgc atc gac gcc atc gcg tat ccc ggc ctc acg gcc aat 864
   Leu Met Glu Arg Ile Asp Ala Ile Ala Tyr Pro Gly Leu Thr Ala Asn
   275 280 285
   tcg gat gcg ggc cgc acg gcc gca ctg gcg cgt agc ctg ctc gac tgg 912
   Ser Asp Ala Gly Arg Thr Ala Ala Leu Ala Arg Ser Leu Leu Asp Trp
   290 295 300
   aaa gtg cat ggc gtg gcc tat gca gcc gcg atg cgc gag acc gcg cag 960
   Lys Val His Gly Val Ala Tyr Ala Ala Ala Met Arg Glu Thr Ala Gln
   305 310 315 320
   gcg ctg gcg cgc gca ctc gat gcg cgg ggc ctg ccc gtg ttt gtc aag 1008
   Ala Leu Ala Arg Ala Leu Asp Ala Arg Gly Leu Pro Val Phe Val Lys
   325 330 335
   gcg cgc ggc ttc acg cag tcg cat cag ctc gcg gtc gaa gcg gcg cgc 1056
   Ala Arg Gly Phe Thr Gln Ser His Gln Leu Ala Val Glu Ala Ala Arg
   340 345 350
   tgg ggc ggt ggg cag cac gcg gcg aag aag atc gcg caa ggc ggc ctg 1104
   Trp Gly Gly Gly Gln His Ala Ala Lys Lys Ile Ala Gln Gly Gly Leu
   355 360 365
   ctg gcc tgc ggc atc ggc ctg ccg atc gcg ccc gtg gaa ggc gac atc 1152
   Leu Ala Cys Gly Ile Gly Leu Pro Ile Ala Pro Val Glu Gly Asp Ile
   370 375 380
   aac ggc ctg cgg ctc ggc gtg ccc gag atc gtg cgc ctg ggc ttc acg 1200
   Asn Gly Leu Arg Leu Gly Val Pro Glu Ile Val Arg Leu Gly Phe Thr
   385 390 395 400
   ccc gac gac atg ccg cag ctg gcc gac tgg atc gcg cga gcg ctc gaa 1248
   Pro Asp Asp Met Pro Gln Leu Ala Asp Trp Ile Ala Arg Ala Leu Glu
   405 410 415
   ggc gac gca gct tct gtg gcc gcc gaa gtg cga gag cgc cgc acg cac 1296
   Gly Asp Ala Ala Ser Val Ala Ala Glu Val Arg Glu Arg Arg Thr His
   420 425 430
   ctc ggc gaa ctg cgc tac atc gtg cgc tga 1326
   Leu Gly Glu Leu Arg Tyr Ile Val Arg
   435 440
<210> 9
<211> 441'
<212> PRT
<213> Variovorax Paradoxus
<400> 9
   Met Pro Ala Ala Ala Leu Gln Arg Arg Pro Trp Val Pro Ala Ala Ser
   1               5                   10                  15
   Glu Asp His Val Leu Ser Ile Ala Ala Asp Ala Ala Ala Arg Asp Ala
               20                  25                  30
   Ala Ser Val Ala Val Glu Ile Glu Arg Leu Val Ala Glu Asn His Arg
           35                  40                  45
   Ile His Asp Val Asp Gly Leu Asn Leu Asn Pro Ala Thr Asn Val Met
       50                  55                  60
   Asn Pro Ala Ala Glu Ala Leu Leu Ser Arg Gly Leu Gly Ser Arg Pro
   65                  70                  75                  80
   Ser Leu Gly Tyr Pro Gly Asp Lys Tyr Glu Met Gly Leu Glu Ala Ile
                   85                  90                  95
   Glu Arg Ile Glu Val Val Ala Ala Glu Leu Ala Ala Glu Val Phe Gly
               100                 105                 110
   Ala Lys Phe Ala Glu Val Arg Val Ser Ser Gly Ala Leu Ser Asn Leu
           115                 120                 125
   Tyr Val Phe Met Ala Thr Cys Arg Pro Gly Asp Thr Ile Ile Val Pro
       130                 135                 140
   Pro Pro Ser Ile Gly Gly His Val Thr His His Ala Ala Gly Ala Ala
   145                 150                 155                 160
   Gly Leu Tyr Gly Leu Lys Pro Val Ser Ala Pro Val Asp Ala Asp Gly
                   165                 170                 175
   Tyr Thr Val Asp Val Ala Ala Leu Ala Lys Leu Ala Gly Glu Val Lys
               180                 185                 190
   Pro Lys Leu Ile Thr Ile Gly Gly Ser Leu Asn Leu Phe Pro His Pro
           195                 200                 205
   Val Pro Ala Ile Arg Glu Ile Ala Asp Gly Val Gly Ala Lys Leu Leu
       210                 215                 220
   Phe Asp Ala Ala His Leu Ser Gly Met Val Ala Gly Lys Ala Trp Pro
   225                 230                 235                 240
   Gln Pro Leu Glu Gln Gly Ala His Ala Ile Thr Met Ser Thr Tyr Lys
                   245                 250                 255
   Ser Leu Gly Gly Pro Ala Gly Gly Leu Ile Val Ser Asn Asp Ala Ala
               260                 265                 270
   Leu Met Glu Arg Ile Asp Ala Ile Ala Tyr Pro Gly Leu Thr Ala Asn
           275                 280                 285
   Ser Asp Ala Gly Arg Thr Ala Ala Leu Ala Arg Ser Leu Leu Asp Trp
       290                 295                 300
   Lys Val His Gly Val Ala Tyr Ala Ala Ala Met Arg Glu Thr Ala Gln
   305                 310                 315                 320
   Ala Leu Ala Arg Ala Leu Asp Ala Arg Gly Leu Pro Val Phe Val Lys
                   325                 330                 335
   Ala Arg Gly Phe Thr Gln Ser His Gln Leu Ala Val Glu Ala Ala Arg
               340                 345                 350
   Trp Gly Gly Gly Gln His Ala Ala Lys Lys Ile Ala Gln Gly Gly Leu
           355                 360                 365
   Leu Ala Cys Gly Ile Gly Leu Pro Ile Ala Pro Val Glu Gly Asp Ile
       370                 375                 380
   Asn Gly Leu Arg Leu Gly Val Pro Glu Ile Val Arg Leu Gly Phe Thr
   385                 390                 395                 400
   Pro Asp Asp Met Pro Gln Leu Ala Asp Trp Ile Ala Arg Ala Leu Glu
                   405                 410                 415
   Gly Asp Ala Ala Ser Val Ala Ala Glu Val Arg Glu Arg Arg Thr His
               420                 425                 430
   Leu Gly Glu Leu Arg Tyr Ile Val Arg
           435                 440
<210> 10
<211> 32
<212> DNA
<213> Artificial
<220>
<223> primer
<400> 10
   cacacaggaa acagcatatg gccatgatta cg 32
<210> 11
<211> 32
<212> DNA
<213> Artificial
<220>
<223> primer
<400> 11
   cgtaatcatg gccatatgct gtttcctgtg tg 32
<210> 12
<211> 30
<212> DNA
<213> Artificial
<220>
<223> primer
<400> 12
   ggaattccat atgcctgccg ccgccctgca 30
<210> 13
<211> 33
<212> DNA
<213> Artificial
<220>
<223> primer
<400> 13
   aactgcagtc agcgcacgat gtagcgcagt tcg 33
<210> 14
<211> 1323
<212> DNA
<213> Variovorax Paradoxus
<220>
<221> CDS
<222> (1)..(1323)
<400> 14
   atg ccc gcc gcc ctc caa cgc cgc tcc tgg gtt ccc gcc gcc agc gaa 48
   Met Pro Ala Ala Leu Gln Arg Arg Ser Trp Val Pro Ala Ala Ser Glu
   1 5 10 15
   gac cat gtg ctc gcg atc gcc gcc gat gcc gct gcg cgc gat gcg ctg 96
   Asp His Val Leu Ala Ile Ala Ala Asp Ala Ala Ala Arg Asp Ala Leu
   20 25 30
   ggc atc gcc gcc gag atc gaa agg ctc gcc gac gac aac cac cgc atc 144
   Gly Ile Ala Ala Glu Ile Glu Arg Leu Ala Asp Asp Asn His Arg Ile
   35 40 45
   cac gac cgc gag ggc ctg aac ctc aac ccc gcc acc aac gtg atg aac 192
   His Asp Arg Glu Gly Leu Asn Leu Asn Pro Ala Thr Asn Val Met Asn
   50 55 60
   ccg gcc gcc gag gcg ctg ctg tcg cgc ggc ctg ggc tcg cgg gca tcg 240
   Pro Ala Ala Glu Ala Leu Leu Ser Arg Gly Leu Gly Ser Arg Ala Ser
   65 70 75 80
   ctg ggc tac cca ggg gac aag tac gaa gtg ggg ctg gag gcc atc gag 288
   Leu Gly Tyr Pro Gly Asp Lys Tyr Glu Val Gly Leu Glu Ala Ile Glu
   85 90 95
   cgc atc gag gtc atc gcg gcc gaa ctg gcc gcc gag gtg ttc ggc tcg 336
   Arg Ile Glu Val Ile Ala Ala Glu Leu Ala Ala Glu Val Phe Gly Ser
   100 105 110
   aag ttc gcc gaa gtg cgc gtg agc tcc ggc gcg ctg tcg aat ctc tat 384
   Lys Phe Ala Glu Val Arg Val Ser Ser Gly Ala Leu Ser Asn Leu Tyr
   115 120 125
   gtg ttc atg gca acc tgc cgg ccc ggc gac acg atc att gcg ccg ccg 432
   Val Phe Met Ala Thr Cys Arg Pro Gly Asp Thr Ile Ile Ala Pro Pro
   130 135 140
   ccg gcc atc ggc ggc cac gtc acg cac cat gcg gcc ggc gcg gcc ggg 480
   Pro Ala Ile Gly Gly His Val Thr His His Ala Ala Gly Ala Ala Gly
   145 150 155 160
   ctc tat gga ttg aag acg gtg ccc gcg ccg gtc gat gcc gac ggc tac 528
   Leu Tyr Gly Leu Lys Thr Val Pro Ala Pro Val Asp Ala Asp Gly Tyr
   165 170 175
   acg gtc gac gcg gcc gcg ctc gcc agg ctc gca cgc gag gtg aag ccg 576
   Thr Val Asp Ala Ala Ala Leu Ala Arg Leu Ala Arg Glu Val Lys Pro
   180 185 190
   aag ctc atc acc atc ggc ggc agc ctg aat ctt ttt ccg cac ccg gtg 624
   Lys Leu Ile Thr Ile Gly Gly Ser Leu Asn Leu Phe Pro His Pro Val
   195 200 205
   cct gcg atc cgc gag gtc gcg gac agc gtg ggc gcc aaa ctg ctg ttc 672
   Pro Ala Ile Arg Glu Val Ala Asp Ser Val Gly Ala Lys Leu Leu Phe
   210 215 220
   gac gct gcg cac ctc tcg ggc atg gtg gcc ggc aag gcc tgg ccg cag 720
   Asp Ala Ala His Leu Ser Gly Met Val Ala Gly Lys Ala Trp Pro Gln
   225 230 235 240
   ccg ctc gag gag ggc gcg cac gcg atc acc atg agc acc tac aag agc 768
   Pro Leu Glu Glu Gly Ala His Ala Ile Thr Met Ser Thr Tyr Lys Ser
   245 250 255
   ctc ggc gga ccg gcc ggc ggg ctg atc gtg tcg aac gat gcg gcg ctg 816
   Leu Gly Gly Pro Ala Gly Gly Leu Ile Val Ser Asn Asp Ala Ala Leu
   260 265 270
   atg gag cgc atc gac gcc atc gcc tac ccc ggc ctc acg gcc aac tcc 864
   Met Glu Arg Ile Asp Ala Ile Ala Tyr Pro Gly Leu Thr Ala Asn Ser
   275 280 285
   gat gcg ggc cgc acc gcg gcg ctg gcg cgc gga ctg ctc gac tgg aag 912
   Asp Ala Gly Arg Thr Ala Ala Leu Ala Arg Gly Leu Leu Asp Trp Lys
   290 295 300
   gtg cac ggc agg gcc tac gcc gcg gca atg cgc gag acc gcg cag gcg 960
   Val His Gly Arg Ala Tyr Ala Ala Ala Met Arg Glu Thr Ala Gln Ala
   305 310 315 320
   ctg gcg cac gcg ctc gac gcc gag ggc ttg ccg gtg ttc gcg aag gcg 1008
   Leu Ala His Ala Leu Asp Ala Glu Gly Leu Pro Val Phe Ala Lys Ala
   325 330 335
   cgc ggc ttc acg cag tcg cac cag ttc gcg ctc gag gcc gcg cac tgg 1056
   Arg Gly Phe Thr Gln Ser His Gln Phe Ala Leu Glu Ala Ala His Trp
               340                 345                 350
   ggc ggt ggg cag cgc gcc gcg aag aaa ctg gcc gag ggc ggc ttg ctg     1104
   Gly Gly Gly Gln Arg Ala Ala Lys Lys Leu Ala Glu Gly Gly Leu Leu
           355                 360                 365
   gcc tgc ggc atc ggc ctg ccg atc gcg ccg gtc gaa gga gac atc aac     1152
   Ala Cys Gly Ile Gly Leu Pro Ile Ala Pro Val Glu Gly Asp Ile Asn
       370                 375                 380
   ggc ctg cgc ctg ggc gtg ccg gag atc gtg cgg ctg ggc ttc acg ccc     1200
   Gly Leu Arg Leu Gly Val Pro Glu Ile Val Arg Leu Gly Phe Thr Pro
   385                 390                 395                 400
   gac gac atg ccg cag ctt gcc tcg tgg atc gcg cgt gcg ctg gag ggc     1248
   Asp Asp Met Pro Gln Leu Ala Ser Trp Ile Ala Arg Ala Leu Glu Gly
                   405                 410                 415
   ggc ggc gca tcg gtg gcg gcc gag gtg cgc gag cgc cgc acg cgg ctc     1296
   Gly Gly Ala Ser Val Ala Ala Glu Val Arg Glu Arg Arg Thr Arg Leu
               420                 425                 430
   ggc ggc ctg cgc tac atc gtg cgc tga                                 1323
   Gly Gly Leu Arg Tyr Ile Val Arg
           435                 440
<210> 15
<211> 440
<212> PRT
<213> Variovorax Paradoxus
<400> 15
   Met Pro Ala Ala Leu Gln Arg Arg Ser Trp Val Pro Ala Ala Ser Glu
   1               5                   10                  15
   Asp His Val Leu Ala Ile Ala Ala Asp Ala Ala Ala Arg Asp Ala Leu
               20                  25                  30
   Gly Ile Ala Ala Glu Ile Glu Arg Leu Ala Asp Asp Asn His Arg Ile
           35                  40                  45
   His Asp Arg Glu Gly Leu Asn Leu Asn Pro Ala Thr Asn Val Met Asn
       50                  55                  60
   Pro Ala Ala Glu Ala Leu Leu Ser Arg Gly Leu Gly Ser Arg Ala Ser
   65                  70                  75                  80
   Leu Gly Tyr Pro Gly Asp Lys Tyr Glu Val Gly Leu Glu Ala Ile Glu
                   85                  90                  95
   Arg Ile Glu Val Ile Ala Ala Glu Leu Ala Ala Glu Val Phe Gly Ser
               100                 105                 110
   Lys Phe Ala Glu Val Arg Val Ser Ser Gly Ala Leu Ser Asn Leu Tyr
           115                 120                 125
   Val Phe Met Ala Thr Cys Arg Pro Gly Asp Thr Ile Ile Ala Pro Pro
       130                 135                 140
   Pro Ala Ile Gly Gly His Val Thr His His Ala Ala Gly Ala Ala Gly
   145                 150                 155                 160
   Leu Tyr Gly Leu Lys Thr Val Pro Ala Pro Val Asp Ala Asp Gly Tyr
                   165                 170                 175
   Thr Val Asp Ala Ala Ala Leu Ala Arg Leu Ala Arg Glu Val Lys Pro
               180                 185                 190
   Lys Leu Ile Thr Ile Gly Gly Ser Leu Asn Leu Phe Pro His Pro Val
           195                 200                 205
   Pro Ala Ile Arg Glu Val Ala Asp Ser Val Gly Ala Lys Leu Leu Phe
       210                 215                 220
   Asp Ala Ala His Leu Ser Gly Met Val Ala Gly Lys Ala Trp Pro Gln
   225                 230                 235                 240
   Pro Leu Glu Glu Gly Ala His Ala Ile Thr Met Ser Thr Tyr Lys Ser
                   245                 250                 255
   Leu Gly Gly Pro Ala Gly Gly Leu Ile Val Ser Asn Asp Ala Ala Leu
               260                 265                 270
   Met Glu Arg Ile Asp Ala Ile Ala Tyr Pro Gly Leu Thr Ala Asn Ser
           275                 280                 285
   Asp Ala Gly Arg Thr Ala Ala Leu Ala Arg Gly Leu Leu Asp Trp Lys
       290                 295                 300
   Val His Gly Arg Ala Tyr Ala Ala Ala Met Arg Glu Thr Ala Gln Ala
   305                 310                 315                 320
   Leu Ala His Ala Leu Asp Ala Glu Gly Leu Pro Val Phe Ala Lys Ala
                   325                 330                 335
   Arg Gly Phe Thr Gln Ser His Gln Phe Ala Leu Glu Ala Ala His Trp
               340                 345                 350
   Gly Gly Gly Gln Arg Ala Ala Lys Lys Leu Ala Glu Gly Gly Leu Leu
           355                 360                 365
   Ala Cys Gly Ile Gly Leu Pro Ile Ala Pro Val Glu Gly Asp Ile Asn
       370                 375                 380
   Gly Leu Arg Leu Gly Val Pro Glu Ile Val Arg Leu Gly Phe Thr Pro
   385                 390                 395                 400
   Asp Asp Met Pro Gln Leu Ala Ser Trp Ile Ala Arg Ala Leu Glu Gly
                   405                 410                 415
   Gly Gly Ala Ser Val Ala Ala Glu Val Arg Glu Arg Arg Thr Arg Leu
               420                 425                 430
   Gly Gly Leu Arg Tyr Ile Val Arg
           435                 440
<210> 16
<211> 30
<212> DNA
<213> Artificial
<220>
<223> primer
<400> 16
   ggaattccat atgcccgccg ccctccaacg 30
<210> 17
<211> 33
<212> DNA
<213> Artificial
<220>
<223> primer
<400> 17
   aactgcagtc agcgcacgat gtagcgcagg ccg 33
<210> 18
<211> 1323
<212> DNA
<213> Variovorax Paradoxus
<220>
<221> CDS
<222> (1)..(1323)
<400> 18
   atg ccc gca gcc ctt cac cgc cgt tcc tgg gtt ccc gcc gcc agc gag       48
   Met Pro Ala Ala Leu His Arg Arg Ser Trp Val Pro Ala Ala Ser Glu
   1               5                   10                  15
   gac cac gtg ctc gcc atc gct gcc gac gcc gcc gcg cgc gac gcg gcg       96
   Asp His Val Leu Ala Ile Ala Ala Asp Ala Ala Ala Arg Asp Ala Ala
               20                  25                  30
   ggc gtg gcc gcc gag gtc gaa cgc ctc gtg gcc gac agc cac cgc atc      144
   Gly Val Ala Ala Glu Val Glu Arg Leu Val Ala Asp Ser His Arg Ile
           35                  40                  45
   cac gac gtc gac ggg ctg aac ctc aac ccc gcc acc aac gtg atg aac      192
   His Asp Val Asp Gly Leu Asn Leu Asn Pro Ala Thr Asn Val Met Asn
       50                  55                  60
   ccc gcc gcc gaa gcg ctg ctg tcg cgc ggg ctg ggc tcg cgt ccg tcg      240
   Pro Ala Ala Glu Ala Leu Leu Ser Arg Gly Leu Gly Ser Arg Pro Ser
   65                  70                  75                  80
   ctg ggc tac ccg ggc gac aag tac gag atg ggg ctg gag gcc atc gag      288
   Leu Gly Tyr Pro Gly Asp Lys Tyr Glu Met Gly Leu Glu Ala Ile Glu
                   85                  90                  95
   cgc atc gag gtc gtc gcc gcc gag ctg gcg gcc gag gtg ttc ggc gcg      336
   Arg Ile Glu Val Val Ala Ala Glu Leu Ala Ala Glu Val Phe Gly Ala
               100                 105                 110
   cgc ttc gcc gag gtg cgc gtg agc tcg ggc gcg ctg tcg aac ctc tac      384
   Arg Phe Ala Glu Val Arg Val Ser Ser Gly Ala Leu Ser Asn Leu Tyr
           115                 120                 125
   gtg ttc atg gcg acc tgc cag ccc ggc gac acg atc atc gcg ccg ccg      432
   Val Phe Met Ala Thr Cys Gln Pro Gly Asp Thr Ile Ile Ala Pro Pro
       130                 135                 140
   ccc gcc atc ggc ggc cat gtg acg cac cac gcg gcg ggc gcg gcc ggg      480
   Pro Ala Ile Gly Gly His Val Thr His His Ala Ala Gly Ala Ala Gly
   145                 150                 155                 160
   ctc tac ggc ctg aag acc gtg ccg gcg ccg gtc gat gcc gac ggc tac      528
   Leu Tyr Gly Leu Lys Thr Val Pro Ala Pro Val Asp Ala Asp Gly Tyr
                   165                 170                 175
   tcg gtc gac gtg gtg gcg ctg gcg aaa ctg gcg cgc gag gtg aag ccg      576
   Ser Val Asp Val Val Ala Leu Ala Lys Leu Ala Arg Glu Val Lys Pro
               180                 185                 190
   aag ctc atc acc atc ggc ggc agc ctc aac ctg ttc ccg cac ccg gtg      624
   Lys Leu Ile Thr Ile Gly Gly Ser Leu Asn Leu Phe Pro His Pro Val
           195                 200                 205
   ccc gcg atc cgc gag gtg gcc gac agc gtg ggc gcc aag gtg ctc ttc      672
   Pro Ala Ile Arg Glu Val Ala Asp Ser Val Gly Ala Lys Val Leu Phe
       210                 215                 220
   gat gcc gcg cac ctc tcg ggc atg gtg gcc ggc aag gct tgg ccg cag      720
   Asp Ala Ala His Leu Ser Gly Met Val Ala Gly Lys Ala Trp Pro Gln
   225                 230                 235                 240
   ccg ctc gaa gac ggc gcg cac gcg atc acg atg agc acc tac aag agc      768
   Pro Leu Glu Asp Gly Ala His Ala Ile Thr Met Ser Thr Tyr Lys Ser
                   245                 250                 255
   ctg ggc ggc ccg gcc ggc ggg ctg atc gtg tcg aac gat gcg gcg ctg      816
   Leu Gly Gly Pro Ala Gly Gly Leu Ile Val Ser Asn Asp Ala Ala Leu
               260                 265                 270
   atg gag cgc atc gac gcc atc gcc tac ccc ggc ctc acg gcc aac tcg      864
   Met Glu Arg Ile Asp Ala Ile Ala Tyr Pro Gly Leu Thr Ala Asn Ser
           275                 280                 285
   gac gcg ggc cgc acc gcg gcg ctg gcg cgc ggt ctg ctc gac tgg aag      912
   Asp Ala Gly Arg Thr Ala Ala Leu Ala Arg Gly Leu Leu Asp Trp Lys
       290                 295                 300
   gtg cac ggc acg gcc tat gcg gcg gcg atg cgc gac acc gcg cag gcg      960
   Val His Gly Thr Ala Tyr Ala Ala Ala Met Arg Asp Thr Ala Gln Ala
   305                 310                 315                 320
   ctg gca cgc gcg ctc gat gcg cta ggc ctg ccg gtg ttc gcc aag gca     1008
   Leu Ala Arg Ala Leu Asp Ala Leu Gly Leu Pro Val Phe Ala Lys Ala
                   325                 330                 335
   cgc ggc ttc acg cag tcg cac cag ttc gcg ctc gag gct gcg cgc tgg     1056
   Arg Gly Phe Thr Gln Ser His Gln Phe Ala Leu Glu Ala Ala Arg Trp
               340                 345                 350
   ggc ggc ggg caa cgc gcc gcg aag cag ctg gcc cgg ggc ggg ttg ttg     1104
   Gly Gly Gly Gln Arg Ala Ala Lys Gln Leu Ala Arg Gly Gly Leu Leu
           355                 360                 365
   gcc tgc ggc atc ggc ctg ccg atc gca ccg gtc gac ggc gac atc aac     1152
   Ala Cys Gly Ile Gly Leu Pro Ile Ala Pro Val Asp Gly Asp Ile Asn
       370                 375                 380
   ggc ctg cgc ctg ggc gtg ccc gag atc gtg cgg ctg ggc ttc acg ccc     1200
   Gly Leu Arg Leu Gly Val Pro Glu Ile Val Arg Leu Gly Phe Thr Pro
   385                 390                 395                 400
   gag gac atg ccg caa ctg gcc ggc tgg atc gcg cgg gca ttg gct ggc     1248
   Glu Asp Met Pro Gln Leu Ala Gly Trp Ile Ala Arg Ala Leu Ala Gly
                   405                 410                 415
   gat gcg ccc gcc gtg gcc gcc gag gtg cgc gag cgg cgc acg cgg ctg     1296
   Asp Ala Pro Ala Val Ala Ala Glu Val Arg Glu Arg Arg Thr Arg Leu
               420                 425                 430
   aac ggc ctg cgc tac atc gtg cgc tga                                 1323
   Asn Gly Leu Arg Tyr Ile Val Arg
           435                 440
<210> 19
<211> 440
<212> PRT
<213> Variovorax Paradoxus
<400> 19
   Met Pro Ala Ala Leu His Arg Arg Ser Trp Val Pro Ala Ala Ser Glu
   1 5 10 15
   Asp His Val Leu Ala Ile Ala Ala Asp Ala Ala Ala Arg Asp Ala Ala
   20 25 30
   Gly Val Ala Ala Glu Val Glu Arg Leu Val Ala Asp Ser His Arg Ile
   35 40 45
   His Asp Val Asp Gly Leu Asn Leu Asn Pro Ala Thr Asn Val Met Asn
   50 55 60
   Pro Ala Ala Glu Ala Leu Leu Ser Arg Gly Leu Gly Ser Arg Pro Ser
   65 70 75 80
   Leu Gly Tyr Pro Gly Asp Lys Tyr Glu Met Gly Leu Glu Ala Ile Glu
   85 90 95
   Arg Ile Glu Val Val Ala Ala Glu Leu Ala Ala Glu Val Phe Gly Ala
   100 105 110
   Arg Phe Ala Glu Val Arg Val Ser Ser Gly Ala Leu Ser Asn Leu Tyr
   115 120 125
   Val Phe Met Ala Thr Cys Gln Pro Gly Asp Thr Ile Ile Ala Pro Pro
   130 135 140
   Pro Ala Ile Gly Gly His Val Thr His His Ala Ala Gly Ala Ala Gly
   145 150 155 160
   Leu Tyr Gly Leu Lys Thr Val Pro Ala Pro Val Asp Ala Asp Gly Tyr
   165 170 175
   Ser Val Asp Val Val Ala Leu Ala Lys Leu Ala Arg Glu Val Lys Pro
   180 185 190
   Lys Leu Ile Thr Ile Gly Gly Ser Leu Asn Leu Phe Pro His Pro Val
   195 200 205
   Pro Ala Ile Arg Glu Val Ala Asp Ser Val Gly Ala Lys Val Leu Phe
   210 215 220
   Asp Ala Ala His Leu Ser Gly Met Val Ala Gly Lys Ala Trp Pro Gln
   225 230 235 240
   Pro Leu Glu Asp Gly Ala His Ala Ile Thr Met Ser Thr Tyr Lys Ser
   245 250 255
   Leu Gly Gly Pro Ala Gly Gly Leu Ile Val Ser Asn Asp Ala Ala Leu
   260 265 270
   Met Glu Arg Ile Asp Ala Ile Ala Tyr Pro Gly Leu Thr Ala Asn Ser
   275 280 285
   Asp Ala Gly Arg Thr Ala Ala Leu Ala Arg Gly Leu Leu Asp Trp Lys
   290 295 300
   Val His Gly Thr Ala Tyr Ala Ala Ala Met Arg Asp Thr Ala Gln Ala
   305 310 315 320
   Leu Ala Arg Ala Leu Asp Ala Leu Gly Leu Pro Val Phe Ala Lys Ala
   325 330 335
   Arg Gly Phe Thr Gln Ser His Gln Phe Ala Leu Glu Ala Ala Arg Trp
   340 345 350
   Gly Gly Gly Gln Arg Ala Ala Lys Gln Leu Ala Arg Gly Gly Leu Leu
   355 360 365
   Ala Cys Gly Ile Gly Leu Pro Ile Ala Pro Val Asp Gly Asp Ile Asn
   370 375 380
   Gly Leu Arg Leu Gly Val Pro Glu Ile Val Arg Leu Gly Phe Thr Pro
   385 390 395 400
   Glu Asp Met Pro Gln Leu Ala Gly Trp Ile Ala Arg Ala Leu Ala Gly
   405 410 415
   Asp Ala Pro Ala Val Ala Ala Glu Val Arg Glu Arg Arg Thr Arg Leu
   420 425 430
   Asn Gly Leu Arg Tyr Ile Val Arg
   435 440
<210> 20
<210> 20
<211> 30
<212> DNA
<213> Artificial
<220>
<223> primer
<400> 20
   ggaattccat atgcccgcag cccttcaccg 30
<210> 21
<211> 33
<212> DNA
<213> Artificial
<220>
<223> primer
<400> 21
   aactgcagtc agcgcacgat gtagcgcagg ccg 33
<210> 22
<211> 1323
<212> DNA
<213> Bosa sp.
<220>
<221> CDS
<222> (1)..(1323)
<400> 22
   atg acg gct ctg ggc agg cgc gat tgg gtg ccg cag gcg agc gag gac      48
   Met Thr Ala Leu Gly Arg Arg Asp Trp Val Pro Gln Ala Ser Glu Asp
   1               5                  10                 15
   tat atc cag cgg atc gcc ggc gag acg gcg ggc cag ccg ctc gat gcc      96
   Tyr Ile Gln Arg Ile Ala Gly Glu Thr Ala Gly Gln Pro Leu Asp Ala
               20                  25                  30
   atc gcg gac agg att acg gcg ctg acc gcc gag aac cgc gcc atc cac     144
   Ile Ala Asp Arg Ile Thr Ala Leu Thr Ala Glu Asn Arg Ala Ile His
           35                  40                  45
   gag cgc gat tgc gtc aat ctc aac ccg gcc acc aat gtc atg aac ccg     192
   Glu Arg Asp Cys Val Asn Leu Asn Pro Ala Thr Asn Val Met Asn Pro
       50                  55                  60
   aag gcc gag gcg ctg ctc tcg gcg ggg atc ggg gcg cgg ccc tcg ctc     240
   Lys Ala Glu Ala Leu Leu Ser Ala Gly Ile Gly Ala Arg Pro Ser Leu
   65                  70                  75                  80
   ggc tat ccc ggc gac aaa tac gag atg ggg ctg gag gcg atc gag cag     288
   Gly Tyr Pro Gly Asp Lys Tyr Glu Met Gly Leu Glu Ala Ile Glu Gln
                   85                  90                  95
   atc gag gtg atc gcg gcc gag ctc gca gcc gag gtt ttc ggc gcg aca     336
   Ile Glu Val Ile Ala Ala Glu Leu Ala Ala Glu Val Phe Gly Ala Thr
               100                 105                 110
   tat gcg gag atc cgc gtg ccc tcc ggc gcc atc gcc aat ctc tac gcc     384
   Tyr Ala Glu Ile Arg Val Pro Ser Gly Ala Ile Ala Asn Leu Tyr Ala
           115                 120                 125
   ttc atg gtt gcg gcg aag gcg ggc gac tgc atc atc gcg ccg ccg ggc     432
   Phe Met Val Ala Ala Lys Ala Gly Asp Cys Ile Ile Ala Pro Pro Gly
       130                 135                 140
   gag atc ggc ggg cat gtc acc cat cac ggc gcg ggc gcg gcc ggg ctc     480
   Glu Ile Gly Gly His Val Thr His His Gly Ala Gly Ala Ala Gly Leu
   145                 150                 155                 160
   tac ggc atc att acc cat ccg gcg ccg atc gac ccc gtg aag tat acg     528
   Tyr Gly Ile Ile Thr His Pro Ala Pro Ile Asp Pro Val Lys Tyr Thr
                   165                 170                 175
   gtc gat gtc gaa aag ctg cgc gcg gac gcg ctc agg ctc cgg ccg aag     576
   Val Asp Val Glu Lys Leu Arg Ala Asp Ala Leu Arg Leu Arg Pro Lys
               180                 185                 190
   ctg atc tcg atc ggc ggc agc ctc aat ctc ttc ccg cat ccg atc cgc     624
   Leu Ile Ser Ile Gly Gly Ser Leu Asn Leu Phe Pro His Pro Ile Arg
           195                 200                 205
   gag atc cgc acc atc gcc gac gag gtc ggt gcg ctc gtg ctg ttc gac     672
   Glu Ile Arg Thr Ile Ala Asp Glu Val Gly Ala Leu Val Leu Phe Asp
       210                 215                 220
   gcg gcg cat atg tcc ggc atg atc gcc ggc cat ggc tgg cag cag ccg     720
   Ala Ala His Met Ser Gly Met Ile Ala Gly His Gly Trp Gln Gln Pro
   225                 230                 235                 240
   ctg gag gag ggt gct cat ctg atg acg atg agc acc tat aag agc ctc     768
   Leu Glu Glu Gly Ala His Leu Met Thr Met Ser Thr Tyr Lys Ser Leu
                   245                 250                 255
   ggc ggg ccg cct tca ggg ttg atc gtc acc aat gat gcc gac atc gcg     816
   Gly Gly Pro Pro Ser Gly Leu Ile Val Thr Asn Asp Ala Asp Ile Ala
               260                 265                 270
   aaa aaa ctc gac gcc atc gcc tat ccc ggg ctc acc gcc aat ttc gat     864
   Lys Lys Leu Asp Ala Ile Ala Tyr Pro Gly Leu Thr Ala Asn Phe Asp
           275                 280                 285
   gca gcc aag tcg gcg tcg ctg gcc gtc tcg ctc ctc gat tgg aaa gca     912
   Ala Ala Lys Ser Ala Ser Leu Ala Val Ser Leu Leu Asp Trp Lys Ala
       290                 295                 300
   cat ggc cgc gcc tat gcg cag gag atg gcc aag aca gcg aag gcc ttg     960
   His Gly Arg Ala Tyr Ala Gln Glu Met Ala Lys Thr Ala Lys Ala Leu
   305                 310                 315                 320
   gcc gag gct ctg tca gag cgg cag gtg ccg gtt ttc gcc cgc gac cgg    1008
   Ala Glu Ala Leu Ser Glu Arg Gln Val Pro Val Phe Ala Arg Asp Arg
                   325                 330                 335
   ggc atg acg acc tcg cat cag ttc gcc atc gaa gcc gcg ccc tat ggc    1056
   Gly Met Thr Thr Ser His Gln Phe Ala Ile Glu Ala Ala Pro Tyr Gly
               340                 345                 350
   ggt ggg cag gcg gca gcg aag cgg ctt cgc gcg gtc aac atc ctg tcc    1104
   Gly Gly Gln Ala Ala Ala Lys Arg Leu Arg Ala Val Asn Ile Leu Ser
           355                 360                 365
   tgc ggc atc ggc ctg ccg ctg ccg gcg gtg gag ggc gat gtg aac ggg    1152
   Cys Gly Ile Gly Leu Pro Leu Pro Ala Val Glu Gly Asp Val Asn Gly
       370                     375                     380
   ctc agg ctc ggc acg ccg gag atc gtg cgc ttt ggc atg acg gcc gcc      1200
   Leu Arg Leu Gly Thr Pro Glu Ile Val Arg Phe Gly Met Thr Ala Ala
   385                 390                     395                 400
   gac atg ccg gag ctg gcg ggt tat atc gcc gag ggg ttg aac ggc tcg      1248
   Asp Met Pro Glu Leu Ala Gly Tyr Ile Ala Glu Gly Leu Asn Gly Ser
                   405                     410                 415
   cgt ccg gcc gag gcg gtg gcg aag gac gtg acg gca ttc cgc ggc cgc      1296
   Arg Pro Ala Glu Ala Val Ala Lys Asp Val Thr Ala Phe Arg Gly Arg
               420                     425                 430
   ttc cgc gag ctg cat ttc atg cgc tga                                  1323
   Phe Arg Glu Leu His Phe Met Arg
           435                 440
<210> 23
<211> 440
<212> PRT
<213> Bosa sp.
<400> 23
   Met Thr Ala Leu Gly Arg Arg Asp Trp Val Pro Gln Ala Ser Glu Asp
   1 5 10 15
   Tyr Ile Gln Arg Ile Ala Gly Glu Thr Ala Gly Gln Pro Leu Asp Ala
   20 25 30
   Ile Ala Asp Arg Ile Thr Ala Leu Thr Ala Glu Asn Arg Ala Ile His
   35 40 45
   Glu Arg Asp Cys Val Asn Leu Asn Pro Ala Thr Asn Val Met Asn Pro
   50 55 60
   Lys Ala Glu Ala Leu Leu Ser Ala Gly Ile Gly Ala Arg Pro Ser Leu
   65 70 75 80
   Gly Tyr Pro Gly Asp Lys Tyr Glu Met Gly Leu Glu Ala Ile Glu Gln
   85 90 95
   Ile Glu Val Ile Ala Ala Glu Leu Ala Ala Glu Val Phe Gly Ala Thr
   100 105 110
   Tyr Ala Glu Ile Arg Val Pro Ser Gly Ala Ile Ala Asn Leu Tyr Ala
   115 120 125
   Phe Met Val Ala Ala Lys Ala Gly Asp Cys Ile Ile Ala Pro Pro Gly
   130 135 140
   Glu Ile Gly Gly His Val Thr His His Gly Ala Gly Ala Ala Gly Leu
   145 150 155 160
   Tyr Gly Ile Ile Thr His Pro Ala Pro Ile Asp Pro Val Lys Tyr Thr
   165 170 175
   Val Asp Val Glu Lys Leu Arg Ala Asp Ala Leu Arg Leu Arg Pro Lys
   180 185 190
   Leu Ile Ser Ile Gly Gly Ser Leu Asn Leu Phe Pro His Pro Ile Arg
   195 200 205
   Glu Ile Arg Thr Ile Ala Asp Glu Val Gly Ala Leu Val Leu Phe Asp
   210 215 220
   Ala Ala His Met Ser Gly Met Ile Ala Gly His Gly Trp Gln Gln Pro
   225 230 235 240
   Leu Glu Glu Gly Ala His Leu Met Thr Met Ser Thr Tyr Lys Ser Leu
   245 250 255
   Gly Gly Pro Pro Ser Gly Leu Ile Val Thr Asn Asp Ala Asp Ile Ala
   260 265 270
   Lys Lys Leu Asp Ala Ile Ala Tyr Pro Gly Leu Thr Ala Asn Phe Asp
   275 280 285
   Ala Ala Lys Ser Ala Ser Leu Ala Val Ser Leu Leu Asp Trp Lys Ala
   290 295 300
   His Gly Arg Ala Tyr Ala Gln Glu Met Ala Lys Thr Ala Lys Ala Leu
   305 310 315 320
   Ala Glu Ala Leu Ser Glu Arg Gln Val Pro Val Phe Ala Arg Asp Arg
   325 330 335
   Gly Met Thr Thr Ser His Gln Phe Ala Ile Glu Ala Ala Pro Tyr Gly
   340 345 350
   Gly Gly Gln Ala Ala Ala Lys Arg Leu Arg Ala Val Asn Ile Leu Ser
   355 360 365
   Cys Gly Ile Gly Leu Pro Leu Pro Ala Val Glu Gly Asp Val Asn Gly
   370 375 380
   Leu Arg Leu Gly Thr Pro Glu Ile Val Arg Phe Gly Met Thr Ala Ala
   385 390 395 400
   Asp Met Pro Glu Leu Ala Gly Tyr Ile Ala Glu Gly Leu Asn Gly Ser
   405 410 415
   Arg Pro Ala Glu Ala Val Ala Lys Asp Val Thr Ala Phe Arg Gly Arg
   420 425 430
   Phe Arg Glu Leu His Phe Met Arg
   435 440
<210> 24
<211> 28
<212> DNA
<213> Artificial
<220>
<223> primer
<400> 24
   ggggattaat gacggctctg ggcaggcg 28
<210> 25
<211> 28
<212> DNA
<213> Artificial
<220>
<223> primer
<400> 25
   aactgcagtc agcgcatgaa atgcagct 28
<210> 26
<211> 28
<212> DNA
<213> Artificial
<220>
<223> primer
<400> 26
   ccgaattcgg aggatggggc atgacggc 28
<210> 27
<211> 33
<212> DNA
<213> Artificial
<220>
<223> primer
<400> 27
   ccgaattcag gcagagatac ggaggatccc atg 33
<210> 28
<211> 34
<212> DNA
<213> Artificial
<220>
<223> primer
<400> 28
   aactgcagtc acgacctgat atggtgcatc ccct 34
<210> 29
<211> 1323
<212> DNA
<213> silicibacter pomeroyi
<220>
<221> CDS
<222> (1)..(1323)
<400> 29
   atg cca cag ctt gcc gcc cgc ccc tgg gtg ccc gcc cat tgc gaa acc      48
   Met Pro Gln Leu Ala Ala Arg Pro Trp Val Pro Ala His Cys Glu Thr
   1               5                   10                  15
   cgc gtt cag caa atc gcc gag acc acg gca cgc gcc gac agc gat gcc      96
   Arg Val Gln Gln Ile Ala Glu Thr Thr Ala Arg Ala Asp Ser Asp Ala
               20                  25                  30
   atc gac gcc cac ctg gag gcg ctg atc gaa gag aac cgc acc atc cat      144
   Ile Asp Ala His Leu Glu Ala Leu Ile Glu Glu Asn Arg Thr Ile His
           35                  40                  45
   gac gcg gaa tgt ttc aac ctg aac ccg gca acc aat gtt atg aac ccg      192
   Asp Ala Glu Cys Phe Asn Leu Asn Pro Ala Thr Asn Val Met Asn Pro
       50                  55                  60
   cgc gcc gag gcg gtg ctg gcg cgg gga ttg ggc agc cgc ccc tcg ctg      240
   Arg Ala Glu Ala Val Leu Ala Arg Gly Leu Gly Ser Arg Pro Ser Leu
   65                  70                  75                  80
   ggc tat ccc ggc gac aaa tac gag atg ggg ctg gag gcg atc gaa gag      288
   Gly Tyr Pro Gly Asp Lys Tyr Glu Met Gly Leu Glu Ala Ile Glu Glu
                   85                  90                  95
   atc gag gtg att gcc gca gaa ctg gcg gca aag gtg ttc aac gcg cgc      336
   Ile Glu Val Ile Ala Ala Glu Leu Ala Ala Lys Val Phe Asn Ala Arg
               100                 105                 110
   tat gcc gag atc cgg gtg ggc tcg ggc gcg ctc gcc aat ctt tat ggc      384
   Tyr Ala Glu Ile Arg Val Gly Ser Gly Ala Leu Ala Asn Leu Tyr Gly
           115                 120                 125
   ttc atg gcg ctg acc cgg ccc ggc gat acg atc atc gcg cca ccg gcc      432
   Phe Met Ala Leu Thr Arg Pro Gly Asp Thr Ile Ile Ala Pro Pro Ala
       130                 135                 140
   agt atc ggt ggc cat gta acc cat cac aag gcg ggc tgt gcg ggg ctt      480
   Ser Ile Gly Gly His Val Thr His His Lys Ala Gly Cys Ala Gly Leu
   145                 150                 155                 160
   tat ggc cta aaa acc atc gag gcg ccg gtg gat gcg gat ggt tac agc      528
   Tyr Gly Leu Lys Thr Ile Glu Ala Pro Val Asp Ala Asp Gly Tyr Ser
                   165                 170                 175
   ctg gat ttg agc gcg ctg gca gag ttg gca gag cgg cac cgg ccc cgg      576
   Leu Asp Leu Ser Ala Leu Ala Glu Leu Ala Glu Arg His Arg Pro Arg
               180                 185                 190
   ctg atc acg gtg ggc ggc tcg ctc aac ctg ttt ccg cat cca gtt gca      624
   Leu Ile Thr Val Gly Gly Ser Leu Asn Leu Phe Pro His Pro Val Ala
           195                 200                 205
   gcc gtg cgc gag att gcc gac agg gtt ggt gcc aag gtt ctg ttc gac      672
   Ala Val Arg Glu Ile Ala Asp Arg Val Gly Ala Lys Val Leu Phe Asp
       210                 215                 220
   gcg gcg cat caa tgc ggc atc atc gcg ggc ggc gcc tgg gcc aac ccg      720
   Ala Ala His Gln Cys Gly Ile Ile Ala Gly Gly Ala Trp Ala Asn Pro
   225                 230                 235                 240
   ctg gac gag ggg gcg cat ctg atg acg atg agc acc tac aag agc ctc      768
   Leu Asp Glu Gly Ala His Leu Met Thr Met Ser Thr Tyr Lys Ser Leu
                   245                 250                 255
   ggc ggc cct gcc ggc ggg ctg att gtg acc aac gag gcc gag atc gcc      816
   Gly Gly Pro Ala Gly Gly Leu Ile Val Thr Asn Glu Ala Glu Ile Ala
               260                 265                 270
   gaa cgg ctg gac gcc atc gcc ttt ccc ggc atg acc gcg aat ttc gac      864
   Glu Arg Leu Asp Ala Ile Ala Phe Pro Gly Met Thr Ala Asn Phe Asp
           275                 280                 285
   gcg gcg aaa tcg gcg gcg ttg gcg atc tcg ctg ttg gac tgg gtc gat      912
   Ala Ala Lys Ser Ala Ala Leu Ala Ile Ser Leu Leu Asp Trp Val Asp
       290                 295                 300
   cat ggc gcg gct tat gcc cag gcc atg gtc gat ctg gcg cag gcg ctg      960
   His Gly Ala Ala Tyr Ala Gln Ala Met Val Asp Leu Ala Gln Ala Leu
   305                 310                 315                 320
   gcc gcc gag ttg gag gcg ctg ggc ctg ccg gtg ttt cat ggc gcg ggc     1008
   Ala Ala Glu Leu Glu Ala Leu Gly Leu Pro Val Phe His Gly Ala Gly
                   325                 330                 335
   ggg gcc acc gcc tcg cac cag ttc gcg gtc gag gcg gcg cgg ttc ggc     1056
   Gly Ala Thr Ala Ser His Gln Phe Ala Val Glu Ala Ala Arg Phe Gly
               340                 345                 350
   ggc ggt cag gcg gcg tcc aag acg ctg agg cgg gca ggc ttc ctg gcc     1104
   Gly Gly Gln Ala Ala Ser Lys Thr Leu Arg Arg Ala Gly Phe Leu Ala
           355                 360                 365
   tgt ggc atc ggc ctg ccg atc gcg ccg gtt gcg ggg gat atg aac ggc     1152
   Cys Gly Ile Gly Leu Pro Ile Ala Pro Val Ala Gly Asp Met Asn Gly
       370                 375                 380
   ctt cgg atc ggc acg ccg gag ctg gtg cgc cga ggg gtc acg cca gag     1200
   Leu Arg Ile Gly Thr Pro Glu Leu Val Arg Arg Gly Val Thr Pro Glu
   385                 390                 395                 400
   cat gcc gcc gaa ctg gcc tgg ctg atc acc caa ggt ctc acc ggc aac     1248
   His Ala Ala Glu Leu Ala Trp Leu Ile Thr Gln Gly Leu Thr Gly Asn
                   405                 410                 415
   gat ccc gaa gcg gtg gcg ctg cgc acg cgt gag atg cgg gcg cgg ttt     1296
   Asp Pro Glu Ala Val Ala Leu Arg Thr Arg Glu Met Arg Ala Arg Phe
               420                 425                 430
   cag ggg atg cac cat atc agg tcg tga                                 1323
   Gln Gly Met His His Ile Arg Ser
           435                 440
<210> 30
<211> 440
<212> PRT
<213> Silicibacter pomeroyi
<400> 30
   Met Pro Gln Leu Ala Ala Arg Pro Trp Val Pro Ala His Cys Glu Thr
   1               5                   10                  15
   Arg Val Gln Gln Ile Ala Glu Thr Thr Ala Arg Ala Asp Ser Asp Ala
               20                  25                  30
   Ile Asp Ala His Leu Glu Ala Leu Ile Glu Glu Asn Arg Thr Ile His
           35                  40                  45
   Asp Ala Glu Cys Phe Asn Leu Asn Pro Ala Thr Asn Val Met Asn Pro
       50                  55                  60
   Arg Ala Glu Ala Val Leu Ala Arg Gly Leu Gly Ser Arg Pro Ser Leu
   65                  70                  75                  80
   Gly Tyr Pro Gly Asp Lys Tyr Glu Met Gly Leu Glu Ala Ile Glu Glu
                   85                  90                  95
   Ile Glu Val Ile Ala Ala Glu Leu Ala Ala Lys Val Phe Asn Ala Arg
               100                 105                 110
   Tyr Ala Glu Ile Arg Val Gly Ser Gly Ala Leu Ala Asn Leu Tyr Gly
           115                 120                 125
   Phe Met Ala Leu Thr Arg Pro Gly Asp Thr Ile Ile Ala Pro Pro Ala
       130                 135                 140
   Ser Ile Gly Gly His Val Thr His His Lys Ala Gly Cys Ala Gly Leu
   145                 150                 155                 160
   Tyr Gly Leu Lys Thr Ile Glu Ala Pro Val Asp Ala Asp Gly Tyr Ser
                   165                 170                 175
   Leu Asp Leu Ser Ala Leu Ala Glu Leu Ala Glu Arg His Arg Pro Arg
               180                 185                 190
   Leu Ile Thr Val Gly Gly Ser Leu Asn Leu Phe Pro His Pro Val Ala
           195                 200                 205
   Ala Val Arg Glu Ile Ala Asp Arg Val Gly Ala Lys Val Leu Phe Asp
       210                 215                 220
   Ala Ala His Gln Cys Gly Ile Ile Ala Gly Gly Ala Trp Ala Asn Pro
   225                 230                 235                 240
   Leu Asp Glu Gly Ala His Leu Met Thr Met Ser Thr Tyr Lys Ser Leu
                   245                 250                 255
   Gly Gly Pro Ala Gly Gly Leu Ile Val Thr Asn Glu Ala Glu Ile Ala
               260                 265                 270
   Glu Arg Leu Asp Ala Ile Ala Phe Pro Gly Met Thr Ala Asn Phe Asp
           275                 280                 285
   Ala Ala Lys Ser Ala Ala Leu Ala Ile Ser Leu Leu Asp Trp Val Asp
       290                 295                 300
   His Gly Ala Ala Tyr Ala Gln Ala Met Val Asp Leu Ala Gln Ala Leu
   305                 310                 315                 320
   Ala Ala Glu Leu Glu Ala Leu Gly Leu Pro Val Phe His Gly Ala Gly
                   325                 330                 335
   Gly Ala Thr Ala Ser His Gln Phe Ala Val Glu Ala Ala Arg Phe Gly
               340                 345                 350
   Gly Gly Gln Ala Ala Ser Lys Thr Leu Arg Arg Ala Gly Phe Leu Ala
           355                 360                 365
   Cys Gly Ile Gly Leu Pro Ile Ala Pro Val Ala Gly Asp Met Asn Gly
       370                 375                 380
   Leu Arg Ile Gly Thr Pro Glu Leu Val Arg Arg Gly Val Thr Pro Glu
   385                 390                 395                 400
   His Ala Ala Glu Leu Ala Trp Leu Ile Thr Gln Gly Leu Thr Gly Asn
                   405                 410                 415
   Asp Pro Glu Ala Val Ala Leu Arg Thr Arg Glu Met Arg Ala Arg Phe
               420                 425                 430
   Gln Gly Met His His Ile Arg Ser
           435                 440
<210> 31
<211> 40
<212> DNA
<213> Artificial
<220>
<223> primer
<400> 31
   gtatcacgag gccctagctg tggtgtcatg gtcggtgatc 40
<210> 32
<211> 40
<212> DNA
<213> Artificial
<220>
<223> primer
<400> 32
   ttcggggatt ccatatgata ccctttttac gtgaacttgc 40
<210> 33
<211> 38
<212> DNA
<213> Artificial
<220>
<223> primer
<400> 33
   ggggggggca tatgcgacct ccttattacg tgaacttg 38
<210> 34
<211> 30
<212> DNA
<213> Artificial
<220>
<223> primer
<400> 34
   ccggatccat gccacagctt gccgcccgcc 30

## Description

### TECHNICAL FIELD

The present invention relates to a method for producing beta (hereinafter, simply referred to as β)-hydroxy-α-L-amino acid and in particular, to a method for producing the L-serine derivative with a novel enzyme.

### BACKGROUND ART

Amino acids such as a serine derivative and amino acids having optical activity in an α position are substances expected to be used as intermediates for pharmaceuticals. Examples of methods for producing optically active α-alkyl serine derivatives, which are optically active amino acid derivatives having two different substituents in the α position, and salts thereof, include the following methods:
1) a method involving asymmetric alkylation of an optically active oxazolidine compound obtained from the optically active serine derivative and pivalaldehyde (Nonpatent Document 1);
2) a method involving asymmetric aldol reaction of α-isocyano carboxylic acid ester and paraformaldehyde with an optically active metal catalyst (Nonpatent Document 2);
3) a method involving asymmetric alkylation of optically active β-lactam compounds obtained from an optically active oxazolidine chromium carbene complex and an oxazine compound (Nonpatent Document 3);
4) a method involving asymmetric ring-opening reaction of an optically active aziridine compound (Nonpatent Document 4)
5) a method involving asymmetric alkylation of an optically active pyrazinone compound obtained from an optically active valine derivative and an optically active alanine derivative (Nonpatent Document 5); and
6) a method involving Sharpless asymmetric dihydroxylation of a 2-methyl-2-propenoic acid derivative followed by introduction of a resulting optically active diol compound into an optically active azido compound for reduction (Nonpatent document 6).

α-Methyl-L-serine is one of the promising substances which may be used as an intermediate of a medicament. In one of the known methods for producing α-methyl-L-serine by means of an enzymatic reaction, D-alanine and 5,10-methylenetetrahydrofolic acid are used as the materials, and 2-methyl serine hydroxymethyl transferase (EC 2.1.2.7) is used as the enzyme (Nonpatent document 7).

Nonpatent Document 1:Helvetica Chimica Acta, 1987, 70, 1194-1216
Nonpatent Document 2: Tetrahedron Letters, 1988, 29, 235-238
Nonpatent Document 3: Journal of Organic Chemistry, 1993, 58, 5918-5924
Nonpatent Document 4: Tetrahedron Letters, 1995, 36, 3639-3642
Nonpatent Document 5: European Journal of Organic Chemistry, 2000, 2809-2820
Nonpatent document 6: Tetrahedron Asymmetry, 2001, 12, 949-957
Nonpatent document 7: Wilson et al., J. Biol. Chem 237 3171-3179

US 3,755,081 describes a process for synthesizing L-serine, comprising the condensation of glycine and formaldehyde in the presence of threonin aldolase. The document does not relate to serine derivatives of formula III.

US 4,782,021 describes a process of producing L-serine including the reaction of glycine with formaldehyde in the presence of microorganisms of the genus *Escherichia* having the ability to produce serine hydroxymethyltransferase. The document does not relate to the production of serine derivatives of formula III.

US 5,346,828 discusses the stereoisomeric enrichment of enantiomeric D, L, mixtures of phenylpropionic acid derivatives using a threonin aldolase.

### DISCLOSURE OF INVENTION

### PROBLEM TO BE SOLVED BY THE INVENTION

As mentioned above, many studies have been conducted on a wide variety of methods for producing the optically active amino acids. However, there are lots of kinds of optically active amino acids and serine derivatives. A simpler method or an effective and low-cost method for producing optically active amino acids and serine derivatives is desired. An object of the present invention is to provide a new simpler method for producing the serine derivatives and optically active substance thereof, as well as enzymes used in the method.

### MEANS FOR SOLVING PROBLEM

Inventors of the preset invention had devoted to develop a novel method for producing the serine derivatives and they discovered a new protein, which catalyzes the reaction in a reaction system where L-amino acid and predetermined aldehyde are involved. In addition, the inventors discovered that this protein could be used to conveniently produce the serine derivatives and also selectively produce the L-serine derivative if a product was an amino acid having optical activity. A reaction system, in which the amino acid and formaldehyde were directly reacted under the absence of 5,10-methylenetetrahydro folic acid, was established. This type of reaction system has not been found before. The present invention is based on the knowledge of these discoveries by the inventors. The present invention provides the method for producing the L-serine derivative and enzymes used in the method, as mentioned below.
[1] A method for producing a L-serine derivative of formula (III): 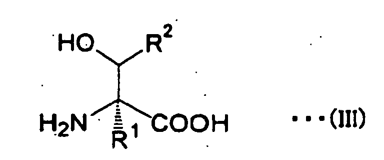 comprising:
   reacting L-α-amino acid of formula (I): 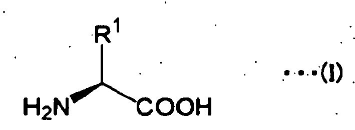 with aldehyde of formula (II): 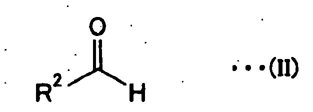 in the presence of an enzyme,
   wherein R¹ of formula (I) is selected from a group of an alkyl group with 1 to 6 carbons, an aryl group with 6 to 14 carbons, a cycloalkyl group with 3 to 10 carbons, an aralkyl group with 7 to 19 carbons, an alkoxyalkyl group with 2 to 11 carbons, a group containing a hetero atom in the carbon skeleton thereof, and a group containing a carbon-carbon unsaturated bond in the carbon skeleton thereof, and these groups may be either linear or branched and may have a substituent, and
   wherein R² of formula (II) is selected from a group of a hydrogen, an alkyl group with 1 to 6 carbons, an aryl group with 6 to 14 carbons, a cycloalkyl group with 3 to 10 carbons, an aralkyl group with 7 to 19 carbons, an alkoxyalkyl group with 2 to 11 carbons, a group containing a hetero atom in the carbon skeleton thereof, and a group containing a carbon-carbon unsaturated bond in the carbon skeleton thereof, and these groups may be either linear or branched and may have a substituent, and
   wherein R¹ of formula (III) is the same as R¹ of formula (I) and R² of formula (III) is the same as R² of formula (II)
   wherein said enzyme is one or more protein(s) selected from a group consisting of the following proteins (A) to (L):
      (A) a protein comprising the amino acid sequence of SEQ ID No: 5;
      (B) a protein comprising an amino acid sequence which has variation of one or 2 to 50 amino acid(s) in the amino acid sequence of SEQ ID NO: 5, wherein said variation is selected from a group consisting of substitution, deletion, insertion, addition and inversion, and having an activity catalyzing the reaction to form said L-serine derivative of formula (III);
      (C) a protein comprising the amino acid sequence of SEQ ID NO: 9;
      (D) a protein comprising an amino acid sequence which has variation of one or 2 to 50 amino acid(s) in the amino acid sequence of SEQ ID NO: 9, wherein said variation is selected from a group consisting of substitution, deletion, insertion; addition and inversion, and having an activity catalyzing the reaction to form said L-serine derivative of formula (III);
      (E) a protein comprising an amino acid sequence of SEQ ID NO: 15;
      (F) a protein comprising an amino acid sequence which has variation of one or 2 to 50 amino acid(s) in the amino acid sequence of SEQ ID NO: 15, wherein said variation is selected from a group consisting of substitution, deletion, insertion, addition and inversion, and having an activity catalyzing the reaction to form said L-serine derivative of formula (III);
      (G) a protein comprising the amino acid sequence of SEQ ID NO: 19;
      (H) a protein comprising an amino acid sequence which has variation of one or 2 to 50 amino acid(s) in the amino acid sequence of SEQ ID NO: 19, wherein said variation is selected from a group consisting of substitution, deletion, insertion, addition and inversion, and having an activity catalyzing the reaction to form said L-serine derivative of formula (III);
      (I) a protein comprising the amino acid sequence of SEQ ID NO: 23;
      (J) a protein comprising an amino acid sequence which has variation of one or 2 to 50 amino acid(s) in the amino acid sequence of SEQ ID NO: 23, wherein said variation is selected from a group consisting of substitution, deletion, insertion, addition and inversion, and having an activity catalyzing the reaction to form said L-serine derivative of formula (III);
      (K) a protein comprising the amino acid sequence of SEQ ID NO: 30; and
      (L) a protein comprising an amino acid sequence which has variation of one or 2 to 50 amino acid(s) in the amino acid sequence of SEQ ID NO: 30, wherein said variation is selected from a group consisting of substitution, deletion, insertion, addition and inversion, and having an activity catalyzing the reaction to form said L-serine derivative of formula (III).
[2] The method for producing the L-serine derivative according to the aforementioned [1], wherein said amino acid of (I) is L-α-alanine and said L-serine derivative of formula (III) is α-methyl-L-serine.
[3] The method for producing the L-serine derivative according to the aforementioned [1], wherein said amino acid of (I) is L-2-amino-n-butyric acid and said L-serine derivative of formula (III) is α-ethyl-L-serine.
[4] The method for producing the L-serine derivative according to the aforementioned [1], wherein said amino acid of (I) is L-α-alanine and said L-serine derivative of formula (III) is α-methyl-L-threonine.
[5] A protein having an activity catalyzing a reaction of a L-α-amino acid of formula (I): 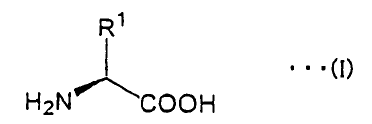 with aldehyde of formula (II) 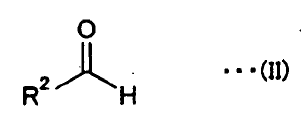 to produce a L-serine derivative of formula (III): 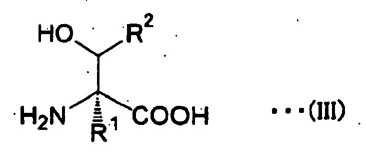
   wherein R² of formula (I) is selected from a group of an alkyl group with 1 to 6 carbons, an aryl group with 6 to 14 carbons, a cycloalkyl group with 3 to 10 carbons, an aralkyl group with 7 to 19 carbons, an alkoxyalkyl group with 2 to 11 carbons, a group containing a hetero atom in the carbon skeleton thereof, and a group containing a carbon-carbon unsaturated bond in the carbon skeleton thereof, and these groups may be either linear or branched and may have a substituent, and
   wherein R² of formula (II) is selected from a group of a hydrogen, an alkyl group with 1 to 6 carbons, an aryl group with 6 to 14 carbons, a cycloalkyl group with 3 to 10 carbons, an aralkyl group with 7 to 19 carbons, an alkoxyalkyl group with 2 to 11 carbons, a group containing a hetero atom in the carbon skeleton thereof, and a group containing a carbon-carbon unsaturated bond in the carbon skeleton thereof, and these groups may be either linear or branched and may have a substituent, and
   wherein R² of formula (III) is the same as R¹ of formula (I) and R² of formula (III) is the same as R² of formula (II) , and
   wherein said protein selected from a group consisting of the following proteins (A) to (L):
      (A) a protein comprising the amino acid sequence of SEQ ID No: 5;
      (B) a protein comprising an amino acid sequence which has variation of one or 2 to 50 amino acid(s) in the amino acid sequence of SEQ ID NO: 5, wherein said variation is selected from a group consisting of substitution, deletion, insertion, addition and inversion, and having an activity catalyzing the reaction to form said L-serine derivative of formula (III);
      (C) a protein comprising the amino acid sequence of SEQ ID NO: 9;
      (D) a protein comprising an amino acid sequence which has variation of one or 2 to 50 amino acid (s) in the amino acid sequence of SEQ ID NO: 9, wherein said variation is selected from a group consisting of substitution, deletion, insertion, addition and inversion, and having an activity catalyzing the reaction to form said L-serine derivative of formula (III);
      (E) a protein comprising an amino acid sequence of SEQ ID NO: 15;
      (F) a protein comprising an amino acid sequence which has variation of one or 2 to 50 amino acid(s) in the amino acid sequence of SEQ ID NO: 15, wherein said variation is selected from a group consisting of substitution, deletion, insertion, addition and inversion, and having an activity catalyzing the reaction to form said L-serine derivative of formula (III);
      (G) a protein comprising the amino acid sequence of SEQ ID NO: 19;
      (H) a protein comprising an amino acid sequence which has variation of one or 2 to 50 amino acid(s) in the amino acid sequence of SEQ ID NO: 19, wherein said variation is selected from a group consisting of substitution, deletion, insertion, addition and inversion, and having an activity catalyzing the reaction to form said L-serine derivative of formula (III);
      (I) a protein comprising the amino acid sequence of SEQ ID NO: 23;
      (J) a protein comprising an amino acid sequence which has variation of one or 2 to 50 amino acid(s) in the amino acid sequence of SEQ ID NO: 23, wherein said variation is selected from a group consisting of substitution, deletion, insertion., addition and inversion, and having an activity catalyzing the reaction to form said L-serine derivative of formula (III);
      (K) a protein comprising the amino acid.sequence of SEQ ID NO: 30; and
      (L) a protein comprising an amino acid sequence which has variation of one or 2 to 50 amino acid(s) in the amino acid sequence of SEQ ID NO: 30, wherein said variation is selected from a group consisting of substitution, deletion, insertion, addition and inversion, and having an activity catalyzing the reaction to form said L-serine derivative of formula (III).
[6] A polynucleotide encoding a protein of the aforementioned [5].
[7] A polynucleotide selected from a group consisting of the following (a) to (1) :
   (a) a nucleotide comprising the nucleotide sequence of SEQ ID NO: 4;
   (b) a polynucleotide which hybridizes with a polynucleotide having a nucleotide sequence complementary to that of SEQ ID NO: 4 under stringent conditions, and which encodes a protein having an activity catalyzing a reaction of an L-α-amino acid formula (I) with an aldehyde of formula (II) to produce an L-serine derivative in the formula (III);
   (c) a nucleotide comprising the nucleotide sequence of SEQ ID NO: 8;
   (d) a polynucleotide which hybridizes with a polynucleotide having a nucleotide sequence complementary to that of SEQ ID NO: 8 under stringent conditions, and which encodes a protein having an activity catalyzing a reaction of an L-α-amino acid formula (I) with an aldehyde of formula (II) to produce an L-serine derivative in the formula (III) ;
   (e) a nucleotide comprising the nucleotide sequence of SEQ ID NO: 14;
   (f) a polynucleotide which hybridizes with a polynucleotide having a nucleotide sequence complementary to that of SEQ ID NO: 14 under stringent conditions, and which encodes a protein having an activity catalyzing a reaction of an L-α-amino acid formula (I) with an aldehyde of formula (II) to produce an L-serine derivative in the formula (III);
   (g) a nucleotide comprising the nucleotide sequence of SEQ ID NO: 18;
   (h) a polynucleotide which hybridizes with a polynucleotide having a nucleotide sequence complementary to that of SEQ ID NO: 18 under stringent conditions, and which encodes a protein having an activity catalyzing a reaction of an L-α-amino acid formula (I) with an aldehyde of formula (II) to produce an L-serine derivative in the formula (III);
   (i) a nucleotide comprising the nucleotide sequence of SEQ ID NO: 22;
   (j) a polynucleotide which hybridizes with a polynucleotide having a nucleotide sequence complementary to that of SEQ ID NO: 22 under stringent conditions, and which encodes a protein having an activity catalyzing a reaction of an L-α-amino acid formula (I) with an aldehyde of formula (II) to produce an L-serine derivative in the formula (III);
   (k) a nucleotide comprising the nucleotide sequence of SEQ ID NO: 29; and
   (l) a polynucleotide which hybridizes with a polynucleotide having a nucleotide sequence complementary to that of SEQ ID NO: 29 under stringent conditions, and which encodes a protein having an activity catalyzing a reaction of an L-α-amino acid formula (I) with an aldehyde of formula (II) to produce an L-serine derivative in the formula (III);
      wherein stringent conditions mean conditions under which poly- nucleotides having 70% or more homology to the respective sequence hybridize, while the polynucleotides having lower homology do not hybridize, and wherein formula (I) is: 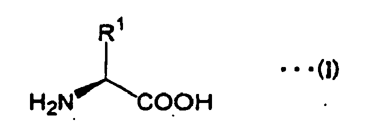 (R¹ of formula (I) is selected from a group of an alkyl group with 1 to 6 carbons, an aryl group with 6 to 14 carbons, a cycloalkyl group with 3 to 10 carbons, an aralkyl group with 7 to 19 carbons, an alkoxyalkyl group with 2 to 11 carbons, a group containing a hetero atom in the carbon skeleton thereof, and a group containing a carbon-carbon unsaturated bond in the carbon skeleton thereof, and these groups may be either linear or branched and may have a substituent);
      wherein said formula (II) is: 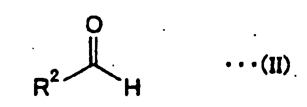 (R² of formula (II) is selected from a group of a hydrogen, an alkyl group with 1 to 6 carbons, an aryl group with 6 to 14 carbons, a cycloalkyl group with 3 to 10 carbons, an aralkyl group with 7 to 19 carbons, an alkoxyalkyl group with 2 to 11 carbons, a group containing a hetero atom in the carbon skeleton thereof, and a group containing a carbon-carbon unsaturated bond in the carbon skeleton thereof, and these groups may be either linear or branched and may have a substituent);
      wherein said formula (III) is: 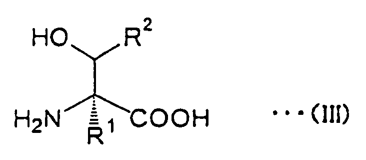 (wherein R¹ of formula (III) is the same as R¹ of formula (I) and R² of formula (III) is the same as R² of formula (II)).
[8] A recombinant polynucleotide having said polynucleotide according to the aforementioned [6] or [7] incorporated therein.
[9] A transformant having said polynucleotide according to the aforementioned [8] incorporated therein.

### EFFECT OF THE INVENTION

The present invention allows L-serine derivative to be produced by a simile method.
Moreover, the present invention allows L-amino acid to be selectively produced in producing the L-serine derivative having an optical activity, which provides an effective method for producing the L-amino acid.

### BRIEF DESCRIPTION OF DRAWINGS

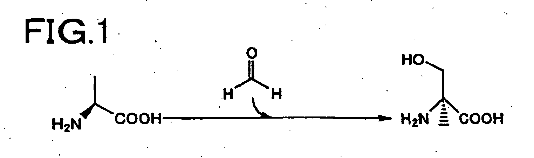

FIG. 1 is a scheme showing the reaction system according to one embodiment of the present invention.

### BEST MODE(S) OF CARRYING OUT THE INVENTION

Hereinafter, embodiments according to the present invention are described with reference to the best mode of carrying out the invention.
It should be note that various types of genetic engineering approaches are described in many standard experimental manuals, such as Molecular Cloning: A Laboratory Manual, 3rd edition, Cold Spring Harbor press (2001/01/15), Saibo Kogaku Handbook (Cellular Engineering Handbook), Toshio KUOKI et al., Yodosya (1992), and Shin Idenshi Kogaku Handbook (New Gene Engineering Handbook), 3rd edition, Matsumura et al., Yodosya (1999), by reference to which, any person skilled in the art may easily use these approaches. In the specification, SEQ ID Nos. refers to the sequence numbers in the sequence listing unless otherwise stated. In the specification, an enzyme is a protein having an activity for catalyzing a chemical reaction.

In the method of the present invention for producing the L-serine derivative, L-α-amino acid of formula (I), and aldehyde of formula (II) are reacted.

R¹ of formula (I) may specifically include the following:
Examples of R¹ as an alkyl group with 1 to 6 carbons may include a methyl group, an ethyl group, a n-propyl group, an isopropyl group, a n-butyl group, an isobutyl group, a sec-butyl group, a tert-butyl group, a n-pentyl group, an isopentyl group, a neo-pentyl group, a n-hexyl group, and an isohexyl group.

Examples of R¹ as an aryl group with 6 to 14 carbons may include a phenyl group, a tolyl group, a xylyl group, a biphenylyl group, a naphthyl group, an antolyl group, and a phenantolyl group.

Examples of R¹ as a cycloalkyl group with 3 to 10 carbons may include a cyclopropyl group, a cyclobutyl group; a cyclopentyl group, a cyclohexyl group, a cycloheptanyl group, a cyclooctanyl group, a cyclononanyl group, and a cyclodecanyl group.

Examples of R¹ as an aralkyl group with 7 to 19 carbons may include phenylalkyl groups such as a benzyl group, a benzhydryl group, a phenethyl group, and a trityl group, a cinnamyl group, a stylyl group, and a naphthylalkyl group.

Examples of R¹ as an alkoxyalkyl group with 2 to 11 carbons may include an alkyl group with 1 to 10 carbons which has a substituent selected from a group consisting of a methoxy group, an ethoxy group, a propoxy group, an isopropoxy group, a butoxy group, a pentyloxy group, a phenoxy group, a heptoxy group, an octoxy group, a nonanoxy group, a decanoxy group, and an undecoxy group.

R² may be a group which has a carbon skeleton containing a hetero atom in any of the aformetioned hydrocarbons. Examples of the hetero atom may include an oxygen atom, a nitrogen atom, and a sulfur atom.
One embodiment of R¹, which is the hydrocarbon group containing the hetero atom in its carbon skeleton, may include a heterocycle-containing hydrocarbon group. The heterocycle-containing hydrocarbon group is a cyclic hydrocarbon group, wherein a ring of the cyclic moiety contains the hetero atom. Examples of the heterocyclic hydrocarbon group may include a heteroaryl group with or without aromaticity and may be either monocyclic or polycyclic group. The specific examples of the heterocyclic hydrocarbon group may include a furilic group, a thienyl group, a pyridil group, a piperidil group, a piperidino group, a morpholino group, an indolyl group, an imidazolyl group, and an alkyl group substituted by any of these heterocyclic groups.

In addition, R¹ may also be a hydrocarbon group which has a carbon skeleton containing a unsaturated carbon-carbon bond in any of the aforementioned groups.
In addition, the aforementioned R¹ may be linear or branched. Moreover, R¹ may be the aforementioned hydrocarbon group which is partially substituted by the following group or to which the following group is partially added: one or more groups which may include a halogen atom, an alkyl group with up to 3 carbons, an alkoxyl group with up to 3 carbons, a keto group (=O), a hydroxyl group(-OH), a thiol group(-SH), an amino group(-NH₂) , an amido group (-CONH₂), an imino group (=NH), a hydrazino group(-NHNH₂).

Examples of the L-α-amino acid of formula (I) may include alanine, valine, leucine, isoleucine, serine, threonine, cysteine, methionine, asparagine, glutamine, phenylalanine, tyrosine, tryptophan, aspartic acid, glutamic acid, lysine, arginine, histidine, 2-amino-n-butyric acid, all of which are of L-α- type, preferably, alanine and 2-amino-n-butyric acid, and more preferably, alanine.

R² of formula (II) may be specifically described as follows.

R² may be hydrogen.
Examples of R² as an alkyl group with 1 to 6 carbons may include a methyl group, an ethyl group, a n-propyl group, an isopropyl group, a n-butyl group, an isobutyl group, a sec-butyl group, a tert-butyl group, a n-pentyl group, an isopentyl group, a neo-pentyl group, n-hexyl group, and an isohexyl group.

Examples of R² as an aryl group with 6 to 14 carbons may include a phenyl group, a tolyl group, a xylyl group, a biphenylyl group, a naphthyl group, an antolyl group, and a phenantolyl group.

In the condition that R² is a cycloalkyl group with 3 to 10 carbons, the concrete examples include a cyclopropyl group, a cyclobutyl group, a cyclopentyl group, a cyclohexyl group, a cycloheptanyl group, a cyclooctanyl group, a cyclononanyl group, and a cyclodecanyl group.

Examples of R² as an aralkyl group with 7 to 19 carbons may include phenylalkyl groups such as a benzyl group, a benzhydryl group, a phenethyl group, and a trityl group, a cinnamyl group, a stylyl group, and a naphthylalkyl group.

Examples of R² as an alkoxyalkyl group with 2 to 11 carbons may include an alkyl group with 1 to 10 carbons which has a substituent selected from a group consisting of a methoxy group, an ethoxy group, a propoxy group, an isopropoxy group, a butoxy group, a pentyloxy group, a phenoxy group, a heptoxy group, an octoxy group, a nonanoxy group, a decanoxy group, and an undecoxy group.

R² may be a group which has a carbon skeleton containing a hetero atom in any of aforementioned hydrocarbon groups. The examples of the hetero atom include an oxygen atom, a nitrogen atom, and a sulfur atom.
One embodiment of R², which is the hydrocarbon group containing the hetero atom in its carbon skeleton may includes a heterocycle-containing hydrocarbon group. The heterocycle-containing hydrocarbon group is a cyclic hydrocarbon group, wherein a ring of a cyclic moiety contains the hetero atom. Examples of the heterocycle-containing hydrocarbon group may include a heteroaryl group with or without aromaticity and may be either monocyclic or polycyclic group. The specific examples of the heterocyclic hydrocarbon group may include a furilic group, a thienyl group, a pyridil group, a piperidil group, a piperidino group, a morpholino group, an indolyl group, an imidazolyl group, and an alkyl group substituted by any of these heterocyclic groups.

In addition, R² may also be a hydrocarbon group which has a carbon skeleton containing a unsaturated carbon-carbon bond in any of the aforementioned groups.
In addition, the aforementioned R² may be linear or branched. Moreover, R² may be the aforementioned hydrocarbon group which is partially substituted by the following group or to which the following group is partially added: one or more groups selected from a group of a halogen atom, an alkyl group with up to 3 carbons, an alkoxyl group with up to 3 carbons, a keto group (=O), a hydroxyl group (-OH), a thiol group (-SH), an amino group(-NH₂), an amido group (-CONH₂) , an imino group (=NH) , a hydrazino group (-NHNH₂).

Examples of compounds of formula (II) may preferably include formaldehyde and acetaldehyde.

R¹ and R² of formula (III) are the same as R¹ and R² of formula (I) and (II), respectively. According to a specific combination of R¹ and R², the product may be an optically-inactive stereoisomer (mesoisomer). The method of the present invention relates to a method for producing an amino acid having an optical activity; therefore, the present invention does not include a combination of R¹ and R² which may form the production of the mesoisomer. For example, α-hydroxymethyl-serine is not L-serine derivative; therefore, the combination of serine as a compound of formula (I) and formaldehyde as a compound of formula (II) is not included in the method for producing the L-serine derivative of the present invention.

The method of the present invention for producing the L-serine derivative involves a process of preferentially producing the L-serine derivative by the reaction with an enzyme according to claim 1. Herein, the word "preferentially producing the L-serine derivative" means that the ratio of the L-serine derivative is higher than the ratio of a D-serine derivative among the serine derivatives to be produced,and the ratio is preferably 70% or more, more preferably 80% or more, and further preferably 90% or more. In this case, the ratio of the L-serine derivative among the L-serine derivatives is calculated by the formula: [L-serine derivativel/([D-serine derivative]+[L-serine derivative])*100.

One preferred embodiment of the method of the present invention includes a reaction system, in which α-methyl-L-serine is produced through the reaction of L-α-alanine and formaldehyde. FIG.1 shows a specific example of the reaction system. The reaction system, in which formaldehyde is reacted directly with the L-amino acid without involving a compound such as 5,10-methylentetrahydro folic acid to produce the L-serine derivative, has not been known at all. According to the method of the present invention, the L-serine derivative may be produced in a simpler reaction system. In the case of using formaldehyde, it is preferable that a small amount of formaldehyde is added sequentially in the reaction system. The sequential addition of formaldehyde may suppress generateing compounds such as byproducts.

Another preferable embodiment of the present invention may include reaction systems, in which α-ethyl-L-serine is produced through the reaction of L-2-amino-n-butyric acid and formaldehyde; and α-methyl-L-threonine is produced through the reaction of L-α-alanine and acetaldehyde.

A reaction temperature is preferably 10 to 60°C and more preferably 20 to 40°C. The pH value for the reaction system is preferably 4 to 10 and more preferably 6 to 8.

An example isolating L-serine derivative from a resulting solution of the aforementioned enzymatic reaction shows as follows. Dissolved proteins are flogulated by bringing pH value down along with thermal sterilization, and then microbes and proteins are removed from the solution by means such as centrifugation, filtration and ultra filtration (UF). Because the resulting solution includes inorganic salts, the solution is desalted to avoid precipitation of those salts at the step of crystallization. Applicable methods may include any method such as nanofiltration (NF), electrodialysis, and ion exchanging with resin.

A solution after the aforementioned desalting where necessary is concentrated. Then, L-serine derivative begins to crystallize; however, it often appears that due to too fine and highly solvable properties thereof to isolate, the ratio of recovering crystals is not allowed to be high, and high viscosity makes handling difficult. Therefore, a crystallization adding poor solvent is preferably conducted after the solution is preliminarily concentrated to some extent where necessary. As an example, the preliminary concentrating may be continued until crystals begin to be precipitated. Preferable examples of a poor solvent herein to be added may include lower alcohol and acetone, which are water soluble. A combination with cooling crystallisation after the crystallisation with the poor solvent may also contribute to improve the ratio of crystallization. The resulting slurry is isolated and wetting cake thereof is dried to obtain a crystal of L-serine derivative.

According to the methods of the present invention, aldehyde of formula (II) is reacted with the L-α-amino acid in the presence of a predetermined enzyme according to claim 1. The enzyme capable catalyzing the reaction may be obtained from a microorganism belonging to any of a genus such as *Ralstonia, Variovorax,* Bosea, and *Silicibacter.* More specific examples of the microorganism may include *Ralstonia* sp., *Variovorax paradoxus*, Bosea sp. and *Silicibacter pomeroyi;* preferably, Ralstonia sp. FERM ABP-10607, *Variovorax; paradoxus* FERM ABP-10608, *Variovorax paradoxus* NBRC 15149, *Variovorax paradoxus* NBRC 15150, *Bosea* sp. FERM ABP-10609 and *Silicibacter pomeroyi* DSM 15171.

Strains having a FERM or NBRC number assigned are deposited strains as mentioned below; therefore, they may be available by referencing to its associated number and following the given procedure.
(1) Nomenclature: Falstonia sp. All (or, AJ110405)
   Deposit Number: FERM ABP-10607
   Depositary authority: International Patent Organism Depositary, National Institute of Advanced Industrial Science and Technology
   Address: Chuoh No.6, Higashi 1-1-1, Tsukuba, Ibaraki, Japan Deposit date: March 8, 2005
(2) Nomenclature: *Varovorax paradoxus* B2-B2 (or, AJ110406 strain)
   Deposit Number: FERM ABF-10608
   Depositary authority: International Patent Organism Depositary, National Institute of Advanced Industrial Science and Technology
   Address: Chuoh No.6, Higashi 1-1-1, Tsukuba, Ibaraki, Japan Deposit date: March 8, 2005
(3) Nomenclature: *Variovorax paradoxus* AJ110408
   Deposit Number: NBRC15149
   Depositary authority: NITE Biological Resource Center Department of Biotechnology (NBRC), Department of Biotechnology (DOB), National Institute of Technology and Evaluation (NITE)
   Address: Kazusa Kamatari 2-5-8, Kisarazu, Chiba, Japan
(4) Nomenclature: *Variovorax paradoxus* AJ110409
   Deposit Number: NBRC15150
   Depositary authority: NITE Biological Resource Center Department of Biotechnology (NBRC), Department of Biotechnology (DOB), National Institute of Technology and Evaluation (NITE)
   Address: Kazusa Kamatari 2-5-8, Kisarazu, Chiba, Japan
(5) Nomenclature: Bosea sp. B2-R1 (or, AJ110407)
   Deposit Number: FERM ABP-10609
   Depositary authority: International Patent Organism Depositary, National Institute of Advanced Industrial Science and Technology
   Address: Chuoh No.6, Higashi 1-1-1, Tsukuba, Ibaraki, Japan Deposit date: March 8, 2005

Other than those strains, *Silicibacter pomeroyi* DSM 15171 has been accessible to public at Deusche Sammulung von Mikroorganismen und Zellkulturen GmbH (Address: Mascheroder Weg 1b, 38124 Braunschweig, GERMANY), and at American Type Culture Collection (Contact: P.O. Box 1549, Manassas, VA 20108, U.S.A.) as its reference number ATCC700808, as of the priority date of the present application.

Tables 1-1 and 1-2 describe the mycological characteristics of *Ralstonia* sp. All strain (or, AJ110405, Deposit NO: FERM ABP-10607).

**Table 1-1**

| *Ralstonia sp..* All | | |
|---|---|---|
| **1. Morphological characteristics** | | |
| Culture condition | | Nutrient agar (Oxoid, Hampshire, England) culture medium, 30°C |
| Cell morphology | | Short bacillus (0.6 x 0.8 µm) |
| Cell polymorphism | | - |
| Motility (flagellar immobility) | | - |
| Spore (sporal site) | | - |

| **2. Culture characteristics** | | |
|---|---|---|
| Culture condition | | Nutrient agar (Oxoid, Hampshire, England) culture medium, 30°C |
| Color | | Pale yellow |
| Luster | | + |
| Color development | | - |
| Culture condition | | Nutrient broth (Oxoid, Hampshire, England) culture medium, 30°C |
| Surface growth | | - |
| Cloudiness of culture medium | | + |
| Culture condition | | Gelatin stick culture 30°C |
| Growth condition | | + |
| Gelatin liquification¹⁾ | | - |
| Culture condition²⁾ | | Litmus milk 30°C |
| Coagulation | | - |
| Liquification | | - |

| **3. Physiological characteristics** | | |
|---|---|---|
| Gram stainability¹⁾ | | - |
| Nitrate reduction³⁾ | | - |
| denitrification³⁾ | | + |
| MR test²⁾ | | - |
| VP test³⁾ | | - |
| Indole production³⁾ | | - |
| Production of hydrogen sulfide³⁾ | | - |
| Starch hydrolysis²⁾ | | - |
| Use of citric acid²⁾ | (Koser) | + |
| | (Christensen) | + |
| Use of | Nitrate | + |
| inorganic nitrogen resource²⁾ | Ammonium salt | + |
| Urease activity³⁾ | | - |
| Catalase²⁾ | | + |
| Oxidase²⁾ | | + |
| Growth range; pH | 6 | + |
| | 8 | + |
| | 9 | +w |
| Growth range; temperature (°C) | 25 | + |
| | 30 | + |
| | 37 | +w |
| | 45 | - |
| Anaerobic growth | | - |
| O-F test (acidic/fermented)²⁾ | | -/- |

**Table 1-2**

| **4. Acid production/gas generation from saccharide²⁾** | | | |
|---|---|---|---|
| L-arabinose | -/- | D-xylose | -/- |
| D-glucose | -/- | D-mannose | -/- |
| D-fractose | -/- | D-galactose | -/- |
| maltose | -/- | sucrose | -/- |
| lactose | -/- | Trehalose | -/- |
| D-sorbitol | -/- | D-mannitol | -/- |
| inositol | -/- | Glycerin | -/- |

| **5. Other physiological characteristics²⁾** | | | |
|---|---|---|---|
| β-galactosidase activity | - | | |
| arginine dihydrolase activity | - | | |
| lysine decarboxylase activity | - | | |
| tryptophan deaminase activity | - | | |
| Gelatinase activity | - | | |

| | | | |
|---|---|---|---|
| +: positive, -: negative, w: weakly reacted | | | |

Tables 2-1 and 2-2 describe the mycological characteristics of *Variovorax paradoxus* B2-B2 (AJ110406 strain, Deposit NO: FERM ABP-10608).

**Table 2-1**

| *Variovorax paradoxus* B2-B2 | | |
|---|---|---|
| **1. Morphological characteristics** | | |
| Culture condition | | Nutrient agar (Oxoid, Hampshire, England) culture medium, 30°C |
| Cell morphology | | (0.6-07 x 1.5-2.0µm) |
| Cell polymorphism | | - |
| Motility (flagellar immobility) | | + |
| Spore (sporal site) | | - |

| **2. Culture characteristics** | | |
|---|---|---|
| Culture condition | | Nutrient agar (Oxoid, Hampshire, England) culture medium, 30°C |
| Color | | Yellow |
| Luster | | + |
| Color development | | + |
| Culture condition | | Nutrient broth (Oxoid, Hampshire, England) culture medium, 30°C |
| Surface growth | | - |
| Cloudiness of culture medium | | + |
| Culture condition | | Gelatin stick culture 30°C |
| Growth condition | | - |
| Gelatin liquification¹⁾ | | - |
| Culture condition²⁾ | | Litmus milk 30°C |
| Coagulation | | - |
| Liquification | | - |

| **3. Physiological characteristics** | | |
|---|---|---|
| Gram stainability¹⁾ | | - |
| Nitrate reduction³⁾ | | - |
| denitrification³⁾ | | + |
| MR test²⁾ | | - |
| VP test³⁾ | | - |
| Indole production³⁾ | | - |
| Production of hydrogen sulfide³⁾ | | - |
| Starch hydrolysis²⁾ | | - |
| Use of citric | (Koser) | - |
| acid²⁾ | (Christensen) | + |
| Use of | Nitrate | +w |
| inorganic nitrogen resource²⁾ | Ammonium salt | +w |
| Urease activity³⁾ | | - |
| Catalase²⁾ | | + |
| oxidase²⁾ | | - |
| Growth range; pH | 6 | + |
| | 8 | + |
| | 9 | + |
| Growth range; temperature (°C) | 25 | + |
| | 30 | + |
| | 37 | +w |
| | 45 | - |
| Anaerobic growth | | + |
| O-F test (acidic/fermented)²⁾ | | -/- |

**Table 2-2**

| **4. Acid production/gas generation from saccharide²⁾** | | | |
|---|---|---|---|
| L-arabinose | -/- | D-xylose | -/- |
| D-glucose | -/- | D- mannose | -/- |
| D-fractose | -/- | D- galactose | -/- |
| maltose | -/- | sucrose | -/- |
| lactose | -/- | Trehalose | -/- |
| D-sorbitol | -/- | D- mannitol | -/- |
| inositol | -/- | Glycerin | -/- |

| **5. Other physiological characteristics²⁾** | | | |
|---|---|---|---|
| β-galactosidase activity | - | | |
| arginine dihydrolase activity | - | | |
| lysine decarboxylase activity | - | | |
| tryptophan deaminase activity | - | | |
| Gelatinase activity | - | | |

| | | | |
|---|---|---|---|
| +: positive, -: negative, w: weakly reacted | | | |

Tables 3-1 and 3-2 describe the mycological characteristics of *Bosea* sp. B2-R1 strain (AJ110407, Deposit NO: FERM ABP-10609).

**Table 3-1**

| *Bosea* sp. B2-R1 | | |
|---|---|---|
| **1. Morphological characteristics** | | |
| Culture condition | | Nutrient agar (Oxoid, Hampshire, England) culture medium, 30°C |
| Cell morphology | | (0.6-07 x 1.5-2.0µm) |
| Cell polymorphism | | - |
| Motility (flagellar immobility) | | + |
| Spore (sporal site) | | - |

| **2. Culture characteristics** | | |
|---|---|---|
| Culture condition | | Nutrient agar (Oxoid, Hampshire, England) culture medium, 30°C |
| Color | | Pale yellow |
| Luster | | + |
| Color development | | - |
| Culture condition | | Nutrient agar (Oxoid, Hampshire, England) culture medium, 30°C |
| Surface growth | | - |
| Cloudiness of culture medium | | + |
| Culture condition | | Gelatin stick culture 30°C |
| Growth condition | | - |
| Gelatin liquification¹⁾ | | - |
| Culture condition²⁾ | | Litmus milk 30°C |
| Coagulation | | - |
| Liquification | | - |

| **3. Physiological characteristics** | | |
|---|---|---|
| Gram stainability¹⁾ | | - |
| Nitrate reduction³⁾ | | - |
| denitrification³⁾ | | - |
| MR test²⁾ | | - |
| VP test³⁾ | | - |
| Indole production³⁾ | | - |
| Production of hydrogen sulfide³⁾ | | - |
| Starch hydrolysis²⁾ | | - |
| Use of citric acid²⁾ | (Koser) | + |
| | (Christensen) | + |
| Use of | Nitrate | + |
| inorganic nitrogen resource²⁾ | Ammonium salt | +w |
| Urease activity³⁾ | | - |
| Catalase²⁾ | | + |
| Oxidase²⁾ | | + |
| Growth range; pH | 6 | + |
| | 8 | + |
| | 9 | + |
| Growth range; temperature (°C) | 25 | + |
| | 30 | + |
| | 37 | + |
| | 45 | - |
| Anaerobic growth | | + |
| O-F test (acidic/fermented)²⁾ | | -/- |

**Table 3-2**

| **4. Acid production/gas generation from saccharide¹⁾** | | | |
|---|---|---|---|
| L-arabinose | -/- | D-xylose | -/- |
| D-glucose | -/- | D- mannose | -/- |
| D-fractose | -/- | D- galactose | -/- |
| maltose | -/- | sucrose | -/- |
| lactose | -/- | Trehalose | -/- |
| D-sorbitol | -/- | D- mannitol | -/- |
| inositol | -/- | Glycerin | -/- |

| **5. Other physiological characteristics** | | | |
|---|---|---|---|
| β-galactosidase activity | - | | |
| arginine dihydrolase activity | - | | |
| lysine decarboxylase activity | - | | |
| tryptophan deaminase activity | - | | |
| Gelatinase activity | - | | |

| | | | |
|---|---|---|---|
| +: positive, -: negative, w: weakly reacted | | | |

References and kits used:
1) BARROW,(G.I.) and FELTHAM,(R.K.A):Cowan and Steel's Manual for the Identification of Medical Bacteria. 3rd edition 1993, Cambridge University Press.
2) Toshikazu SAKAZAKI, Etsuro YOSHIZAKI, and Kanji MIKI: Shin Saikin-baichi-gaku Kouza PART II(The Course of Culturing Medium for Microorganism PART II) <2nd edition>, 1988, Kindai Shuppan, Tokyo.
3) Bacteriopexy kit: API20, NE.
   (bioMerieux, France:http://www.biomerieux.fr/home_en.htm).

More specifically, the enzymes used in the reaction for producing the L-serine derivative in the present invention include the following proteins:
(A) a protein comprising the amino acid sequence of SEQ ID No: 5;
(B) a protein comprising an amino acid sequence which has variation of one or 2 to 50 amino acid (s) in the amino acid sequence of SEQ ID NO: 5, wherein said variation is selected from a group consisting of substitution, deletion, insertion, addition and inversion, and having an activity catalyzing the reaction to form said L-serine derivative of formula (III);
(C) a protein comprising the amino acid sequence of SEQ ID NO: 9;
(D) a protein comprising an amino acid sequence which has variation of one or 2 to 50 amino acid(s) in the amino acid sequence of SEQ ID NO: 9, wherein said variation is selected from a group consisting of substitution, deletion, insertion, addition and inversion, and having an activity catalyzing the reaction to form said L-serine derivative of formula (III);
(E) a protein comprising an amino acid sequence of SEQ ID NO: 15;
(F) a protein comprising an amino acid sequence which has variation of one or 2 to 50 amino acid(s) in the amino acid sequence of SEQ ID NO: 15, wherein said variation is selected from a group consisting of substitution, deletion, insertion, addition and inversion, and having an activity catalyzing the reaction to form said L-serine derivative of formula (III);
(G) a protein comprising the amino acid sequence of SEQ ID NO: 19;
(H) a protein comprising an amino acid sequence which has variation of one or 2 to 50 amino acid(s) in the amino acid sequence of SEQ ID NO: 19, wherein said variation is selected from a group consisting of substitution, deletion, insertion, addition and inversion, and having an activity catalyzing the reaction to form said L-serine derivative of formula (III);
(I) a protein comprising the amino acid sequence of SEQ ID NO: 23;
(J) a protein comprising an amino acid sequence which has variation of one or 2 to 50 amino acid(s) in the amino acid sequence of SEQ ID NO: 23, wherein said variation is selected from a group consisting of substitution, deletion, insertion, addition and inversion, and having an activity catalyzing the reaction to form said L-serine derivative of formula (III);
(K) a protein comprising the amino acid sequence of SEQ ID NO: 30; and
(L) a protein comprising an amino acid sequence which has variation of one or 2 to 50 amino acid(s) in the amino acid sequence of SEQ ID NO: 30, wherein said variation is selected from a group consisting of substitution, deletion, insertion, addition and inversion, and having an activity catalyzing the reaction to form said L-serine derivative of formula (III).

The use of any of aforementioned proteins may achieve the convenient production of the L-serine derivative. In particular, in the system where L-α-alanine reacts with formaldehyde, substantially α-methyl-L-serine only may be produced, the optically active amino acid being efficiently obtained.

The protein (A) having the amino acid sequence of SEQ ID NO: 5 may be isolated from the *Ralstonia* sp. FERM ABP-10607. The protein (C) having the amino acid sequence of SEQ ID NO: 9 may be isolated from the *Variovorax paradoxus* FERM ABP-10608. The protein (E) having the amino acid sequence of SEQ ID NO: 15 may be isolated from *Variovorax paradoxus* NBRC15149. The protein (G) having the amino acid sequence of SEQ ID NO: 19 may be isolated from *Variovorax paradoxus NBRC15150.* The protein (I) having the amino acid sequence of SEQ ID NO: 23 may be isolated from *Bosea* sp. FERM ABP-10609. The protein (K) having the amino acid sequence of SEQ ID NO: 30 may be isolated from *Silicibacter pomeroyi* DSM 15171.

The genome sequences of *Silicibacter pomeroyi* DSM 15171 had been disclosed and a part of the sequence of SEQ ID NO: 30 had been registered as a serine hydroxymethyltransferase (AAV96754.1). As a result of that the serine hydroxymethyltransferase (AAV96754.1) having the registered sequence was expressed with *Eshcerichia coli,* an objective protein was not detected and the resulting products did not have any activity of serine hydroxymethyltransferase. However, as a result of research by the inventers, it was discovered that nine amino acid residues at the further upstream of the registered sequence is necessary for activity of 2-methylserine hydroxymethyltransferase. The sequence of SEQ ID NO: 30 has a sequence in which the nine amino acid residues is added to the upstream of the registered sequence. As a result of expressing the sequence of SEQ ID NO: 30 with *Eshcerichia coli*, it was confirmed that an objective protein was detected, which having an activity of 2-methylserine hydroxymethyltransferase.

As mentioned above, according to the method of the present invention, proteins, which are substantially the same as the proteins (A), (C), (E), (G), (I), and (K) may be used. First, taking the protein (A) as an example, the protein (B), which is substantially the same as the protein (A) mentioned above, is provided. Herein, the term "a small number" indicates the number of amino acids to the extent where the protein structure and activity in an amino acid residue is not substantially affected, specifically 2 to 50, preferably 2 to 30, and more preferably 2 to 10, depending on the position of the amino acid residue in the protein structure and type of the amino acid residue. The amino acid sequence of the protein (B), which has variation of one or a small number of amino acid(s) in which the variation is selected from a group of substitution, deletion, insertion, addition, and inversion, preferably conserves approximately half or more of oxygen activity, preferably 80% or more, more preferably 90% or more, and further preferably 95% compared with the protein (A) under the condition of 30°C, (pH 7 to 8.

The variation in amino acid as described in the aforementioned (B) may be achieved by alternating the nucleotide sequence so that the amino acid at the specific site of the gene encoding the protein is substituted, deleted, inserted, or added using, e.g., the site-specific mutagenic method. Alternatively, the polynucleotide having the nucleotide sequence altered as mentioned above may be obtained through the known conventional mutation process. The mutation process includes an in vitro treatment of the DNA encoding the (A) with hydroxylamine or the like and a method, in which the microorganism belonging to genus *Escherichia,* which carries DNA encoding the (A) is treated by means of UV irradiation or with one of mutagenic agents commonly used for artificial mutation such as N-methyl-N'-nitro-N-nitrosoguanidine(NTG) and nitrous acid.

The aforementioned variation such as substitution, deletion, addition, and inversion also include naturally-occurring variation such as differences in species and strain of the microorganism. By expressing the DNA having the variation mentioned above in appropriate cells to examine the enzyme activity of the expressed products the DNA encoding the protein which is substantially the same protein as the protein (A), may be obtained.

Like the correspondence between the proteins (A) and (B), the protein (D) is an example of a protein substantially same as the protein (C); the protein (F) is an example of a protein substantially same as the protein (E); the protein (H) is an example of a protein substantially same as the protein (G); the protein (J) is an example of a protein substantially same as the protein (I) ; and the protein (L) is an example of a protein substantially same as the protein (K).

Example of the proteins which are substantially the same as the proteins (A), (C), (E), (G), (I) and (K) may include proteins which has an amino acid sequence with homology of preferably 70% or more, more preferably 80% or more, and further preferably 90% or more, with respect to each of the proteins. In the present specification, the homology of the amino acid sequence was obtained by calculating a Marching count indicating with percentage over the full-length of a polypeptide to be coded into ORF, using GENETYX software Ver7.0.9 (Genetics) with Unit Size to Compare=2, or by its equivalent calculation method.

The present invention provides polynucleotides encoding the aforementioned proteins. Due to codon degenerates, a certain amino acid sequence may be defined by more than one nucleotide sequence. That is, the polynucleotide of the present invention includes a polynucleotide having the nucleotide sequences encoding the aforementioned proteins (A), (B), (C), (D), (E), (F), (G), (H), (I), (J), (K) and (L).

Specifically, examples of the polynucleotide of the present invention may include the polynucleotides of the following (a), (c), (e), (g), (i), and (k):
(a) a polynucleotide comprising a nucleotide sequence of SEQ ID NO: 4;
(c) a polynucleotide comprising a nucleotide sequence of SEQ ID NO: 8;
(e) a polynucleotide comprising a nucleotide sequence of SEQ ID NO: 14;
(g) a polynucleotide comprising a nucleotide sequence of SEQ ID NO: 18;
(i) a polynucleotide comprising a nucleotide sequence of SEQ ID NO: 22; and
(k) a polynucleotide comprising a nucleotide sequence of SEQ ID NO: 29.

The polynucleotide (a), encoding the protein (A), may be isolated from the *Ralstonia* sp. FERM ABP-10607. The polynucleotide (c), encoding the protein (C), may be isolated from the *Variovorax paradoxus* FERM ABP-10608. The polynucleotide (e), encoding the protein (E), may be isolated from the *Variovorax paradoxus* NBRC15149. The polynucleotide (g), encoding the protein (G), may be isolated from the *Variovorax paradoxus* NBRC15150. The polynucleotide (i), encoding the protein (I), may be isolated from the *Bosea* sp. FERM ABP-10609. The polynucleotide (k), encoding the protein (K), may be isolated from the *Silicibacter pomeroyi* DSM 15171.

Taking the polynucleotide (a) as an example, a method for isolating polynucleotides is described. The DNA having the nucleotide sequence listed in the sequence of SEQ ID NO.4 may be obtained from a chromosome DNA or a DNA library of *Ralstonia* sp. by PCR (polymerase chain reaction, refer to White,T.J.et al;Trends Genet.,5,185 (1989)) or hybridization. A primer used for PCR may be designed based on, for example, the internal amino acid sequence determined by reference to a purified protein having an activity for catalyzing the reaction involved in the method of the present invention. Alternatively, the primer or a probe for hybridization may be designed based on the nucleotide sequence of SEQ ID NO: 4 or may be isolated using the probe. A combination of a primer having a sequence corresponding to a 5' non-translation region and a sequence corresponding to 3' non-translation region, between which a coding region lies, may be used for the primer for PCR to amplify the full-length of the protein coding region.

The primer may be synthesized in the usual manner, for example, by the phosphoamidite method (refer to Tetrahedron Letters (1981), 22, 1859) using DNA synthesizing equipment Model 380B (Applied Biosystems). The PCR process may be conducted using, for example, Gene Amp PCR System 9600 (PERKIN ELMER) and TaKaRa LA PCR in vitro Cloning Kit (TaKaRa Bio) according to the method specified by the suppliers including the manufacturers.

The polynucleotides, which are substantially the same as the aforementioned polynucleotides (a), (c), (e), (g), (i) and (k) are also included in the present invention. The following polynucleotide (b) is an example of a polynucleotide substantially same as the polynucleotide (a); the following polynucleotide (d) is an example of a polynucleotide substantially same as the polynucleotide (c); the following polynucleotide (f) is an example of a polynucleotide substantially same as the polynucleotide (e); the following polynucleotide (h) is an example of a polynucleotide substantially same as the polynucleotide (g); the following polynucleotide (j) is an example of a polynucleotide substantially same as the polynucleotide (i); and the following polynucleotide (1) is an example of a polynucleotide substantially same as the polynucleotide (k).

(b) a polynucleotide which hybridizes with a polynucleotide having a nucleotide sequence complementary to that of SEQ ID NO: 4 under stringent conditions, and which encodes a protein having an activity catalyzing a reaction of an L-α-amino acid formula (I) with an aldehyde of formula (II) to produce an L-serine derivative in the formula (III) ;
(d) a polynucleotide which hybridizes with a polynucleotide having a nucleotide sequence complementary to that of SEQ ID NO: 8 under stringent conditions, and which encodes a protein having an activity catalyzing a reaction of an L-α-amino acid formula (I) with an aldehyde of formula (II) to produce an L-serine derivative in the formula (III);
(f) a polynucleotide which hybridizes with a polynucleotide having a nucleotide sequence complementary to that of SEQ ID NO: 14 under stringent conditions, and which encodes a protein having an activity catalyzing a reaction of an L-α-amino acid formula (I) with an aldehyde of formula (II) to produce an L-serine derivative in the formula (III);
(h) a polynucleotide which hybridizes with a polynucleotide having a nucleotide sequence complementary to that of SEQ ID NO: 18 under stringent conditions, and which encodes a protein having an activity catalyzing a reaction of an L-α-amino acid formula (I) with an aldehyde of formula (II) to produce an L-serine derivative in the formula (III);
(j) a polynucleotide which hybridizes with a polynucleotide having a nucleotide sequence complementary to that of SEQ ID NO: 18 under stringent conditions, and which encodes a protein having an activity catalyzing a reaction of an L-α-amino acid formula (I) with an aldehyde of formula (II) to produce an L-serine derivative in the formula (III); and
(l) a polynucleotide which hybridizes with a polynucleotide having a nucleotide sequence complementary to that of SEQ ID NO: 29 under stringent conditions, and which encodes a protein having an activity catalyzing a reaction of an L-α-amino acid formula (I) with an aldehyde of formula (II) to produce an L-serine derivative in the formula (III).

For the polynucleotide to be hybridized, a probe, for example, may be used. In each case, the probe may be prepared in the usual manner based on the nucleotide sequences of SEQ ID Nos: 4, 8, 14, 18, 22, and 29. The objective polynucleotide may be isolated by picking up the nucleotide to be hybridized using the probe in the usual manner. The DNA probe, for example, may be prepared by amplifying the nucleotide sequences cloned into plasmid or a phage vector, cutting out the desired nucleotide sequence by a restriction enzyme, and then extracting the nucleotide sequence. The portion to be cut out may be adjusted according to the objective DNA. Once the aforementioned polynucleotide, which is substantially the same as any other nucleotide, has been detected, the polynucleotide may be amplified in the usual manner such as PCR.

The "stringent conditions" mean conditions under which a so-called specific hybrid is formed but a nonspecific hybrid is not formed. Although it is difficult to clearly define the condition in terms of numerical values, such conditions are those under which the DNAs having high homology, for example, 70% or more, more preferably 80% or more, further preferably 90% or more, and still further preferably 95% or more, are hybridized while the DNAs having lower homology are not hybridized. The homology (%) of the nucleotide sequences is represented by numeric values obtained by percentage calculation over the full-length of ORF of each gene (including a stop codon) using GENETYX software Ver7.0.9 (Genetics) with Unit Size to Compare=6, pick up location=1. As another example, stringent conditions may be those of ordinary washing conditions in Southern hybridization, under which the DNAs are hybridized at 60°C and salt concentration of 1 x SSC, 0.1% SDS, and preferably 0.1 x SSC, 0.1% SDS. The genes hybridized under such conditions may include a gene containing a stop codon or a gene without activity due to a mutation in the activity center region. However, these may be easily screened off by inserting the obtained genes in a commercially-available expression vector, expressing the genes in an appropriate host, and determining the enzyme activity of the expressed product by a method described later.

As mentioned above, desirably, the aforementioned polynucleotide (b) conserves approximately half or more of catalytic activity, preferably 80% or more, and more preferably 90% or more compared with the protein (A) having the amino acid sequence encoded by the nucleotide sequence of SEQ ID NO: 4 under the condition of 30°C, pH 8.0. Likewise, the aforementioned relationship is applicable to the aforementioned polynucleotide (d) in connection with the protein (C); to the aforementioned polynucleotide (f) in connection with the protein (E); to the aforementioned polynucleotide (h) in connection with the protein (G); to the aforementioned polynucleotide (j) in connection with the protein (I); and to the aforementioned polynucleotide (1) in connection with the protein (K).

According to the method of the present invention, the enzyme may be used in any form as long as it is capable of catalyzing the aforementioned reaction in the reaction system. Examples of the specific forms thereof may include a cultured product of enzyme-producing microorganism, cells of the microorganism separated from the cultured product, and a processed cell product. The cultured product of the microorganism is a product obtained by culturing the microorganism. More specifically, the cultured product may include a product such as a mixture containing the cells of the microorganisms, the cultivation medium used for culturing the microorganism, and the substances produced by the cultured microorganism. The cells of the microorganisms may be washed before using as the washed cells. The processed cell product may be disrupted, lysed, and/or freeze-dried cells, as well as a crude-purified protein that is collected from the processed cells, and a purified protein that is further purified. As for the purified proteins, a partially-purified protein which is obtained by a variety of types of purification methods may be used. Alternatively, a fixed protein thereof which is fixed by a covalent bond method, an adsorption method, or an entrapment method may be used. Depending on the employed microorganism, a part of the cells may be lysed during cultivation. In this case, the supernatant of the cultivation medium may also be used as an enzyme-containing substance.

Next, the method for producing the proteins of the present invention, and the method for preparing the recombinants and transformants used in producing the proteins will be described hereinbelow using the aforementioned protein (A) as an example. These methods of the present invention will be also applicable to other proteins.

The transformant which expresses the aforementioned protein (A) may be prepared using a previously prepared recombinant polynucleotide which contains the polynucleotide having any of the aforementioned nucleotide sequences incorporated therein. The transformant which expresses the aforementioned protein (A) may be obtained by, for example, preparing a recombinant DNA containing the DNA having the nucleotide sequence of SEQ ID NO: 4, and then introducing the resulting DNA into an appropriate host. Examples of the host for expressing the protein identified by the DNA having the nucleotide sequence of SEQ ID NO: 4 may include a variety of prokaryotic cells, including microorganisms belonging to genus *Escherichia* such as *Escherichia coli*, microorganisms belonging to genus *Corynebacterium, Bacillus subtilis*, and a variety of eukaryotic cells including *Saccharomyces cerevisiae, Pichia stipitis,* and *Aspergillus oryzae.* Using the host which may be easily handled without any expensive components upon cultivation, L-serine derivative may be easily produced on a large scale.

The recombinant DNA for introducing the DNA having the nucleotide sequence listed in the sequence listing under SEQ ID NO.4 into a host may be prepared by inserting the DNA into a vector suitable for the type of the host so that the inserted DNA can express the protein encoded thereby. If the promoter inherently existing with the gene encoding the aforementioned enzyme derived from microorganisms such as *Ralstonia* sp., *Variovorax paradoxus,* and *Bosea* sp. are capable of functioning in the host cell, such a promoter may be used as the promoter for facilitating the expression of the protein. Alternatively, if necessary, any other promoter which may function in the host may be coupled to the DNA such as the DNA of SEQ ID NO: 4 so that the proteins are expressed under the control of the promoter.

A method for transforming the recombinant DNA to introduce into the host cell may include the D.M.Morrison method (Methods in Enzymology 68, 326 (1979)) and a method for improving the permeability of the DNA by treating a recipient strain cell with calcium chloride (Mandel,M. and Higa, A., J. Mol. Biol., 53, 159 (1970)).

In the case where the objective proteins are put in large-scale production using the recombinant DNA technology, the mode, in which the proteins associate together to form protein inclusion bodies within the transformant producing the proteins, is also one of the best modes for carrying out the present invention. The advantages of this expression production method may be protection of the objective protein from digestion due to protease in the microbial cells, and ready purification of the objective protein that may be performed by disruption of the microbial cells and following centrifugation. To obtain the active protein from the protein inclusion body, a series of manipulations such as solubilization and activity regeneration is required, and thus, the manipulations are more complicated than those used when directly producing the active protein. However, when a protein which affects microbial cell growth is produced on a large scale in the microbial cells, effects thereof may be avoided by accumulating the protein as an inactive inclusion body in the microbial cells.

Examples of the methods for producing the objective protein on a large scale as an inclusion body may include methods of expressing the objective protein alone under the control of a strong promoter, as well as methods of expressing the objective protein as a fusion protein with a protein known to be expressed in a large amount.

As the host to be transformed, any strain commonly used in expressing heterogenes may be used. Suitable examples thereof may include the *Escherichia coli* JM109, DH5α, HB101, and BL21(DE3) strains, which are subspecies of the *Escherichia coli* K12 strain. The method for transforming the host and the method for selecting out the transformants are described in Molecular Cloning: A Laboratory Manual, 3rd edition, Cold Spring Harbor press (2001/01/15). An example of the method for preparing the transformed *Escherichia coli* strain and producing a predetermined enzyme using the transformed *E*. *coli* strain will be specifically described hereinbelow.

As the promoter for expressing the DNA encoding the protein having catalytic activity used for the present invention, the promoters typically used for producing xenogenic proteins in *E*. *coli* may be used, and examples thereof may include strong promoters such as T7 promoter, *lac* promoter, *trp* promoter, *trc* promoter, *tac* promoter, and PR promoter and PL promoter, T5 promoter of lambda phage. As the vector, pUC19, pUC18, pBR322, pHSG299, pHSG298, pHSG399, pHSG398, RSF1010, PACYC177, pACYC184, pMW119, pMW118, pMW219, pMW218, pQE30, and derivatives thereof may be used. Other vectors of phage DNA may also be used. In addition, expression vectors which contain a promoter and can express the inserted DNA sequence may also be used.

In order to produce the protein being used for the present invention in the form of a fusion protein inclusion body, a fusion protein gene is prepared by linking a gene encoding the second protein, preferably a hydrophilic peptide at corresponding upstream or downstream part of the aforementioned protein. Such a gene encoding another protein may be those which increase the amount of the accumulated fusion protein and enhance solubility of the fusion protein after denaturation and regeneration steps. Examples of candidates thereof may include T7 gene 10, β-galactosidase gene, dehydrofolic acid reductase gene, interferon y gene, interleukin-2 gene and prochymosin gene.

Such a gene may be ligated to the gene encoding the protein so that reading frames of codons are matched. This may be achieved by ligating at an appropriate restriction enzyme site or using a synthetic DNA having an appropriate sequence.

In some cases, it is preferable to ligate a terminator, i.e. the transcription termination sequence, downstream of the fusion protein gene in order to increase the production amount. Examples of this terminator may include T7 terminator, fd phage terminator, T4 terminator, tetracycline resistant gene terminator, and *E. coli trpA* gene terminator.

The vector for introducing the gene encoding the protein having catalytic activity or the fusion protein thereof into E. *coli* may preferably be of a so-called multicopy type. Examples thereof may include plasmids having a replication origin derived from ColEl, such as pUC based plasmids, pBR322 based plasmids or derivatives thereof. As used herein, the "derivative" means the plasmid modified by the substitution, deletion, insertion, addition and/or inversion of bases. "Modified" referred to herein includes the modification by mutagenesis with the mutagen or UV irradiation and natural mutation.

In order to select the transformants, it is preferable to employ a vector having a marker such as an ampicillin resistant gene. As such a plasmid, expression vectors having the strong promoter are commercially available (pUC series: Takara Bio Co., Ltd., pPROK series and pKK233-2: Clontech, etc.).

A DNA fragment where the promoter, the gene encoding the protein having the objective activity or the fusion protein of the objective protein with the other protein, and in some cases the terminator are ligated sequentially, is then ligated to the vector DNA to obtain a recombinant DNA.

The resulting recombinant DNA is used to transform *Escherichia coli,* and then the transformed *Escherichia coli* is cultured, to express and produce the predetermined protein or its fused protein.

In the case of expressing the fusion protein, the fusion protein may be composed so as to be able to cleave out the objective protein therefrom using a restriction protease which recognizes a sequence not present in the objective protein, such as blood coagulation factor Xa, kallikrein.

As production media, media such as M9-casamino acid medium and LB medium which are typically used for cultivation of *E*. *coli* may be used. The conditions for cultivation and a production induction may be appropriately selected depending on types of the marker and the promoter of the vector and the host used.

The following methods are available for recovering the objective protein or the fusion protein containing the objective protein. If the objective protein or the fusion protein thereof is solubilized in the microbial cells, the cells may be collected and then disrupted or lysed to thereby obtain a crude enzyme solution for use. If necessary, the crude solution may be purified using techniques such as ordinary precipitation, filtration and column chromatography, to obtain the purified objective protein or the fusion protein. In this case, the purification may be performed using an antibody against the objective protein or the fusion protein. In the case where the protein inclusion body is formed, it may be solubilized with a denaturant, and then the denaturant may be removed by means of dialysis or the like to obtain the objective protein.

### EXAMPLES

The present invention will be described in more detail with reference to the following non-limiting examples.

### Example 1: Detection of 2-methyl serine hydroxylmethyl transferase activity

In a Nutrient Broth agar medium (Difco), microorganisms listed in Table 4 were cultured at 30°C for 24 hours. A platinum loopful of the resulting cells were inoculated into 3 ml of Nutrient Broth liquid medium and then cultured at 30°C for 24 hours, with 120 reciprocations/minute. 0.15 ml of the resulting cultured mixture was inoculated into 3 ml of Nutrient Broth liquid medium containing 0.2% α-methyl-DL-serine and cultured at 30°C for 24 hours with 120 reciprocations/minute.

After cultivation, the cells were centrifuged and then washed twice with an equal volume of 50 mM potassium phosphate buffer (pH 7.4) containing 0.1 mM pyridoxal phosphate. 50 mM potassium phosphate buffer (pH 7.4) containing 0.1 mM pyridoxal phosphate was used to prepare a total amount (0.3 ml) of cell suspension, and then the suspension was ultrasonically disrupted at 4°C. Supernatant obtained by centrifugation (16,000 g, 10 min.) was dialyzed with 50 mM potassium phosphate buffer (pH 7.4) containing 0.1 mM pyridoxal phosphate to obtain a cell-free extracted solution.

0.05 ml of the cell-free extracted solution was added in a reaction mixture-1, which has a composition composed of 50 mM potassium phosphate buffer (pH 7.4), 10 mM α-methyl-DL-serine, 0.1 mM pyridoxal phosphate, and 1 mM magnesium sulfate, and then a total amount (0.1 ml) of mixture was reacted at 30°C for 10 minutes. The reaction was stopped by mixing 0.1 ml of alkaline agent (5N-potassium hydroxide) supplied with the Formaldehyde Kit-Test Wako (Wako). Subsequently, the formaldehyde detection reaction was executed by reference to the manual supplied with the kit to measure absorbance at 550 nm (E11). As a control, another reaction was performed in the same way as the above, except that α-methyl-DL-serine was replaced with water in the aforementioned solution-1, and the absorbance (E10) of the resulting mixture was measured. An alteration in adsorption specific to α-methyl-DL-serine was calculated (EΔ1=E11-E10). The results are shown in Table 4. As the results shows, 2-methyl serine hydroxymethyl transferase activity was confirmed. Table 4.

| Strain | EΔ1 |
|---|---|
| *Ralstonia* sp. All | 0.70 |
| *Variovorax paradoxus* B2-B2 | 0.86 |
| *Bosea* sp. B2-R1 | 0.42 |

### Example 2: Purification of 2-methyl serine hydroxymethyl transferase derived from the Ralstonia sp. A11 (AJ110405) strain

### (1) Preparation of Cell-Free Extracted Solution

Cells of *Ralstonia* species were cultured in the Nutrient Broth agar medium (Difco) at 30°C for 25.5 hours. The cultured cells were inoculated into 50 ml of Nutrient Broth liquid medium contained in a 500 ml Sakaguchi flask, and cultured at 30°C for 21 hours, 120 reciprocation /minute. The resulting culture mixture was inoculated into 2 L of liquid medium containing 0.2% α-methyl-DL-serine, 0.17% Yeast Nitrogen Base w/o amino acid and ammonium sulfate (pH 7.0). 100 ml each of the mixture was dispensed into 500 ml Sakaguchi flasks, and then cultured at 30°C for 24 hours, 120 reciprocation/minute. The resulting cells were collected by means of centrifugation (8,000g, 10 minutes) and washed twice with 25 mM Tris-HCl buffer (pH 8.0) containing 0.02 mM pyridoxal phosphate, and then 50 ml of cell suspension was prepared. The cells were ultrasonically disrupted and centrifuged (18,000g, 10 minutes), and the resulting supernatant was ultra-centrifuged (200,000g, 30 minutes). The resulting supernatant was dialyzed using the same buffer, thus obtaining a cell-free extracted solution.

### (2) Anion-exchange Chromatography

The cell-free extracted solution obtained in the aforementioned (1) was applied in ResourceQ columns (Amersham Biosciences), which had been previously put in the equilibrium state with the 25 mM tris-HCl buffer (pH8.0) containing 0.02 mM pyridoxal phosphate, and the enzyme was eluted by a linear concentration gradient of 0-1M sodium chloride. This process was conducted three times by dividing the cell-free extracted solution into three aliquots.

### (3) Hydrophobic Interaction Chromatography

Active fractions of the enzyme obtained in the aforementioned (2) were dialyzed in the 25 mM tris-HCl buffer (pH7.4) containing 0.02 mM pyridoxal phosphate (hereinafter, simply referred to as the buffer I), and mixed with the buffer I containing an equivalent amount of 2M ammonium sulfate, and then applied in the Phenyl-Sepharose columns (Amersham Biosciences) which had been previously put in the equilibrium state with the buffer I containing 1M ammonium sulfate. Then, the enzyme was eluted by the linear concentration gradient of 1-0M ammonium sulfate.

### (4) Hydroxyapatite Column Chromatography

The active fractions obtained in the aforementioned (3) were dialyzed with the 2.5 mM potassium phosphate buffer (pH 7.0) containing 0.02 mM pyridoxal phosphate, and then applied in the CellulofineHAp columns (SEIKAGAKU Corp.) which had been previously put in the equilibrium state with the same buffer. The enzyme was eluted in the 2.5-500 mM potassium phosphate buffer (pH7.0).
The active fractions of the enzyme having 0.72U/mg of relative activity, which were obtained in this manner, were electrophoresed with SDS-polyacrylamide, and the gel was stained with Coomassie brilliant blue staining fluid. A homogeneous band appeared at a position of approximately 47,000 of molecular weight.

### Example 3: Determination of the amino acid sequence and the encoding nucleotide sequence of 2-methyl serine hydroxymethyl transferase derived from the Ralstonia sp. A11 (AJ110405) strain

The product corresponding 100 pmol purified enzyme, which had been prepared in Example 2, were electrophoresed in SDS-polyacrylamide, transcribed on a PVDF membrane, and then put in a protein sequencer to determine 30 amino acids (SEQ ID NO: 1).

Second, 5 µg of genome DNA derived from the Ralstonia sp. AJ110405 was cleaved with SalI(75U), and then ligated to SalI cassettes in line with the method described in the manual supplied with TaKaRa LA PCR in vitro Cloning Kit. Using the ligated mixture as a template, PCR (94°C: 30 sec., 47°C: 2 min., 72°C: 1 min., 30 cycles) was performed with a combination of a cassette primer C1 and a primer ALD_RV_S1 (SEQ ID NO: 2). Subsequently, using the PCR reaction mixture as a template, the second PCR (94°C: 30 sec., 55°C: 2-min., 72°C: 1 min., 30 cycles) was performed with a cassette primer C2 and a primer ALD_RV_S2 (SEQ ID NO: 3). Approximately 0.7 kb-length fragments, of which amplification had been confirmed, were ligated to pGEM-Teasy (Promega) to transform *Escherichia coli* JM109. Defining the nucleotide sequence of approximately 0.7 kb of fragments, the nucleotide sequence encoding the N terminal amino acid sequence of the objective protein was detected. Using approximately 0.3 kb-length gene fragments obtained by treating the plasmid with EcoRI/SphI as a probe, chromosome DNAs were subject to Southern analysis after treatment with various types of restriction enzymes. When the chromosome DNAs were treated with *Sph*I, positive a signal was confirmed in an approximately 2.4 kb region.

Subsequently, the chromosomal DNAs were treated with *SphI,* and then electrophoresed in an agarose gel, to purify the approximately 2.5 kb fragment. The fragment was then ligated to the pUC18 *SphI* site. Using this reaction mixture, *Escherichia coli* JM109 was transformed to create a library. The aforementioned probe was used to perform colony hybridization for obtaining positive colonies. A plasmid was extracted from the positive colonies. Using the resulting plasmid as pSKA04098, the nucleotide sequences of inserted sequence were determined. ORF (SEQ ID NO.4) encoding 438 amino acids was found.

### Example 4: Expression of 2-methyl serine hydroxymethyl transferase gene derived from the Ralstonia sp. All (AJ110405) in Escherichia coli

Using pSKA04098 as a template, PCR was performed with a primer Ral_Eco (SEQ ID NO: 6) and a primer Ral_ter_Pst (SEQ ID NO: 7) to amplify a 1.3 kb region of 2-methyl serine hydroxymethyl transferase. The amplified sequence was treated with EcoRI/PstI, and then ligated to pUC18 which had been previously treated with EcoRI/PstI, to transform *Escherichia coli* JM109. The transformant having plasmid (pRal2) containing the objective gene fragments was obtained and designated JM109/pRal2.

JM109/pRal2 was pre-cultured in the LB culture medium containing 100mg/l ampicillin at 37°C for 16 hours. 0.15 ml of pre-cultured solution was inoculated into the 3 ml LB culture medium containing 100mg/l ampicillin and cultured at 37°C. One hour after the onset, IPTG was added so that the final concentration thereof reached 1 mM, and then the mixture was further subjected to culturing for four hours. The resulting cells were collected by centrifugation and washed with the 50 mM phosphoric acid buffer (pH 7.4) containing 0.1 mM pyridoxal phosphate. A cell suspension was then prepared using 0.3 mL of the same buffer. The cells were ultrasonically disrupted and centrifuged (18,000g, 10 min., 4°C) to obtain a supernatant as a cell-free extracted solution. 2-methyl serine hydroxymethyl transferase activity was measured as to the supernatant. The measured value was 0.03 U/mg. On the other hand, in the case of using a cell-free extracted solution obtained in line with the aforementioned method using the transformant JM109/pUC18, which is a transformant obtained by introducing pUC18 into JM109, the measured activity thereof was lower than the detection limit.

### Example 5: Production of α-methyl-L-serine with 2-methyl serine hydroxymethyl transferase Derived from Ralstonia sp. All (AJ110405)

50 µl of purified enzyme solution prepared in Example 2 was added in a solution composed of 100 mM formaldehyde, 100 mM L-alanine, 0.1 mM pyridoxal phosphate, and 100 mM phosphoric acid buffer (pH7.4). The reaction was performed at 30°C for 20 hours. As formaldehyde, the highest quality of formaldehyde liquid product [code No.: 16223-55] from Nakarai Tesk was used. After the reaction, 100 µl of 1 mM aqueous copper sulfate and 50 µl of water were added into 50 µl of the reaction mixture, and then HPLC analysis was performed using Sumichiral OA-6100 (Sumitomo Kagaku Analysis Center)(mobile phase: 0.5 mM aqueous copper sulfate, column temperature: 30°C, flow rate: 1 ml/min., detection: UV215nm). The result showed that 27.5 mM α-methyl-L-serine was produced but no peak attributed to α-methyl-D-serine was detected. As a reference standard, α-methyl-L-serine [Code NO: 29001-2500], α-methyl-D-serine [Code NO: 29002-2500] from Acros Organics were used.

### Example 6: Production of α-methyl-L-serine under the mediation of 2-methyl serine hydroxymethyl transferase gene-expressed Escherichia coli derived from Ralstonia sp. All (AJ110405)

JM109/pRal2 was cultured in the LB medium containing 100 mg/l at 30°C for 24 hours, and the further cultured in the LB medium containing 100 mg/l and 0.5 mM IPTG at 34°C for 16 hours. The cells obtained in 400 ml of the cultured medium were collected by centrifugation, and then washed with the 100 mM phosphoric acid buffer (pH7.4) containing 0.1 mM pyridoxal phosphate. The resulting cells were suspended in 100 ml of reaction mixture (300 mM L-alanine, 0.1 mM pyridoxal phosphate, 100 mM phosphoric acid buffer (pH7.4)). 50.5 ml of 600 mM aqueous formaldehyde was added in the reaction mixture at 30°C over 24 hours while stirring. As formaldehyde, the highest quality of formaldehyde liquid product [code No.: 16223-55] from Nakarai Tesk was used. After the adding process, 50 µl of 1 mM aqueous copper sulfate and 100 µl of water were added into 50 µl of the reaction mixture, and HPLC analysis was performed using Sumichiral OA-6100 (Sumitomo Kagaku Analysis Center)(mobile phase: 0.5 mM aqueous copper sulfate, column temperature: 30°C, flow rate: 1 ml/min., detection: UV215nm). The result showed that 27.0 mmol α-methyl-L-serine was produced but no peak attributed to α-methyl-D-serine was detected.

### Example 7: Acquisition of 2-methyl serine hydroxymethyl transferase gene derived from Variovorax paradoxus B2-B2 (AJ110406)

In line with the same manner described in Example 3, an ORF domain derived from Ralstonia sip. AJ110405 was amplified by PCR. Using the resulting PCR product as a probe, genome DNA of *Variovorax paradoxus* B2-B2 was subject to Southern analysis after treatment with PstI. A positive signal was found in a region of approximately 2 kb length.

Subsequently, the genome DNA of the *Variovorax paradoxus* B2-B2 was treated with PstI and electrophoresed in the agarose gel. Approximately 2 kb of fragments were purified and ligated to the pUC118 PstI site. Using this reaction mixture, *Escherichia coli* JM109 was transformed to create a library. The aforementioned probe was used to hybridize colonies to collect positive colonies. A plasmid was extracted from the positive colonies. Using the resulting plasmid as pUCB2-B2, the nucleotide sequence of the inserted sequence was determined. Existence of ORF composed of 441 amino acids (SEQ ID NO.8) was confirmed.

Subsequently, Using pTV118N (Takara Bio) as a template, a primer pTV118N_Nde (SEQ ID NO.10) and pTV118N_Ndec (SEQ ID NO: 11) were used to obtain pTV118Nd using Quikchange site direct mutagenesis kit (Promega). The amplified fragment of 1.2 kb was obtained by PCR with a primer B2-B2_Nde (SEQ ID NO: 12) and a primer B2-B2_ter_Pst (SEQ ID NO.13) using pUCB2-B2 as a template. The resulting fragment was treated with *Nde*I/*Pst*I and inserted into pTV118Nd NdeI/PstI site, which was designated pTVVHMT01. Using this plasmid, *Escherichia coli* JM109 was transformed. The transformant was designated JM109/pTVVHMT01.

JM109/pTVVHMT01 was pre-cultured in the LB medium containing 100mg/l ampicillin and 0.1 mM IPTG at 37°C for 16 hours. The resulting cells were collected by centrifugation and washed with 50 mM phosphoric acid buffer (pH 7.4) containing 0.1 mM pyridoxal phosphate, and then a cell suspension was prepared with the same buffer. The cells were ultrasonically disrupted and centrifuged (18,000 g, 10 min., 4°C) to obtain a supernatant as a cell-free extracted solution. 2-methyl serine hydroxymethyl transferase activity was measured as to the supernatant. Generated formaldehyde was determined after reaction in a reaction mixture containing 50 mM Tris-HCl buffer (pH8.5), 10 mM α-methyl-L-serine, and 0.1 mM pyridoxal phosphate at a reaction temperature 30°C. The measured value was 9 mU/mg. On the other hand, in the case of using a cell-free extracted solution obtained in line with the aforementioned method using the transformant JM109/pTV118Nd, which is a transformant obtained by introducing pTV118Nd into JM109, the measured activity thereof was lower than the detection limit.

Example 8: Acquisition of 2-methyl serine hydroxymethyl transferase genes derived from the *Variovorax paradoxus* NBRC15149
In line with the same manner described in Example 3, an ORF domain derived from *Ralstonia* sp. AJ110405 was amplified by PCR. Using the resulting PCR product as a probe, genome DNA of *Variovorax paradoxus* NBRC 15149 was subjected to Southern analysis after treatment with PstI. A positive signal was found in a 2 kb-length region.

Subsequently, the genome DNA of the *Variovorax paradoxus* NBRC 15149 was treated with PstI and electrophoresed in the agarose gel. Approximately 2 kb of fragments were purified and ligated to the pUC118 PstI site. Using this reaction mixture, *Escherichia coli* JM109 was transformed to create a library. The aforementioned probe was used to hybridize colonies to collect positive colonies. A plasmid was extracted from the positive colonies. Using the resulting plasmid as pUC15149, the nucleotide sequence of the inserted sequence was determined. Existence of ORF composed of 440 amino acids (SEQ ID NO: 14) was confirmed. Using pUC15149 as a template, PCR was performed with a primer 15149_Nde (SEQ ID NO: 16) and a 15149_ter_Pst (SEQ ID NO: 17). The amplified fragment of 1.2 kb was obtained by PCR. The resulting fragment was treated with *Nde*I/*Pst*I, and inserted into pTV118Nd *Nde*I/*Pst*I site, which was designated pTWHMT02. Using this plasmid, *Escherichia coli* JM109 was transformed. The transformant was designated JM109/pTVVHMT02.

JM109/pTVVHMT02 was pre-cultured in the LB medium containing 100mg/l ampicillin and 0.1 mM IPTG at 37°C for 16 hours. The resulting cells were collected by centrifugation and washed with 50 mM phosphoric acid buffer (pH7.4) containing 0.1 mM pyridoxal phosphate, and then a cell suspension was prepared with the the same buffer. The cells were ultrasonically disrupted and centrifuged (18,000 g, 10 min., 4°C) to obtain a supernatant as a cell-free extracted solution. 2-methyl serine hydroxymethyl transferase activity was measured as to the supernatant. The measured value was 13 mU/mg.

### Example 9: Acquisition of 2-methyl serine hydroxymethyl transferase genes derived from the Variovorax paradoxus NBRC15150 strain

In accordance with the same manner described in Example 3, an ORF domain derived from *Ralstonia* sp. AJ110405 was amplified by PCR. Using the resulting PCR product as a probe, genome DNA of *Variovorax paradoxus* NBRC 15150 was subject to Southern analysis after treatment with PstI. A positive signal was found in a 2 kb-length region.

Subsequently, the genome DNA of the *Variovorax paradoxus* NBRC 15150 was treated with *Pst*I and electrophoresed in the agarose gel. Approximately 2 kb of fragments were purified and ligated to the pUC118 PstI site. Using this reaction mixture, *Escherichia coli* JM109 was transformed to create a library. The aforementioned probe was used to hybridize colonies to collect positive colonies. A plasmid was extracted from the positive colonies. Using the resulting plasmid as pUC15150, the nucleotide sequence of the inserted sequence was determined. Existence of ORF composed of 440 amino acids (SEQ ID NO: 18) was confirmed. Using pUC15150 as a template, PCR was performed with a primer 15150_Nde (SEQ ID NO: 20) and a primer 15150_ter_Pst (SEQ ID NO: 21). The amplified fragment of 1.2 kb was obtained by PCR. The resulting fragment was treated with *Nde*I/*Pst*I, and inserted into pTV118Nd *Nde*I/*Pst*I site, which was designated pTVVHMT03. Using this plasmid, *Escherichia coli* JM109 was transformed. The transformant was designated JM109/pTVVHMT03.

JM109/pTVVHMT03 was pre-cultured in the LB medium containing 100mg/l ampicillin and 0.1 mM IPTG at 37°C for 16 hours. The resulting cells were collected by centrifugation and washed with 50 mM phosphoric acid buffer (pH7.4) containing 0.1 mM pyridoxal phosphate, and then a cell suspension was prepared with the same buffer. The cells were ultrasonically disrupted and centrifuged (18,000 g, 10 min., 4°C) to obtain a supernatant as a cell-free extracted solution. 2-methyl serine hydroxymethyl transferase activity was measured as to the supernatant. The measured value was 36 mU/mg.

### Example 10: Acquisition of 2-methyl serine hydroxymethyl transferase genes derived from the Bosea sp. B2-R1 (AJ110407) strain

In line with the same manner described in Example 3, an OFR domain derived from *Ralstonia* sp. AJ110405 was amplified by PCR. Using the resulting PCR product as a probe, genome DNA of *Bosea* sp. B2-R1 was subject to Southern analysis after treatment with *Pst*I. A positive signal was found in a 5 kb-length region.

Subsequently, the genome DNA of the *Bosea sp.* B2-R1 strain was treated with *Pst*I and electrophoresed in the agarose gel. Approximately 5 kb of fragments were purified and ligated to the pUC118 *Pst*I site. Using this reaction mixture, *Escherichia coli* JM109 was transformed to create a library. The aforementioned probe was used to hybridize colonies to collect positive colonies. A plasmid was extracted from the positive colonies. Using the resulting plasmid as pUCB2-R1, the nucleotide sequence of the inserted sequence was determined. Existence of ORF composed of 440 amino acids (SEQ ID NO: 22) were confirmed. Using pUCB2-R1 as a template, PCR was performed with a primer B2-R1_Psh (SEQ ID NO: 24) and a primer B2-R1_ter_Pst (SEQ ID NO: 25). The amplified fragment of 1.2 kb was obtained by PCR. The resulting fragments was treated with *Psh*BI/*Pst*I, and inserted into pTV118 *Nde*I/*Pst*I site, which was designated pTVBHMT. Using this plasmid, *Escherichia coli* JM109 was transformed. The transformant was designated JM109/ pTVBHMT. Using pUCB2-R1 as a template, PCR was performed with a primer B2-R1_Eco (SEQ ID NO: 26) and a B2-R1_ter_Pst (SEQ ID NO: 25). The amplified fragment of 1.2 kb was obtained by PCR. The resulting fragment was treated with *Eco*RI/*Pst*I, and inserted into pUC18 EcoRI/PstI site, which was designated pUCBHMT. Using this plasmid, *Escherichia coli* JM109 was transformed. The transformant was designated JM109/pUCBHMT.

JM109/pTVBHMT was pre-cultured in the LB medium containing 100mg/1 ampicillin and 0.1 mM IPTG at 37°C for 16 hours. The resulting cells were collected by centrifugation and washed with 50 mM phosphoric acid buffer (pH7.4) containing 0.1 mM pyridoxal phosphate, and then a cell suspention was prepared with the same buffer. The cells were ultrasonically disrupted and centrifuged (18,000 g, 10 min., 4°C) to obtain a supernatant as a cell-free extracted solution. 2-methyl serine hydroxymethyl transferase activity was measured as to the supernatant. The measured value was 95 mU/mg. JM109/pUCBHMT was pre-cultured in the LB medium containing 100 mg/l ampicillin and 0.1 mM IPTG at 37°C for 16 hours. The resulting cells were collected by centrifugation and washed with 50 mM phosphoric acid buffer (pH 7.4) containing 0.1 mM pyridoxal phosphate, and then a cell suspention was prepared with the same buffer. The cells were ultrasonically disrupted and centrifuged (18,000 g, 10 min., 4°C) to obtain a supernatant as a cell-free extracted solution. 2-methyl serine hydroxymethyl transferase activity was measured as to the supernatant. The measured value was 318 mU/ mg.
In the case of using a cell-free extracted solution obtained in line with the aforementioned method using the JM109/pUC18, the measured activity thereof was lower than the detection limit.

### Example 11: Acquisition of 2-methyl serine hydroxymethyl transferase gene derived from Silicibacter pomeroyi DSM15171

Using genome DNA of *Silicibacter pomeroyi* DSM15171 as a template, the amplified fragment of 1.3 kb including SEQ ID NO: 29 was obtained by PCR with a primer Silici ATG EcoRI (SEQ ID NO: 27) and Silici_ter_Pst (SEQ ID NO: 28). The resulting fragment was treated with *Eco*RI/*Pst*I, and inserted into pUC18 *Eco*RI/*Pst*I site, which was designated pUCSHMT. Using this plasmid, *Escherichia coli* JM109 was transformed. The transformant was designated JM109/pUCSHMT.

JM109/pUCSHMT was pre-cultured in the LB medium containing 100mg/l ampicillin and 0.1 mM IPTG at 37°C for 16 hours. The resulting cells were collected by centrifugation and washed with 50 mM phosphoric acid buffer (pH7.4) containing 0.1 mM pyridoxal phosphate, and then a cell suspension was prepared with the same buffer. The cells were ultrasonically disrupted and centrifuged (18,000 g, 10 min. 4°C) to obtain a supernatant as a cell-free extracted solution. 2-methyl serine hydroxymethyl transferase activity was measured as to the supernatant. Separated formaldehyde was determined after reaction in a reaction mixture containing 50 mM Tris-HCl buffer (pH 7.4), 10 mM α-methyl-L-serine, and 0.1 mM pyridoxal phosphate at a reaction temperature 30°C. The measured value was 190 mU/mg.

Subsequently, using genome DNA of *Silicibacter pomeroyi* DSM15171 as a template, the PCR product of approximately 1.3 kb length including SEQ ID NO: 29 was obtained by PCR with a primer Silici ATG pQE30 BamHI (SEQ ID NO: 34) and Silici_ter_Pst (SEQ ID NO: 28). The resulting product was digested with *Bam*HI-*Pst*I to prepare DNA fragments, and the fragment was inserted into pQE30 *Bam*HI-*Pst*I site, thus obtaining a plasmid which was capable of forming 2-methyl serine hydroxymethyl transferase coupled with 6 x His at the 5' terminal thereof. The plasmid was designated pQE30SHMT. Using this plasmid, *Escherichia coli* JM109 was transformed. The transformant was designated JM109/pQE30SHMT.

The obtained JM109/pQE30SHMT was cultured in the LB medium containing 100mg/l ampicillin (LB + amp) at 30°C for 14 hours, and further cultured for three hours after adding 1 mM IPTG. The resulting cells were collected by centrifugation and washed with 50 mM phosphoric acid buffer (pH 7.4) containing 0.1 mM pyridoxal phosphate, and then a cell suspention was prepared with the same buffer. The cells were ultrasonically disrupted and centrifuged (18,000 g, 10 min., 4°C) to obtain a supernatant using QIAexprssionist (QIAGEN) in accordance with the attached protocol thereof. The obtained protein was designated His fused SHMT.

Using the obtained His fused SHMT, a reaction was performed in a 100 µL solution containing 10 mM formaldehyde, 100 mM L-alanine, 0.1 mM pyridoxal phosphate, 100mM phosphoric acid buffer (pH 7.4) at 30°C for 10 min. As formaldehyde, the highest quality of formaldehyde liquid product [code No.: 16223-55] from Nakarai Tesk was used. After the reaction, 200 µl of 1 mM aqueous copper sulfate was added into 100 µl of the reaction mixture, and HPLC analysis was performed using Sumichiral OA-6100 (Sumitomo Kagaku Analysis Center)(mobile phase: 0.5 mM aqueous copper sulfate, column temperature: 30°C, flow rate: 1 ml/min., detection: UV215nm). The result showed that 0.367 mM α-methyl-L-serine was produced but no peak attributed to α-methyl-D-serine was detected.

### Example 12: Production of α-methyl-L-serine with Escherichia coli Expressing 2-methyl serine hydroxymethyl transferase Gene Derived from Variovorax paradoxus strain

JM109/pTWHMT01, JM109/pTWHMT02, and JM109/pTVVHMT03 prepared in line with the aforementioned example were respectively pre-cultured in the LB medium containing 100mg/l ampicillin and 0.1 mM IPTG at 37°C for 16 hours. The resulting cells were collected from 100ml medium by centrifugation and washed with 100 mM phosphoric acid buffer (pH7.4) containing 0.1 mM pyridoxal phosphate.

The resulting cells were respectively suspended in 100 ml of a reaction mixture (150 mM L-alanine, 0.1. mM pyridoxal phosphate, 100 mM phosphoric acid buffer (pH7.4)). 50.5 ml of 300 mM aqueous formaldehyde was added in the reaction mixture at 30°C over 24 hours while stirring. As formaldehyde, the highest quality of formaldehyde liquid product [code No.: 16223-55] from Nakarai Tesk was used. After the adding process, 50 µl of 1 mM aqueous copper sulfate and 100 µl of water were added into 50 µl of the reaction mixture, and HPLC analysis was performed using Sumichiral OA-6100 (Sumitomo Kagaku Analysis Center) (mobile phase: 0.5 mM aqueous copper sulfate, column temperature: 30°C, flow rate: 1 ml/min., detection: UV215nm). The results were shown in Talbe 5. It was confirmed that α-methyl-L-serine was produced but no peak attributed to α-methyl-D-serine was detected.

**Table 5**

| Strain | Yield of α-methyl-L-serine (mmol) |
|---|---|
| JM109/pTVVHMT01 | 1.2 |
| JM109/pTWHMT02 | 5.3 |
| JM109/pTVVHMT03 | 13.7 |

### Example 13: Preparation of a Strain Expressing 2-methyl serine hydroxymethyl transferase derived from Bosea sp. B2-R1 (AJ110407)

Fragments of 0.3 kb comprising a promoter region of trp operon on the chromosome DNAs of *Eshcherichia coli* W3110 was amplified with oligonucleotides of SEQ ID NO: 31 and NO: 32 as primers, and then the resulting DNA fragments were ligated into pGEM-Teasy vector (Promega). In the ligation solution, E. coli JM109 was transformed,and a strain which contained a objective plasmid, in which trp promoter had been inserted in the opposite direction of lac promoter, was selected from ampicillin-resistant strains. Subsequently, this plasmid was ligated with a DNA fragment comprising trp promoter treated by *Eco*O109I/*Eco*RI and a product obtained by treating pUC19 (Takara) with *Eco*O109I/*Eco*RI. In the ligation solution, *E. coli* JM109 was transformed, and a strain containing a objective plasmid was selected from ampicillin-resistant strains. This plasmid was treated with *Hind*III/*Pvu*II and the resulting DNA fragment was ligated with 0.7 kb of DNA fragment which prepared by treating pKK223-3 (Amersham Pharmacia) with *Hind*III/*Hin*cI and comprised TrrnB terminater. Using this ligation solution, *Eshcherichia coli* JM109 was transformed, and a strain containing a objective plasmid was selected from ampicillin-resistant strains. The plasmid was designated ptrp2. Using this ptrp2 as a template, fragments of 0.3 kb comprising trp promoter was amplified with primers (SEQ ID NO: 31 and 33). In order to replace the promoter region of the ptrp2 with the resulting PCR product, a fragment prepared by digesting the resulting PCR product with *Eco*O109I/*Nde*I was ligated with a product prepared by treating ptrp2 with *Eco*O109I/*Nde*I. Using resulting products, *Eshcherichia coli* JM109 was transformed, and a strain containing a objective plasmid was selected from ampicillin-resistant strains. This plasmid was designated ptrp4. Subsequently, pTWV228 (TaKaRa) was treated with NdeI, and then the terminal thereof was made blunt-ended. The resulting product was treated with AatII, obtaining a fragment of 1.9 kb. The resulting fragment was ligated with a 0.4 kb with fragment prepared by treating ptrp4 *Aat*II/*Hind*III and a 0.7 kb fragment prepared by treating pKK223-3 with *Pvu*II/*Hin*dIII. Using the resulting ligation solution, *Escherichia coli* JM109 was transformed, and a strain containing a objective plasmid was selected from ampicillin-resistant strains. This plasmid was designated ptrp13.

Using genome DNA of *Bosea* sp. B2-R1 as a template, PCR was performed with a primer B2-R1_Psh (SEQ ID NO: 24) and a primer B2-R1_ter_Pst (SEQ ID NO: 25). The amplified fragment of 1.2 kb was obtained by PCR. The resulting fragment was treated with *Psh*BI/*Pst*I, and inserted into ptrp13 *Nde*I/*Pst*I site, which was designated ptrp13BHMT. Using this plasmid, *Escherichia coli* JM109 was transformed. The transformant was designated JM109/ ptrp13BHMT.

JM109/ptrp13BMT mentioned above was cultured on a LB agar medium (10 g/l peptone, 5 g/l yeast extract, 10 g/l NaCl) containing 100 µg/ml ampicillin at 30°C for 24 hours. Subsequently, cells corresponding to one eighth of a plate was transferred to 50 mL LB medium (10 g/l peptone, 5 g/l yeast extract, 10 g/l NaCl). A cultured mixture was prepared by culturing at 30°C for 16 hours with a shaker (120 rpm). Subsequently, 1 ml of the resulting cultured mixture was inoculated to a 300 ml medium containing the following composition, and then a batch culture was performed in a 1.0-liter volume fermentor for laboratory under the condition of stirring at revolution speed 700 rpm, and aeration (1/1 vvm). After sugar was consumed, an 15 ml aliquot of the cultured mixture was inoculated in a 300 ml medium composed of the same composition, and then a culture was performed in the same type of fermentor under the condition of stirring, aeration (1/1vvm), sugar feeding and at 35°C. pH thereof was automatically adjusted to 7.0 by ammonium gas.

### Composition of the Medium (g/1)

Glucose 25.0
MgSO₄•7H₂O 1.0
(NH₄)₂SO₄ 5.0
H₃PO₄ 3.5
FeSO₄ 7aq 0.05
InsO₄ 7aq 0.05 Thiamine HCl 0.001
Pyridoxyne HCl 0.01
GD113 0.1
Ampicillin 0.1
Glucose and magnesium sulfate were individually sterilized. The pH of other elements was adjusted at 5.0 with KOH.

### Composition of Feeding Sugar Solution (g/l)

Glucose 500.0
pH Not adjusted

### Example 14: Production of α-methyl-L-serine with 2-methyl serine hydroxymethyl transferase Derived from Bosea sp. B2-R1 (AJ110407)

Using the 30 ml of the resulting cultured mixture of JM109/ptrp13BHMT prepared in accordance with the aforementioned Example 13, the reaction to produce α-methyl-L-serine with 2-methyl serine hydroxymethyl transferase was conducted. 150 ml of 2400mM hormaldehyde aqueous solution was added into the 300 ml cultured mixture (1200 mM L-alanine, 0.1 mM pyridoxal phosphate, and 100 mM phosphoric acid buffer (pH 7.4), 10 % of the cultured mixture of JM109/ptrp13BHMT) at 30°C over 48 hours while stirring. As formaldehyde, the highest quality of formaldehyde liquid product [code No.: 16223-55] from Nakarai Tesk was used. After the adding process, 950 µl of 1 mM aqueous copper sulfate was added to 50 ml of the resulting solution mixture. After 5-fold dilution by adding water, HPLC analysis was performed using Sumichiral OA-6100 (Sumitomo Kagaku Analysis Center)(mobile phase: 0.5 mM aqueous copper sulfate, column temperature: 30°C, flow rate: 1 ml/min., detection: UV215nm). The result showed that 327.3 mmol α-methyl-L-serine was produced but no peak attributed to α-methyl-D-serine was detected.

### Example 15: Purification of α-methyl-L-serine

The cells are roughly removed from the resulting reacting mixture prepared in accordance with Example 14. 3.3 g sulfuric acid was added to the 451 g resulting reaction mixture (α-methyl-L-serine: 9.08%) to adjust to pH 3, and then dissolved proteins were flocculated by heating at 50°C for 1 hour. The resulting product was filtered with 0.2 µm MF, obtaining 482 g solution (α-methyl-L-serine: 8.45%, D-alanine: 0.13%, L-alanine: 0.08%) including residual washing water. 165 g of this solution was diluted with water to be 5% α-methyl-L-serine. After the dilution, the solution was fed into a resin column filled with 120 ml H-type strong-acid cation exchange resin (Bayel, Lewatit S-1468), thus adsorbing α-methyl-L-serine. After washing by flowing through with water of which volume was the twofold volume of the resin, 352 g of aqueous solution (Content of α-methyl serine: approximately 3.8 %) was obtained by elution using 1 M ammonia water.

The resulting solution was evaporated under reduced pressure to roughly remove ammonia. The pH of the solution at this stage was approximately 8.2. To remove remaining cations therefrom, the solution was flown through a column filled with 20 ml H-type weak-acid cation exchange resin (IONAC, A-365), thus obtaining 204 g outputted solution including washing solution, of which pH was 5.2. Subsequently, the outputted solution was condensed. The condensing process was stopped when crystal began to precipitate out. At this moment, volume weight of the solution was 35.55 g (α-methyl-L-serine: 37.2%). Then, crystallization by adding poor solvent was performed as to the solution by adding 67 g methanol at room temperature. Then, after maturing at 10°C for 1 hour while stirring, crystal was separated and washed with 12 g of 75 % methanol aqueous solution. The obtained wet crystal was dried under reduced pressure at 40°C, resulting in 12.13 g of dried crystal. The content of α-methyl-L-serine therein was 100.7% comparing with a commercially available standard. The resulting crystal contained 0.06 % D-alanine and 0.05 % L-alanine as impurities.

### Example 16: Production of α-ethyl-L-serine with Esherichia coli Expressing 2-methyl serine hydroxymethyl transferase Gene Derived from Bosea sp.

JM109/pUCHHMT as cell-enzyme was suspended in 100 ml solution mixture (150 mM L-2-amino-n-butyric acid, 0.1 mM pyridoxal phosphate, 100 mM phosphoric acid buffer (pH 7.4)). 50.5 ml of 300 mM aqueous formaldehyde was added in the reaction mixture at 30°C over 24 hours while stirring. As formaldehyde, the highest quality of formaldehyde liquid product [code No.: 16223-55] from Nakarai Tesk was used. After the cell separation (8000 g, 10 min), the resulting mixture (50 ml containing 632 mg of 4.75 mmol α-ethyl-serine) was applied into Mega Bond Elut SCX (10G) (Varian, Inc) which had been previously activated with 50 ml methanol and equilibrated with 100 ml H₂O. Then, after washing with 100 ml water, fractions of 2.5 ml were respectively eluted with 0.5 N-HCl. HPLC analysis was performed as to each fraction to confirm abundance of α-ethyl-serine/(α-ethyl serine + L-2-amino-n-butyric acid), thus obtaining the separated fraction of which abundance was more than 99%. Non-separated fractions were evaporated to dryness, and then dissolved into approximately 10 ml water. The pH of the resulting solution was adjusted to around pH 7.0 by NaOH, and again separation process was performed in accordance with the same manner mentioned above. This operation was repeated twice to collect separated fractions, and these separated fractions were evaporated to dryness. The resulting product was dissolved into approximately 100 ml water and anion exchange resin (DEAE-cellulose, whatman) was added therein, and its pH was adjusted around 6.0. After that, the resin was removed by filtration. Acetone was dropped into the resulting filtrate to precipitate crystal. The precipitated crystal was dried, obtaining white crystal (348 mg, 2.6 mmol). The structure of the obtained crystal was determined by NMR spectrum and ESI-MS analysis, and then optical rotation thereof was measured by a optical rotation measuring device (DIP-370) manufactured by Nippon Bunko ([a]_{D}²⁰ =- 3.4 ± 0.4 (c=1, 5N-HCl), [a]_{D}²⁰ = -4.5 ± 0.04 (C=10, 5N-HCl)). It was confirmed that the major product by the enzymatic reaction had (-)-form, i.e., (s)-form in reference to values described in a journal (Journal of Peptide Science, 2001, 7, 619-625; Tetrahedron Letters, 1988, 29, 235-238).

After the cell separation (8000 g, 10 min), 5 mmol NaHCO₃ was added to the resulting mixture (25 ml containing 316 mg of 2.38 mmol α-ethyl-serine), and then pH thereof was adjusted to 9.5 under ice cooling. 2.38 mmol Benzoyl chroride dissolved in 10 ml acetone was dropped therein over 1 hour while keeping pH 9-10. After that, reaction was performed for 3 hours at room temperature, and then HCl was added therein to adjust to pH 2.0. After EtOAc (10 ml x 3) extraction and dehydration with MgSO₄, the reaction product was evaporated to dryness. According to HPLC analysis, it was confirmed that the residue included a large amount of benzoic acid. For that reason, the product was dissolved in methanol and separating was performed by TLC (PLC plate 20 x 20 cm, Silica gel 60F₂₅₄ 2 mm (Merck), developing solvent: EtOAc/AcOH = 20/1). After detection step by UV irradiation, slica gel was collected by scraping, and was subjected to extraction with methanol (100 ml).
The resulting product was evaporated to dryness, thus obtaining white crystal. The white crystal was crystallized again by the following steps: adding a small amount of water thereto and adjusting pH around 8.0 with NaOH to dissolve them; and dropping HCl and adjusting pH around 2.0, to obtain the crystal again. The resulting crystal was separated by filtration. After the filtered crystal was dried, they were dissolved in a small amount of 2-propanol, and then hexane was dropped therein to crystallize again, thus obtaining 110 mg standard product (0.46 mmol). The result of HPLC analysis indicated 99% area. The structure was determined by NMR spectrum and ESI-MS analysis, and then optical rotation was measured ([a]_{D}²⁰ = -11.4 ± 0.2 (C=1, methanol)). It was confirmed that the major product by the enzymatic reaction had (-)-form, i.e., (s)-form in reference to values described in a journal (Journal of Peptide Science, 2001, 7, 619-625).

### Example 17: Production of α-methyl threonine with 2-methyl serine hydroxymethyl transferase Derived from Ralstonia sp. All (AJ110405)

50 µl of purified enzyme solution prepared in accordance with Example 2 was added in a solution composed of 51 mM acetaldehyde, 50 mM L-alanine, 0.1 mM pyridoxal phosphate, and 100 mM phosphoric acid buffer (pH 7.4). The reaction was performed at 30°C for 17.5 hours. After the reaction, a supernatant was prepared by centrifugation (18,000g, 10 min, 4°C). The peak attributed to the molecular ion of α-methyl threonine was detected by ESI-MS analysis as to the obtained supernatant.

### Industrial Applicability

The method of the present invention is useful in the industries involving in amino acid production. It is expected that the present invention would contribute to the production of various types of serine derivatives and optically active amino acid, and specifically, the method may be used in producing, for example, intermediates for drugs and medicals.

### FREE TEXT OF SEQUENCE LISTING

SEQ ID NO: 1: primer
SEQ ID NO: 2: primer
SEQ ID NO: 3: primer
SEQ ID NO: 6: primer
SEQ ID NO: 7: primer
SEQ ID NO: 10: primer
SEQ ID NO: 11: primer
SEQ ID NO: 12: primer
SEQ ID NO: 13: primer
SEQ ID NO: 16: primer
SEQ ID NO: 17: primer
SEQ ID NO: 20: primer
SEQ ID NO: 21: primer
SEQ ID NO: 24: primer
SEQ ID NO: 25: primer
SEQ ID NO: 26: primer
SEQ ID NO: 27: primer
SEQ ID NO: 28: primer
SEQ ID NO: 31: primer
SEQ ID NO: 32: primer
SEQ ID NO: 33: primer
SEQ ID NO: 34: primer

## Claims

1. A method for producing a L-serine derivative of formula (III): 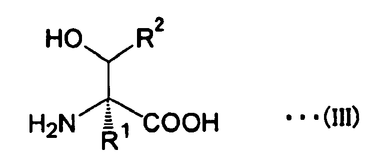 comprising:
reacting L-α-amino acid of formula (I): 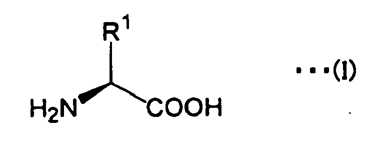 with aldehyde of formula (II): 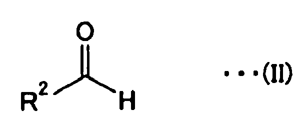 in the presence of an enzyme,
wherein R¹ of formula (I) is selected from a group of an alkyl group with 1 to 6 carbons, an aryl group with 6 to 14 carbons, a cycloalkyl group with 3 to 10 carbons, an aralkyl group with 7 to 19 carbons, an alkoxyalkyl group with 2 to 11 carbons, a group containing a hetero atom in the carbon skeleton thereof, and a group containing a carbon-carbon unsaturated bond in the carbon skeleton thereof, and these groups may be either linear or branched and may have a substituent, and
wherein R² of formula (II) is selected from a group of a hydrogen, an alkyl group with 1 to 6 carbons, an aryl group with 6 to 14 carbons, a cycloalkyl group with 3 to 10 carbons, an aralkyl group with 7 to 19 carbons, an alkoxyalkyl group with 2 to 11 carbons, a group containing a hetero atom in the carbon skeleton thereof, and a group containing a carbon-carbon unsaturated bond in the carbon skeleton thereof, and these groups may be either linear or branched and may have a substituent, and
wherein R¹ of formula (III) is the same as R¹ of formula (I) and R² of formula (III) is the same as R² of formula (II),
wherein said enzyme is one or more protein(s) selected from a group consisting of the following proteins (A) to (L) :
(A) a protein comprising the amino acid sequence of SEQ ID No: 5;
(B) a protein comprising an amino acid sequence which has variation of one or 2 to 50 amino acids in the amino acid sequence of SEQ ID NO: 5, wherein said variation is selected from a group consisting of substitution, deletion, insertion, addition and inversion, and having an activity catalyzing the reaction to form said L-serine derivative of formula (III);
(C) a protein comprising the amino acid sequence of SEQ ID NO: 9;
(D) a protein comprising an amino acid sequence which has variation of one or 2 to 50 amino acids in the amino acid sequence of SEQ ID NO: 9, wherein said variation is selected from a group consisting of substitution, deletion, insertion, addition and inversion, and having an activity catalyzing the reaction to form said L-serine derivative of formula (III);
(E) a protein comprising an amino acid sequence of SEQ ID NO: 15;
(F) a protein comprising an amino acid sequence which has variation of one or 2 to 50 amino acids in the amino acid sequence of SEQ ID NO: 15, wherein said variation is selected from a group consisting of substitution, deletion, insertion, addition and inversion, and having an activity catalyzing the reaction to form said L-serine derivative of formula (III) ;
(G) a protein comprising the amino acid sequence of SEQ ID NO: 19;
(H) a protein comprising an amino acid sequence which has variation of one or 2 to 50 amino acids in the amino acid sequence of SEQ ID NO: 19, wherein said variation is selected from a group consisting of substitution, deletion, insertion, addition and inversion, and having an activity catalyzing the reaction to form said L-serine derivative of formula (III);
(I) a protein comprising the amino acid sequence of SEQ ID NO: 23;
(J) a protein comprising an amino acid sequence which has variation of one or 2 to 50 amino acids in the amino acid sequence of SEQ ID NO: 23, wherein said variation is selected from a group consisting of substitution, deletion, insertion, addition and inversion, and having an activity catalyzing the reaction to form said L-serine derivative of formula (III);
(K) a protein comprising the amino acid sequence of SEQ ID NO: 30; and
(L) a protein comprising an amino acid sequence which has variation of one or 2 to 50 amino acids in the amino acid sequence of SEQ ID NO: 30, wherein said variation is selected from a group consisting of substitution, deletion, insertion, addition and inversion, and having an activity catalyzing the reaction to form said L-serine derivative of formula (III) .

2. The method for producing the L-serine derivative according to claim 1, wherein said amino acid of (I) is L-α-alanine and said L-serine derivative of formula (III) is α-methyl-L-serine.

3. The method for producing the L-serine derivative according to claim 1, wherein said amino acid of (I) is L-2-amino-n-butyric acid and said L-serine derivative of formula (III) is α-ethyl-L-serine.

4. The method for producing the L-serine derivative according to claim 1, wherein said amino acid of (I) is L-α-alanine and said L-serine derivative of formula (III) is α-methyl-L-threonine.

5. A protein having an activity catalyzing a reaction of a L-α-amino acid of formula (I): 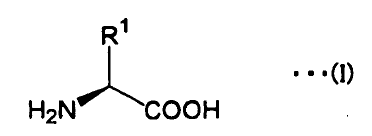 with aldehyde of formula (II) 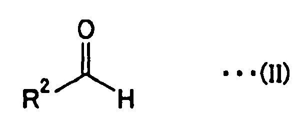 to produce a L-serine derivative of formula (III): 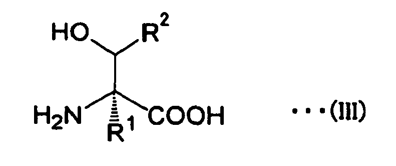
wherein R¹ of formula (I) is selected from a group of an alkyl group with 1 to 6 carbons, an aryl group with 6 to 14 carbons, a cycloalkyl group with 3 to 10 carbons, an aralkyl group with 7 to 19 carbons, an alkoxyalkyl group with 2 to 11 carbons, a group containing a hetero atom in the carbon skeleton thereof, and a group containing a carbon-carbon unsaturated bond in the carbon skeleton thereof, and these groups may be either linear or branched and may have a substituent, and
wherein R² of formula (II) is selected from a group of a hydrogen, an alkyl group with 1 to 6 carbons, an aryl group with 6 to 14 carbons, a cycloalkyl group with 3 to 10 carbons, an aralkyl group with 7 to 19 carbons, an alkoxyalkyl group with 2 to 11 carbons, a group containing a hetero atom in the carbon skeleton thereof, and a group containing a carbon-carbon unsaturated bond in the carbon skeleton thereof, and these groups may be either linear or branched and may have a substituent, and
wherein R¹ of formula (III) is the same as R¹ of formula (I) and R² of formula (III) is the same as R² of formula (II), and
wherein said protein is selected from a group consisting of the following proteins (A) to (L) :
(A) a protein comprising the amino acid sequence of SEQ ID No: 5;
(B) a protein comprising an amino acid sequence which has variation of one or 2 to 50 amino acids in the amino acid sequence of SEQ ID NO: 5, wherein said variation is selected from a group consisting of substitution, deletion, insertion, addition and inversion, and having an activity catalyzing the reaction to form said L-serine derivative of formula (III) ;
(C) a protein comprising the amino acid sequence of SEQ ID NO: 9;
(D) a protein comprising an amino acid sequence which has variation of one or 2 to 50 amino acids in the amino acid sequence of SEQ ID NO: 9, wherein said variation is selected from a group consisting of substitution, deletion, insertion, addition and inversion, and having an activity catalyzing the reaction to form said L-serine derivative of formula (III) ;
(E) a protein comprising an amino acid sequence of SEQ ID NO: 15;
(F) a protein comprising an amino acid sequence which has variation of one or 2 to 50 amino acids in the amino acid sequence of SEQ ID NO: 15, wherein said variation is selected from a group consisting of substitution, deletion, insertion, addition and inversion, and having an activity catalyzing the reaction to form said L-serine derivative of formula (III) ;
(G) a protein comprising the amino acid sequence of SEQ ID NO: 19;
(H) a protein comprising an amino acid sequence which has variation of one or 2 to 50 amino acids in the amino acid sequence of SEQ ID NO: 19, wherein said variation is selected from a group consisting of substitution, deletion, insertion, addition and inversion, and having an activity catalyzing the reaction to form said L-serine derivative of formula (III) ;
(I) a protein comprising the amino acid sequence of SEQ ID NO: 23;
(J) a protein comprising an amino acid sequence which has variation of one or 2 to 50 amino acids in the amino acid sequence of SEQ ID NO: 23, wherein said variation is selected from a group consisting of substitution, deletion, insertion, addition and inversion, and having an activity catalyzing the reaction to form said L-serine derivative of formula (III);
(K) a protein comprising the amino acid sequence of SEQ ID NO: 30; and
(L) a protein comprising an amino acid sequence which has variation of one or 2 to 50 amino acids in the amino acid sequence of SEQ ID NO: 30, wherein said variation is selected from a group consisting of substitution, deletion, insertion, addition and inversion, and having an activity catalyzing the reaction to form said L-serine derivative of formula (III).

6. A polynucleotide encoding a protein of claim 5.

7. A polynucleotide selected from a group consisting of the following (a) to (1) :
(a) a nucleotide comprising the nucleotide sequence of SEQ ID NO: 4;
(b) a polynucleotide which hybridizes with a polynucleotide having a nucleotide sequence complementary to that of SEQ ID NO: 4 under stringent conditions, and which encodes a protein having an activity catalyzing a reaction of an L-α-amino acid formula (I) with an aldehyde of formula (II) to produce an L-serine derivative in the formula (III);
(c) a nucleotide comprising the nucleotide sequence of SEQ ID NO: 8;
(d) a polynucleotide which hybridizes with a polynucleotide having a nucleotide sequence complementary to that of SEQ ID NO: 8 under stringent conditions, and which encodes a protein having an activity catalyzing a reaction of an L-α-amino acid formula (I) with an aldehyde of formula (II) to produce an L-serine derivative in the formula (III);
(e) a nucleotide comprising the nucleotide sequence of SEQ ID NO: 14;
(f) a polynucleotide which hybridizes with a polynucleotide having a nucleotide sequence complementary to that of SEQ ID NO: 14 under stringent conditions, and which encodes a protein having an activity catalyzing a reaction of an L-α-amino acid formula (I) with an aldehyde of formula (II) to produce an L-serine derivative in the formula (III);
(g) a nucleotide comprising the nucleotide sequence of SEQ ID NO: 18;
(h) a polynucleotide which hybridizes with a polynucleotide having a nucleotide sequence complementary to that of SEQ ID NO: 18 under stringent conditions, and which encodes a protein having an activity catalyzing a reaction of an L-α-amino acid formula (I) with an aldehyde of formula (II) to produce an L-serine derivative in the formula (III);
(i) a nucleotide comprising the nucleotide sequence of SEQ ID NO: 22;
(j) a polynucleotide which hybridizes with a polynucleotide having a nucleotide sequence complementary to that of SEQ ID NO: 22 under stringent conditions, and which encodes a protein having an activity catalyzing a reaction of an L-α-amino acid formula (I) with an aldehyde of formula (II) to produce an L-serine derivative in the formula (III);
(k) a nucleotide comprising the nucleotide sequence of SEQ ID NO: 29; and
(l) a polynucleotide which hybridizes with a polynucleotide having a nucleotide sequence complementary to that of SEQ ID NO: 29 under stringent conditions, and which encodes a protein having an activity catalyzing a reaction of an L-α-amino acid formula (I) with an aldehyde of formula (II) to produce an L-serine derivative in the formula (III);
wherein stringent conditions mean conditions under which polynucleotides having 90% or more homology to the respective sequence hybridize, while polynucleotides having lower than 90% homology do not hybridize; and
wherein formula (I) is: 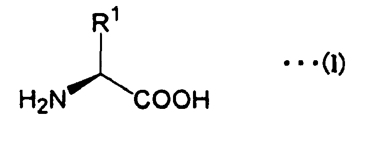
(R¹ of formula (I) is selected from a group of an alkyl group with 1 to 6 carbons, an aryl group with 6 to 14 carbons, a cycloalkyl group with 3 to 10 carbons, an aralkyl group with 7 to 19 carbons, an alkoxyalkyl group with 2 to 11 carbons, a group containing a hetero atom in the carbon skeleton thereof, and a group containing a carbon-carbon unsaturated bond in the carbon skeleton thereof, and these groups may be either linear or branched and may have a substituent);
wherein said formula (II) is: 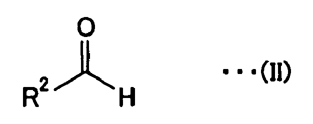
(R² of formula (II) is selected from a group of a hydrogen, an alkyl group with 1 to 6 carbons, an aryl group with 6 to 14 carbons, a cycloalkyl group with 3 to 10 carbons, an aralkyl group with 7 to 19 carbons, an alkoxyalkyl group with 2 to 11 carbons, a group containing a hetero atom in the carbon skeleton thereof, and a group containing a carbon-carbon unsaturated bond in the carbon skeleton thereof, and these groups may be either linear or branched and may have a substituent);
wherein said formula (III) is: 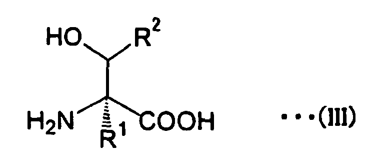
(wherein R¹ of formula (III) is the same as R¹ of formula (I) and R² of formula (III) is the same as R² of formula (II)).

8. A recombinant polynucleotide having said polynucleotide according to claim 6 or 7 incorporated therein.

9. A transformant having said polynucleotide according to claim 8 incorporated therein.

## Patentansprüche

1. Verfahren zum Herstellen eines L-Serinderivats der Formel (III): 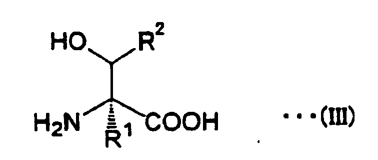 umfassend:
das Umsetzen einer L-α-Aminosäure der Formel (I): 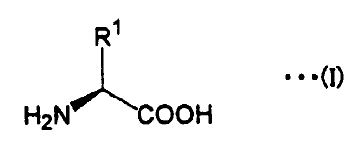
mit einem Aldehyd der Formel (II): 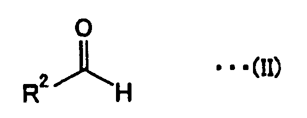 in Gegenwart eines Enzyms,
wobei R¹ der Formel (I) ausgewählt wird aus einer Gruppe bestehend aus einer Alkylgruppe mit 1 bis 6 Kohlenstoffatomen, einer Arylgruppe mit 6 bis 14 Kohlenstoffatomen, einer Cycloalkylgruppe mit 3 bis 10 Kohlenstoffatomen, einer Arylalkylgruppe mit 7 bis 19 Kohlenstoffatomen, einer Alkoxyalkylgruppe mit 2 bis 11 Kohlenstoffatomen, einer Gruppe, die ein Heteroatom an ihrem Kohlenstoffrückgrat enthält und einer Gruppe, die eine ungesättigte Kohlenstoff-Kohlenstoff-Bindung in ihrem Rückgrat enthält, wobei diese Gruppen entweder geradkettig oder verzweigt sein können und einen Substituenten tragen können, und
wobei R² der Formel (II) ausgewählt wird aus einer Gruppe bestehend aus einem Wasserstoffatom, einer Alkylgruppe mit 1 bis 6 Kohlenstoffatomen, einer Arylgruppe mit 6 bis 14 Kohlenstoffatomen, einer Cycloalkylgruppe mit 3 bis 10 Kohlenstoffatomen, einer Arylalkylgruppe mit 7 bis 19 Kohlenstoffatomen, einer Alkoxyalkylgruppe mit 2 bis 11 Kohlenstoffatomen, einer Gruppe, die ein Heteroatom in ihrem Kohlenstoffrückgrat enthält, und einer Gruppe, die eine ungesättigte Kohlenstoff-Kohlenstoff-Bindung in ihrem Kohlenstoffrückgrat enthält, wobei diese Gruppen entweder geradkettig oder verzweigt sein können und einen Substituenten tragen können, und
wobei R¹ der Formel (III) der gleiche ist, wie R¹ der Formel (I) und R² der Formel (III) der gleiche ist wie R² der Formel (II),
wobei das Enzym ein oder mehr Proteine, ausgewählt aus der Gruppe bestehend aus den folgenden Proteinen (A) bis (L), ist (sind):
(A) ein Protein, das die Aminosäuresequenz der SEQ ID Nr.: 5 umfasst;
(B) ein Protein, das eine Aminosäuresequenz umfasst, die eine Abweichung von einer oder 2 bis 50 Aminosäuren gegenüber der Aminosäuresequenz von SEQ ID Nr.: 5 hat, wobei die Abweichung ausgewählt wird aus einer Gruppe bestehend aus Substitution, Deletion, Insertion, Addition und Inversion, und das eine Aktivität hat, die Reaktion zu katalysieren, um das L-Serinderivat der Formel (III) zu bilden;
(C) ein Protein, das die Aminosäuresequenz der SEQ ID Nr.: 9 umfasst;
(D) ein Protein, das eine Aminosäuresequenz umfasst, die eine Abweichung von einer oder 2 bis 50 Aminosäuren gegenüber der Aminosäuresequenz von SEQ ID Nr.: 9 hat, wobei die Abweichung ausgewählt wird aus einer Gruppe bestehend aus Substitution, Deletion, Insertion, Addition und Inversion, und das eine Aktivität hat, die Reaktion zu katalysieren, um das L-Serinderivat der Formel (III) zu bilden;
(E) ein Protein, das eine Aminosäuresequenz der SEQ ID Nr.: 15 umfasst;
(F) ein Protein, das eine Aminosäuresequenz umfasst, die eine Abweichung von einer oder 2 bis 50 Aminosäuren gegenüber der Aminosäuresequenz von SEQ ID Nr.: 15 hat, wobei die Abweichung ausgewählt wird aus einer Gruppe bestehend aus Substitution, Deletion, Insertion, Addition und Inversion, und das eine Aktivität hat, die Reaktion zu katalysieren, um das L-Serinderivat der Formel (III) zu bilden;
(G) ein Protein, das die Aminosäuresequenz der SEQ ID Nr.: 19 umfasst;
(H) ein Protein, das eine Aminosäuresequenz umfasst, die eine Abweichung von einer oder 2 bis 50 Aminosäuren gegenüber der Aminosäuresequenz von SEQ ID Nr.: 19 hat, wobei die Abweichung ausgewählt wird aus einer Gruppe bestehend aus Substitution, Deletion, Insertion, Addition und Inversion, und das eine Aktivität hat, die Reaktion zu katalysieren, um das L-Serinderivat der Formel (III) zu bilden;
(I) ein Protein, das die Aminosäuresequenz der SEQ ID Nr.: 23 umfasst;
(J) ein Protein, das eine Aminosäuresequenz umfasst, die eine Abweichung von einer oder 2 bis 50 Aminosäuren gegenüber der Aminosäuresequenz von SEQ ID Nr.: 23 hat, wobei die Abweichung ausgewählt wird aus einer Gruppe bestehend aus Substitution, Deletion, Insertion, Addition und Inversion, und das eine Aktivität hat, die Reaktion zu katalysieren, um das L-Serinderivat der Formel (III) zu bilden;
(K) ein Protein, das die Aminosäuresequenz der SEQ ID Nr.: 30 umfasst; und
(L) ein Protein, das eine Aminosäuresequenz umfasst, die eine Abweichung von einer oder 2 bis 50 Aminosäuren gegenüber der Aminosäuresequenz von SEQ ID Nr.: 30 hat, wobei die Abweichung ausgewählt wird aus einer Gruppe bestehend aus Substitution, Deletion, Insertion, Addition und Inversion, und das eine Aktivität hat, die Reaktion zu katalysieren, um das L-Serinderivat der Formel (III) zu bilden;

2. Verfahren zum Herstellen des L-Serinderivats gemäß Anspruch 1, wobei die Aminosäure von (I) L-α-Alanin und das L-Serinderivat der Formel (III) α-Methyl-L-serin ist.

3. Verfahren zum Herstellen des L-Serinderivats gemäß Anspruch 1, wobei die Aminosäure von (I) L-2-Amino-n-buttersäure und das L-Serinderivat von Formel (III) α-Ethyl-L-serin ist.

4. Verfahren zum Herstellen des L-Serinderivats gemäß Anspruch 1, wobei die Aminosäure von (I) L-α-Alanin und das L-Serinderivat von Formel (III) α-Methyl-L-threonin ist.

5. Protein mit einer Aktivität zur Katalyse der Reaktion einer L-α-Aminosäure der Formel (I): 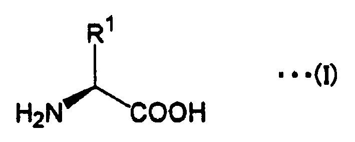 mit einem Aldehyd der Formel (II): 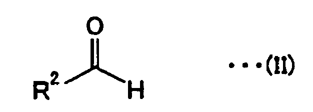 ein L-Serinderivat der Formel (III): 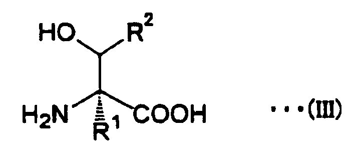 herzustellen,
wobei R¹ der Formel (I) ausgewählt wird aus einer Gruppe bestehend aus einer Alkylgruppe mit 1 bis 6 Kohlenstoffatomen, einer Arylgruppe mit 6 bis 14 Kohlenstoffatomen, einer Cycloalkylgruppe mit 3 bis 10 Kohlenstoffatomen, einer Arylalkylgruppe mit 7 bis 19 Kohlenstoffatomen, einer Alkoxyalkylgruppe mit 2 bis 11 Kohlenstoffatomen, einer Gruppe, die ein Heteroatom an ihrem Kohlenstoffrückgrat enthält, und einer Gruppe, die eine ungesättigte Kohlenstoff-Kohlenstoff-Bindung in ihrem Rückgrat enthält, wobei diese Gruppen entweder geradkettig oder verzweigt sein können und einen Substituenten tragen können, und
wobei R² der Formel (II) ausgewählt wird aus einer Gruppe bestehend aus einem Wasserstoffatom, einer Alkylgruppe mit 1 bis 6 Kohlenstoffatomen, einer Arylgruppe mit 6 bis 14 Kohlenstoffatomen, einer Cycloalkylgruppe mit 3 bis 10 Kohlenstoffatomen, einer Arylalkylgruppe mit 7 bis 19 Kohlenstoffatomen, einer Alkoxyalkylgruppe mit 2 bis 11 Kohlenstoffatomen, einer Gruppe, die ein Heteroatom in ihrem Kohlenstoffrückgrat enthält, und einer Gruppe, die eine ungesättigte Kohlenstoff-Kohlenstoff-Bindung in ihrem Kohlenstoffrückgrat enthält, wobei diese Gruppen entweder geradkettig oder verzweigt sein können und einen Substituenten tragen können, und
wobei R¹ der Formel (III) der gleiche ist, wie R¹ der Formel (I) und R² der Formel (III) der gleiche ist wie R² der Formel (II), und
wobei das Protein ausgewählt wird aus der Gruppe bestehend aus den folgenden Proteinen (A) bis (L):
(A) ein Protein, das die Aminosäuresequenz der SEQ ID Nr.: 5 umfasst;
(B) ein Protein, das eine Aminosäuresequenz umfaßt, die eine Abweichung von einer oder 2 bis 50 Aminosäuren gegenüber der Aminosäuresequenz von SEQ ID Nr.: 5 hat, wobei die Abweichung ausgewählt wird aus einer Gruppe bestehend aus Substitution, Deletion, Insertion, Addition und Inversion, und das eine Aktivität hat, die Reaktion zu katalysieren, um das L-Serinderivat der Formel (III) zu bilden;
(C) ein Protein, das die Aminosäuresequenz der SEQ ID Nr.: 9 umfasst;
(D) ein Protein, das eine Aminosäuresequenz umfasst, die eine Abweichung von einer oder 2 bis 50 Aminosäuren gegenüber der Aminosäuresequenz von SEQ ID Nr.: 9 hat, wobei die Abweichung ausgewählt wird aus einer Gruppe bestehend aus Substitution, Deletion, Insertion, Addition und Inversion, und das eine Aktivität hat, die Reaktion zu katalysieren, um das L-Serinderivat der Formel (III) zu bilden;
(E) ein Protein, das eine Aminosäuresequenz der SEQ ID Nr.: 15 umfasst;
(F) ein Protein, das eine Aminosäuresequenz umfasst, die eine Abweichung von einer oder 2 bis 50 Aminosäuren gegenüber der Aminosäuresequenz von SEQ ID Nr.: 15 hat, wobei die Abweichung ausgewählt wird aus einer Gruppe bestehend aus Substitution, Deletion, Insertion, Addition und Inversion, und das eine Aktivität hat, die Reaktion zu katalysieren, um das L-Serinderivat der Formel (III) zu bilden;
(G) ein Protein, das die Aminosäuresequenz der SEQ ID Nr. 19 umfasst;
(H) ein Protein, das eine Aminosäuresequenz umfasst, die eine Abweichung von einer oder 2 bis 50 Aminosäuren gegenüber der Aminosäuresequenz von SEQ ID Nr.: 19 hat, wobei die Abweichung ausgewählt wird aus einer Gruppe bestehend aus Substitution, Deletion, Insertion, Addition und Inversion, und das eine Aktivität hat, die Reaktion zu katalysieren, um das L-Serinderivat der Formel (III) zu bilden;
(I) ein Protein, das die Aminosäuresequenz der SEQ ID Nr.: 23 umfasst;
(J) ein Protein, das eine Aminosäuresequenz umfasst, die eine Abweichung von einer oder 2 bis 50 Aminosäuren gegenüber der Aminosäuresequenz von SEQ ID Nr.: 23 hat, wobei die Abweichung ausgewählt wird aus einer Gruppe bestehend aus Substitution, Deletion, Insertion, Addition und Inversion, und das eine Aktivität hat, die Reaktion zu katalysieren, um das L-Serinderivat der Formel (III) zu bilden;
(K) ein Protein, das die Aminosäuresequenz der SEQ ID Nr.: 30 umfasst; und
(L) ein Protein, das eine Aminosäuresequenz umfasst, die eine Abweichung von einer oder 2 bis 50 Aminosäuren gegenüber der Aminosäuresequenz von SEQ ID Nr.: 30 hat, wobei die Abweichung ausgewählt wird aus einer Gruppe bestehend aus Substitution, Deletion, Insertion, Addition und Inversion, und das eine Aktivität hat, die Reaktion zu katalysieren, um das L-Serinderivat der Formel (III) zu bilden;

6. Polynukleotid, das für ein Protein gemäß Anspruch 5 kodiert.

7. Polynukleotid ausgewählt aus der Gruppe bestehend aus den folgenden (a) bis (1):
(a) ein Nukleotid, das die Nukleotidsequenz der SEQ ID Nr.: 4 umfasst;
(b) ein Polynukleotid, das mit einem Polynukleotid mit einer Nukleotidsequenz komplementär zu derjenigen von SEQ ID Nr.: 4 unter stringenten Bedingungen hybridisiert, und das für ein Protein kodiert, das eine Aktivität zur Katalyse einer Reaktion einer L-α-Aminosäure der Formel (I) mit einem Aldehyd der Formel (II) hat, um ein L-Serinderivat in der Formel (III) herzustellen;
(c) ein Nukleotid, das die Nukleotidsequenz der SEQ ID Nr.: 8 umfasst;
(d) ein Polynukleotid, das mit einem Polynukleotid mit einer Nukleotidsequenz komplementär zu derjenigen von SEQ ID Nr.: 8 unter stringenten Bedingungen hybridisiert, und das für ein Protein kodiert, das eine Aktivität zur Katalyse einer Reaktion einer L-α-Aminosäure der Formel (I) mit einem Aldehyd der Formel (II) hat, um ein L-Serinderivat in der Formel (III) herzustellen;
(e) ein Nukleotid, das die Nukleotidsequenz der SEQ ID Nr.: 14 umfasst;
(f) ein Polynukleotid, das mit einem Polynukleotid mit einer Nukleotidsequenz komplementär zu derjenigen von SEQ ID Nr.: 14 unter stringenten Bedingungen hybridisiert, und das für ein Protein kodiert, das eine Aktivität zur Katalyse einer Reaktion einer L-α-Aminosäure der Formel (I) mit einem Aldehyd der Formel (II) hat, um ein L-Serinderivat in der Formel (III) herzustellen;
(g) ein Nukleotid, das die Nukleotidsequenz der SEQ ID Nr.: 18 umfasst;
(h) ein Polynukleotid, das mit einem Polynukleotid mit einer Nukleotidsequenz komplementär zu derjenigen von SEQ ID Nr.: 18 unter stringenten Bedingungen hybridisiert, und das für ein Protein kodiert, das eine Aktivität zur Katalyse einer Reaktion einer L-α-Aminosäure der Formel (I) mit einem Aldehyd der Formel (II) hat, um ein L-Serinderivat in der Formel (III) herzustellen;
(i) ein Nukleotid, das die Nukleotidsequenz der SEQ ID Nr.: 22 umfasst;
(j) ein Polynukleotid, das mit einem Polynukleotid mit einer Nukleotidsequenz komplementär zu derjenigen von SEQ ID Nr.: 22 unter stringenten Bedingungen hybridisiert, und das für ein Protein kodiert, das eine Aktivität zur Katalyse einer Reaktion einer L-α-Aminosäure der Formel (I) mit einem Aldehyd der Formel (II) hat, um ein L-Serinderivat in der Formel (III) herzustellen;
(k) ein Nukleotid, das die Nukleotidsequenz der SEQ ID Nr.: 29 umfasst; und
(l) ein Polynukleotid, das mit einem Polynukleotid mit einer Nukleotidsequenz komplementär zu derjenigen von SEQ ID Nr.: 29 unter stringenten Bedingungen hybridisiert, und das für ein Protein kodiert, das eine Aktivität zur Katalyse einer Reaktion einer L-α-Aminosäure der Formel (I) mit einem Aldehyd der Formel (II) hat, um ein L-Serinderivat in der Formel (III) herzustellen;
wobei stringente Bedingungen bedeuten, bei denen Polynukleotide mit 90 % oder mehr Homologie zu der entsprechenden Sequenz hybridisieren, während Polynukleotide mit einer niedrigeren Homologie als 90 % nicht hybridisieren; und
wobei die Formel (I) ist: 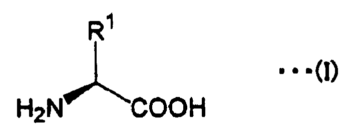 (R¹ von Formel (I) wird ausgewählt aus einer Gruppe bestehend aus einer Alkylgruppe mit 1 bis 6 Kohlenstoffatomen, einer Arylgruppe mit 6 bis 14 Kohlenstoffatomen, einer Cycloalkylgruppe mit 3 bis 10 Kohlenstoffatomen, einer Arylalkylgruppe mit 7 bis 19 Kohlenstoffatomen, einer Alkoxyalkylgruppe mit 2 bis 11 Kohlenstoffatomen, einer Gruppe, die ein Heteroatom an ihrem Kohlenstoffrückgrat enthält, und einer Gruppe, die eine ungesättigte Kohlenstoff-Kohlenstoff-Bindung in ihrem Rückgrat enthält, wobei diese Gruppen entweder geradkettig oder verzweigt sein können und einen Substituenten tragen können);
wobei die Formel (II) ist: 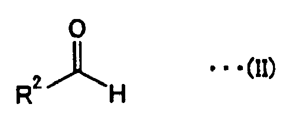 (R² von Formel (II) wird ausgewählt aus einer Gruppe bestehend aus einem Wasserstoffatom, einer Alkylgruppe mit 1 bis 6 Kohlenstoffatomen, einer Arylgruppe mit 6 bis 14 Kohlenstoffatomen, einer Cycloalkylgruppe mit 3 bis 10 Kohlenstoffatomen, einer Arylalkylgruppe mit 7 bis 19 Kohlenstoffatomen, einer Alkoxyalkylgruppe mit 2 bis 11 Kohlenstoffatomen, einer Gruppe, die ein Heteroatom in ihrem Kohlenstoffrückgrat enthält, und einer Gruppe, die eine ungesättigte Kohlenstoff-Kohlenstoff-Bindung in ihrem Kohlenstoffrückgrat enthält, wobei diese Gruppen entweder geradkettig oder verzweigt sein können und einen Substituenten tragen können);
wobei die Formel (III) ist: 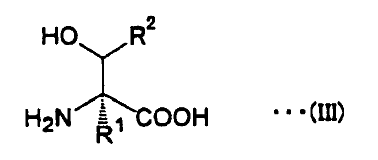 (wobei R¹ der Formel (III) derselbe ist wie R¹ der Formel (I) und R² der Formel (III) derselbe ist wie R² der Formel (II)).

8. Rekombinantes Polynukleotid, in das das Polynukleotid gemäß Anspruch 6 oder 7 eingebaut ist.

9. Transformant, in den das Polynukleotid gemäß Anspruch 8 eingebaut ist.

## Revendications

1. Procédé de production d'un dérivé L-sérine de formule (III) 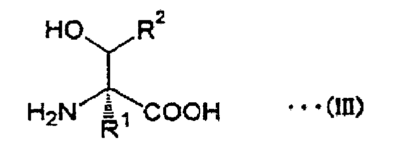 comprenant :
la réaction d'un acide L-α-aminé de formule (I) : 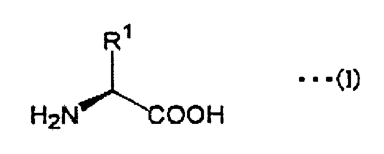 avec un aldéhyde de formule (II) : 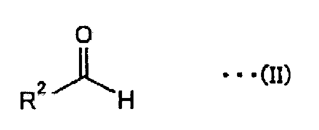 en présence d'une enzyme,
dans lequel R¹ de la formule (I) est choisi dans un groupe constitué par un groupe alkyle avec 1 à 6 carbones, un groupe aryle avec 6 à 14 carbones, un groupe cycloalkyle avec 3 à 10 carbones, un groupe aralkyle avec 7 à 19 carbones, un groupe alcoxyalkyle avec 2 à 11 carbones, un groupe contenant un hétéroatome dans le squelette carboné de celui-ci, et un groupe contenant une liaison insaturée carbone-carbone dans le squelette carboné de celui-ci, et ces groupes peuvent être soit linéaires, soit ramifiés et peuvent avoir un substituant, et
dans lequel R² de la formule (II) est choisi dans un groupe constitué par un hydrogène, un groupe alkyle avec 1 à 6 carbones, un groupe aryle avec 6 à 14 carbones, un groupe cycloalkyle avec 3 à 10 carbones, un groupe aralkyle avec 7 à 19 carbones, un groupe alcoxyalkyle avec 2 à 11 carbones, un groupe contenant un hétéroatome dans le squelette carboné de celui-ci, et un groupe contenant une liaison insaturée carbone-carbone dans le squelette carboné de celui-ci, et ces groupes peuvent être soit linéaires, soit ramifiés et peuvent avoir un substituant, et
dans lequel R¹ de la formule (III) est identique à R¹ de la formule (I) et R² de la formule (III) est identique à R² de la formule (II),
dans lequel ladite enzyme est une ou plusieurs protéine(s) choisie(s) dans un groupe constitué par les protéines (A) à (L) suivantes :
(A) une protéine comprenant la séquence d'acides aminés de SEQ ID No: 5 ;
(B) une protéine comprenant une séquence d'acides aminés qui a une variation de un ou de 2 à 50 acides aminés dans la séquence d'acides aminés de SEQ ID No: 5, dans laquelle ladite variation est choisie dans un groupe constitué par une substitution, une délétion, une insertion, une addition et une inversion, et ayant une activité catalysant la réaction pour former ledit dérivé L-sérine de formule (III) ;
(C) une protéine comprenant la séquence d'acides aminés de SEQ ID No: 9;
(D) une protéine comprenant une séquence d'acides aminés qui a une variation de un ou de 2 à 50 acides aminés dans la séquence d'acides aminés de SEQ ID No: 9, dans laquelle ladite variation est choisie dans un groupe constitué par une substitution, une délétion, une insertion, une addition et une inversion, et ayant une activité catalysant la réaction pour former ledit dérivé L-sérine de formule (III) ;
(E) une protéine comprenant une séquence d'acides aminés de SEQ ID No: 15 ;
(F) une protéine comprenant une séquence d'acides aminés qui a une variation de un ou de 2 à 50 acides aminés dans la séquence d'acides aminés de SEQ ID No: 15, dans laquelle ladite variation est choisie dans un groupe constitué par une substitution, une délétion, une insertion, une addition et une inversion, et ayant une activité catalysant la réaction pour former ledit dérivé L-sérine de formule (III) ;
(G) une protéine comprenant la séquence d'acides aminés de SEQ ID No: 19 ;
(H) une protéine comprenant une séquence d'acides aminés qui a une variation de un ou de 2 à 50 acides aminés dans la séquence d'acides aminés de SEQ ID No: 19, dans laquelle ladite variation est choisie dans un groupe constitué par une substitution, une délétion, une insertion, une addition et une inversion, et ayant une activité catalysant la réaction pour former ledit dérivé L-sérine de formule (III) ;
(I) une protéine comprenant la séquence d'acides aminés de SEQ ID No: 23 ;
(J) une protéine comprenant une séquence d'acides aminés qui a une variation de un ou de 2 à 50 acides aminés dans la séquence d'acides aminés de SEQ ID No: 23, dans laquelle ladite variation est choisie dans un groupe constitué par une substitution, une délétion, une insertion, une addition et une inversion, et ayant une activité catalysant la réaction pour former ledit dérivé L-sérine de formule (III) ;
(K) une protéine comprenant la séquence d'acides aminés de SEQ ID No: 30 ; et
(L) une protéine comprenant une séquence d'acides aminés qui a une variation de un ou de 2 à 50 acides aminés dans la séquence d'acides aminés de SEQ ID No: 30, dans laquelle ladite variation est choisie dans un groupe constitué par une substitution, une délétion, une insertion, une addition et une inversion, et ayant une activité catalysant la réaction pour former ledit dérivé L-sérine de formule (III).

2. Procédé de production du dérivé L-sérine selon la revendication 1, dans lequel ledit acide aminé de (I) est la L-α-alanine et ledit dérivé L-sérine de formule (III) est l'α-méthyl-L-sérine.

3. Procédé de production du dérivé L-sérine selon la revendication 1, dans lequel ledit acide aminé de (I) est l'acide L-2-amino-n-butyrique et ledit dérivé L-sérine de formule (III) est l'α-éthyl-L-sérine.

4. Procédé de production du dérivé L-sérine selon la revendication 1, dans lequel ledit acide aminé de (I) est la L-α-alanine et ledit dérivé L-sérine de formule (III) est l'α-méthyle-L-thréonine.

5. Protéine ayant une activité catalysant une réaction d'un acide L-α-aminé de formule (I) : 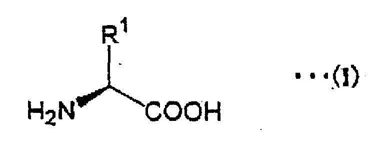 avec une aldéhyde de formule (II) 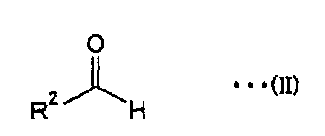 pour produire un dérivé L-sérine de formule (III) : 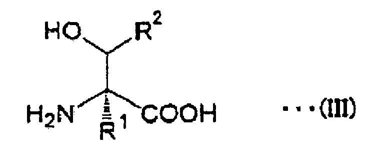 dans laquelle R¹ de la formule (I) est choisi dans un groupe constitué par un groupe alkyle avec 1 à 6 carbones, un groupe aryle avec 6 à 14 carbones, un groupe cycloalkyle avec 3 à 10 carbones, un groupe aralkyle avec 7 à 19 carbones, un groupe alcoxyalkyle avec 2 à 11 carbones, un groupe contenant un hétéroatome dans le squelette carboné de celui-ci, et un groupe contenant une liaison insaturée carbone-carbone dans le squelette carboné de celui-ci, et ces groupes peuvent être soit linéaires, soit ramifiés et peuvent avoir un substituant, et
dans laquelle R² de la formule (II) est choisi dans un groupe constitué par un hydrogène, un groupe alkyle avec 1 à 6 carbones, un groupe aryle avec 6 à 14 carbones, un groupe cycloalkyle avec 3 à 10 carbones, un groupe aralkyle avec 7 à 19 carbones, un groupe alcoxyalkyle avec 2 à 11 carbones, un groupe contenant un hétéroatome dans le squelette carboné de celui-ci, et un groupe contenant une liaison insaturée carbone-carbone dans le squelette carboné de celui-ci, et ces groupes peuvent être soit linéaires, soit ramifiés et peuvent avoir un substituant, et
dans laquelle R¹ de la formule (III) est identique à R¹ de la formule (I) et R² de la formule (III) est identique à R² de la formule (II), et
dans laquelle ladite protéine est choisie dans un groupe constitué par les protéines (A) à (L) suivantes :
(A) une protéine comprenant la séquence d'acides aminés de SEQ ID No: 5 ;
(B) une protéine comprenant une séquence d'acides aminés qui a une variation de un ou de 2 à 50 acides aminés dans la séquence d'acides aminés de SEQ ID No: 5, dans laquelle ladite variation est choisie dans un groupe constitué par une substitution, une délétion, une insertion, une addition et une inversion, et ayant une activité catalysant la réaction pour former ledit dérivé L-sérine de formule (III) ;
(C) une protéine comprenant la séquence d'acides aminés de SEQ ID No: 9 ;
(D) une protéine comprenant une séquence d'acides aminés qui a une variation de un ou de 2 à 50 acides aminés dans la séquence d'acides aminés de SEQ ID No: 9, dans laquelle ladite variation est choisie dans un groupe constitué par une substitution, une délétion, une insertion, une addition et une inversion, et ayant une activité catalysant la réaction pour former ledit dérivé L-sérine de formule (III) ;
(E) une protéine comprenant une séquence d'acides aminés de SEQ ID No: 15 ;
(F) une protéine comprenant une séquence d'acides aminés qui a une variation de un ou de 2 à 50 acides aminés dans la séquence d'acides aminés de SEQ ID No: 15, dans laquelle ladite variation est choisie dans un groupe constitué par une substitution, une délétion, une insertion, une addition et une inversion, et ayant une activité catalysant la réaction pour former ledit dérivé L-sérine de formule (III) ;
(G) une protéine comprenant la séquence d'acides aminés de SEQ ID No: 19 ;
(H) une protéine comprenant une séquence d'acides aminés qui a une variation de un ou de 2 à 50 acides aminés dans la séquence d'acides aminés de SEQ ID No: 19, dans laquelle ladite variation est choisie dans un groupe constitué par une substitution, une délétion, une insertion, une addition et une inversion, et ayant une activité catalysant la réaction pour former ledit dérivé L-sérine de formule (III) ;
(I) une protéine comprenant la séquence d'acides aminés de SEQ ID No: 23 ;
(J) une protéine comprenant une séquence d'acides aminés qui a une variation de un ou de 2 à 50 acides aminés dans la séquence d'acides aminés de SEQ ID No: 23, dans laquelle ladite variation est choisie dans un groupe constitué par une substitution, une délétion, une insertion, une addition et une inversion, et ayant une activité catalysant la réaction pour former ledit dérivé L-sérine de formule (III) ;
(K) une protéine comprenant la séquence d'acides aminés de SEQ ID No: 30 ; et
(L) une protéine comprenant une séquence d'acides aminés qui a une variation de un ou de 2 à 50 acides aminés dans la séquence d'acides aminés de SEQ ID No: 30, dans laquelle ladite variation est choisie dans un groupe constitué par une substitution, une délétion, une insertion, une addition et une inversion, et ayant une activité catalysant la réaction pour former ledit dérivé L-sérine de formule (III).

6. Polynucléotide codant pour une protéine selon la revendication 5.

7. Polynucléotide choisi dans un groupe constitué par (a) à (1) suivantes :
(a) un nucléotide comprenant la séquence nucléotidique de SEQ ID No: 4 ;
(b) un polynucléotide qui s'hybride avec un polynucléotide ayant une séquence nucléotidique complémentaire de celle de SEQ ID No: 4 dans des conditions stringentes, et qui code pour une protéine ayant une activité catalysant une réaction d'un acide L-α-aminé de formule (I) avec une aldéhyde de formule (II) pour produire un dérivé L-sérine de formule (III) ;
(c) un nucléotide comprenant la séquence nucléotidique de SEQ ID No: 8 ;
(d) un polynucléotide qui s'hybride avec un polynucléotide ayant une séquence nucléotidique complémentaire de celle de SEQ ID No: 8 dans des conditions stringentes, et qui code pour une protéine ayant une activité catalysant une réaction d'un acide L-α-aminé de formule (I) avec un aldéhyde de formule (II) pour produire un dérivé L-sérine de formule (III) ;
(e) un nucléotide comprenant la séquence nucléotidique de SEQ ID No: 14 ;
(f) un polynucléotide qui s'hybride avec un polynucléotide ayant une séquence nucléotidique complémentaire de celle de SEQ ID No: 14 dans des conditions stringentes, et qui code pour une protéine ayant une activité catalysant une réaction d'un acide L-α-aminé de formule (I) avec un aldéhyde de formule (II) pour produire un dérivé L-sérine de formule (III) ;
(g) un nucléotide comprenant la séquence nucléotidique de SEQ ID No: 18 ;
(h) un polynucléotide qui s'hybride avec un polynucléotide ayant une séquence nucléotidique complémentaire de celle de SEQ ID No: 18 dans des conditions stringentes, et qui code pour une protéine ayant une activité catalysant une réaction d'un acide L-α-aminé de formule (I) avec un aldéhyde de formule (II) pour produire un dérivé L-sérine de formule (III) ;
(i) un nucléotide comprenant la séquence nucléotidique de SEQ ID No: 22 ;
(j) un polynucléotide qui s'hybride avec un polynucléotide ayant une séquence nucléotidique complémentaire de celle de SEQ ID No: 22 dans des conditions stringentes, et qui code pour une protéine ayant une activité catalysant une réaction d'un acide L-α-aminé de formule (I) avec un aldéhyde de formule (II) pour produire un dérivé L-sérine de formule (III) ;
(k) un nucléotide comprenant la séquence nucléotidique de SEQ ID No: 29 ; et
(l) un polynucléotide qui s'hybride avec un polynucléotide ayant une séquence nucléotidique complémentaire de celle de SEQ ID No: 29 dans des conditions stringentes, et qui code pour une protéine ayant une activité catalysant une réaction d'un acide L-α-aminé de formule (I) avec un aldéhyde de formule (II) pour produire un dérivé L-sérine de formule (III) ;
dans lequel des conditions stringentes signifient des conditions dans lesquelles les polynucléotides ayant 90 % d'homologie ou plus avec la séquence respective s'hybrident, tandis que les polynucléotides ayant une homologie inférieure à 90 % ne s'hybrident pas ; et
dans lequel la formule (I) est : 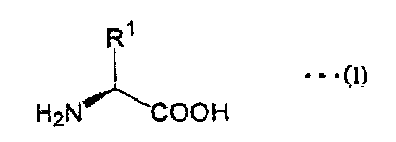 (R¹ de la formule (I) est choisi dans un groupe constitué par un groupe alkyle avec 1 à 6 carbones, un groupe aryle avec 6 à 14 carbones, un groupe cycloalkyle avec 3 à 10 carbones, un groupe aralkyle avec 7 à 19 carbones, un groupe alcoxyalkyle avec 2 à 11 carbones, un groupe contenant un hétéroatome dans le squelette carboné de celui-ci, et un groupe contenant une liaison insaturée carbone-carbone dans le squelette carboné de celui-ci, et ces groupes peuvent être soit linéaires, soit ramifiés et peuvent avoir un substituant) ;
dans lequel ladite formule (II) est : 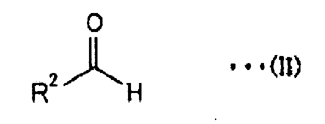 (R² de la formule (II) est choisi dans un groupe constitué par un hydrogène, un groupe alkyle avec 1 à 6 carbones, un groupe aryle avec 6 à 14 carbones, un groupe cycloalkyle avec 3 à 10 carbones, un groupe aralkyle avec 7 à 19 carbones, un groupe alcoxyalkyle avec 2 à 11 carbones, un groupe contenant un hétéroatome dans le squelette carboné de celui-ci, et un groupe contenant une liaison insaturée carbone-carbone dans le squelette carboné de celui-ci, et ces groupes peuvent être soit linéaires, soit ramifiés et peuvent avoir un substituant) ;
dans lequel ladite formule (III) est : 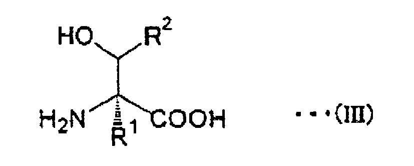 (dans laquelle R¹ de la formule (III) est identique à R¹ de la formule (I) et R² de la formule (III) est identique à R² de la formule (II)).

8. Polynucléotide recombinant ayant ledit polynucléotide selon la revendication 6 ou 7 incorporé en lui.

9. Transformant ayant ledit polynucléotide selon la revendication 8 incorporé en lui.
